(19) Europäisches Patentamt
European Patent Office
Office européen des brevets

(11) Veröffentlichungsnummer : **0 172 360**
**B1**

(12) # EUROPÄISCHE PATENTSCHRIFT

(45) Veröffentlichungstag der Patentschrift : **09.11.88**

(51) Int. Cl.[4] : **C 07 C121/46**, C 09 K 19/30

(21) Anmeldenummer : **85107913.7**

(22) Anmeldetag : **26.06.85**

(54) **Cyclohexancarbonitrile und deren Verwendung als Komponenten für flüssigkristalline Gemische.**

(30) Priorität : **29.06.84 CH 3152/84**
**17.04.85 CH 1638/85**

(43) Veröffentlichungstag der Anmeldung : **26.02.86 Patentblatt 86/09**

(45) Bekanntmachung des Hinweises auf die Patenterteilung : **09.11.88 Patentblatt 88/45**

(84) Benannte Vertragsstaaten : **AT BE CH DE FR GB IT LI NL SE**

(56) Entgegenhaltungen :
**EP-A- 0 107 759**
**EP-A- 0 154 849**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Boller, Arthur, Dr.**
**Benkenstrasse 65**
**CH-4102 Binningen (CH)**
Erfinder : **Petrzilka, Martin, Dr.**
**Schwarzackerstrasse 54**
**CH-4303 Kaiseraugst (CH)**
Erfinder : **Schadt, Martin, Dr.**
**Liestalerstrasse 77**
**CH-4411 Seltisberg (CH)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 172 360 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Cyclohexancarbonitrile, deren Herstellung, flüssigkristalline Gemische, die solche Verbindungen enthalten, sowie die Verwendung für elektro-optische Zwecke.

Flüssige Kristalle haben in den letzten Jahren vor allem als Dielektrika in Anzeigevorrichtungen stark an Bedeutung gewonnen, da die optischen Eigenschaften solcher Substanzen durch eine angelegte Spannung beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Zelle), dem Schadt-Helfrich-Effekt (Drehzelle), dem Gast/Wirt-Effekt (Guest-Host-Zelle) oder einem cholesterisch-nematischen Phasenübergang (Phase-Change-Zelle).

Die verwendeten Flüssigkristalle müssen eine gute Stabilität gegenüber Wärme, Feuchtigkeit, Luft, elektromagnetischer Strahlung, elektrischen Feldern und dergleichen besitzen. Ferner sollten sie farblos sein, kurze Ansprechzeiten und niederen Viskosität aufweisen, einen guten Kontrast ergeben und im ganzen Temperaturbereich, in welchem die Flüssigkristallzelle betrieben werden soll, eine nematische bzw. cholesterische Mesophase besitzen. Da Flüssigkristalle üblicherweise als Mischungen zur Anwendung gelangen, ist es zudem wichtig, dass die Komponenten untereinander gut mischbar sind und dabei eine nematische bzw. cholesterische Mesophase ausbilden. Weitere Eigenschaften, wie beispielsweise die elektrische Leitfähigkeit, die Schwellenspannung, die Multiplexierbarkeit und die dielektrische Anisotropie, müssen je nach verwendetem Zellentyp unterschiedliche Bedingungen erfüllen.

Flüssigkristalle mit negativer Anisotropie der Dielektrizitätskonstante ($\Delta\varepsilon = \varepsilon_{\parallel} - \varepsilon_{\perp} < 0$, wobei $\varepsilon_{\parallel}$ die Dielektrizitätskonstante entlang der Moleküllängsachse und $\varepsilon_{\perp}$ die Dielektrizitätskonstante senkrecht dazu bedeuten) können beispielsweise in Flüssigkristallzellen vom Lichtstreuungstyp, in DAP-Zellen oder in Guest/Host-Zellen angewendet werden. Ferner wurde zur Verbesserung des Multiplexverhältnisses bei der Multiplex-Ansteuerung von Flüssigkristallanzeigen, insbesondere von Drehzellen und Guest/Host-Zellen, eine Zwei-Frequenz-Adressierung vorgeschlagen (z. B. Deutsche Offenlegungsschriften 2856134 und 2907940). Zur Verbesserung der dielektrischen Anisotropie oberhalb der « Cross-over-Frequenz » (dielektrische Relaxationsfrequenz, bei welcher $\varepsilon_{\parallel} = \varepsilon_{\perp}$ wird) können den entsprechenden Dielektrika Komponenten mit negativer dielektrischer Anisotropie zugesetzt werden (z. B. Deutsche Offenlegungsschrift 3221462).

Es sind bereits Verbindungen mit grosser negativer Anisotropie der dielektrizitätskonstante bekannt, beispielsweise Pyridazine, Pyridazinoxide und Verbindungen mit einer 2,3-Dicyano-1,4-phenylen-Gruppe. Diese weisen jedoch verschiedene Nachteile auf, wie z. B. schlechte Löslichkeit in Mischungen, hohe Viskosität, hohe Schmelzpunkte, starke smektische Tendenzen, chemische Instabilität, hohe elektrische Leitfähigkeit oder starke Klärpunktsdepressionen in Mischungen, und sind daher nur sehr beschränkt anwendbar.

Es wurde nun gefunden, dass die Verbindungen der allgemeinen Formel

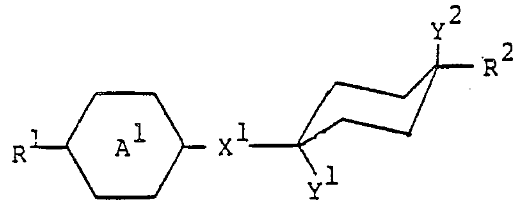

(I)

worin $X^1$ eine einfache Kovalenzbindung, $-CH_2CH_2-$, $-COO-$, $-OOC-$ oder 1,4-Phenylen darstellt; eines der Symbole $Y^1$ und $Y^2$ Wasserstoff und das andere Cyano bezeichnet; $R^2$ trans-1-Alkenyl, cis-2-Alkenyl, trans-3-Alkenyl, 4-Alkenyl oder Alkyl bedeutet; $R^1$ für die Gruppe $R^{11}-A^4-A^5-$ steht und $A^4$ und $A^5$ unabhängig voneinander eine einfache Kovalenzbindung, 1,4-Phenylen oder trans-1,4-Cyclohexylen darstellen; $R^{11}$ trans-1-Alkenyl, cis-2-Alkenyl, trans-3-Alkenyl, 4-Alkenyl, trans-2-Alkenyloxy, 3-Alkenyloxy oder, wenn $R^2$ für eine Alkenylgruppe steht, auch Alkyl oder Alkoxy bedeutet; Ring $A^1$ 1,4-Phenylen oder eine Gruppe der allgemeinen Formel

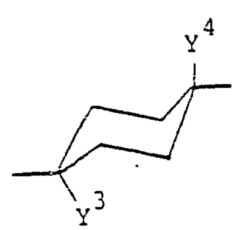

(II)

darstellt ; und $Y^3$ und $Y^4$ Wasserstoff bezeichnen oder, sofern $X^1$ $—CH_2CH_2—$, —COO— oder —OOC— darstellt, eines der Symbole $Y^3$ und $Y^4$ auch Cyano bezeichnet, eine relativ grosse negative Anisotropie der Dielektrizitätskonstante, gute Mischbarkeit mit andern Flüssigkristallkomponenten und niedere Viskosität aufweisen. Sie sind farblos und besitzen eine gute chemische Stabilität. Die Verbindungen der Formel I, worin $R^1$ und/oder $R^2$ eine trans-1-Alkenyl-, trans-3-Alkenyl- oder trans-2-Alkenyloxy-Gruppe aufweisen, besitzen besonders günstige Mesophasenbereiche, während die Verbindungen der Formel I, worin $R^1$ und/oder $R^2$ eine cis-2-Alkenyl-, 4-Alkenyl- oder 3-Alkenyloxy-Gruppe aufweisen, vor allem ein günstiges Verhältnis der elastischen Konstanten $k_{33}$ (bend) und $k_{11}$ (splay) aufweisen und vergleichsweise niedrige Schwellenspannungen ergeben. Die erfindungsgemässen Verbindungen sind somit besonders geeignet als Komponenten von Flüssigkristallmischungen mit negativer dielektrischer Anisotropie und als Komponenten von Flüssigkristallmischungen, welche bei der Zwei-Frequenz-Matrix-Adressierung verwendet werden. Die Verbindungen der Formel I, worin $A^4$ und $A^5$ einfache Kovalenzbindungen bedeuten, d. h. $R^1$ für $R^{11}$ steht, eignen sich vor allem als niederviskose Basiskomponenten, während die übrigen Verbindungen der Formel I hauptsächlich als Zusätze zur Klärpunktserhöhung Anwendung finden. Die erfindungsgemässen Verbindungen können aber auch in Mischungen mit positiver dielektrischer Anisotropie verwendet werden, beispielsweise um die Schwallenspannung an die verwendete elektro-optische Zelle anzupassen.

Alkenyl-, Alkenyloxy-, Alkyl- und Alkoxygruppen in der allgemeinen Formel I besitzen im allgemeinen bis zu 12, vorzugsweise bis zu 7 Kohlenstoffatome.

Im Rahmen der vorliegenden Erfindung bedeutet der Ausdruck « Alkyl » vorzugsweise geradkettiges Alkyl, wie Methyl, Aethyl, Propyl, Butyl, Pentyl, Hexyl, Heptyl, Octyl, Nonyl, Decyl, Undecyl und Dodecyl. Der Ausdruck « trans-1-Alkenyl » umfasst 1E-Alkenylreste wie Vinyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undedenyl und 1E-Dodecenyl. Der Ausdruck « cis-2-Alkenyl » umfasst 2Z-Alkenylreste wie 2-Propenyl, 2Z-Butenyl, 2Z-Pentenyl, 2Z-Hexenyl, 2Z-Heptenyl, 2Z-Octenyl, 2Z-Nonenyl, 2Z-Decenyl, 2Z-Undecenyl und 2Z-Dodecenyl. Der Ausdruck « trans-3-Alkenyl » umfasst 3E-Alkenylreste wie 3-Butenyl, 3E-Pentenyl, 3E-Hexenyl, 3E-Heptenyl, 3E-Octenyl, 3E-Nonenyl, 3E-Decenyl, 3E-Undecenyl und 3E-Dodecenyl. Der Ausdruck « 4-Alkenyl » umfasst Reste wie 4-Pentenyl und die E- und/oder Z-form von 4-Hexenyl, 4-Heptenyl, 4-Octenyl, 4-Nonenyl, 4-Decenyl, 3-Undecenyl und 4-Dodecenyl. Der Ausdruck « Alkoxy » umfasst vorzugsweise geradkettiges Alkoxy, wie Methoxy, Aethoxy, Propyloxy, Butyloxy, Pentyloxy, Hexyloxy, Heptyloxy, Octyloxy, Nonyloxy, Decyloxy, Undecyloxy und Dodecyloxy. Der Ausdruck « trans-2-Alkenyloxy » umfasst 2E-Alkenyloxyreste wie 2-Propenyloxy, 2E-Butenyloxy, 2E-Pentenyloxy, 2E-Hexenyloxy, 2E-Heptenyloxy, 2E-Octenyloxy, 2E-Nonenyloxy, 2E-Decenyloxy, 2E-Undecenyloxy und 2E-Dodecenyloxy. Der Ausdruck « 3-Alkenyloxy » umfasst Reste wie 3-Butenyloxy und die E- und/oder Z-Form von 3-Pentenyloxy, 3-Hexenyloxy, 3-Heptenyloxy, 3-Octenyloxy, 3-Nonenyloxy, 3-Decenyloxy, 3-Undecenyloxy und 3-Dodecenyloxy.

Im Rahmen der vorliegenden Erfindung bedeutet der Ausdruck « Halogen », Chlor, Brom oder Jod und der Ausdruck « Alkalimetall » Lithium, Natrium oder Kalium. Der Ausdruck « Aryl » bedeutet Phenyl, Tolyl und dergleichen.

Die obige Formel I umfasst die Teilformeln

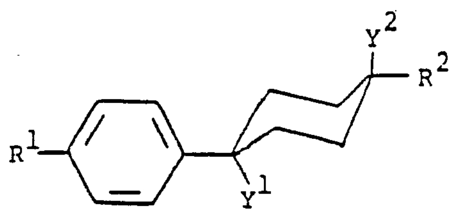

(Ia)

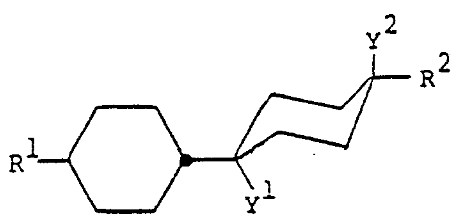

(Ib)

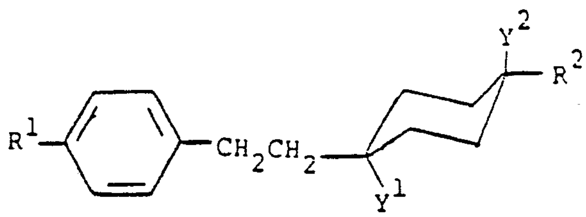

(Ic)

(Fortsetzung)

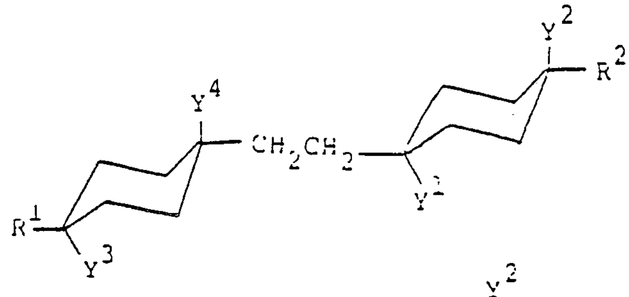
(Id)

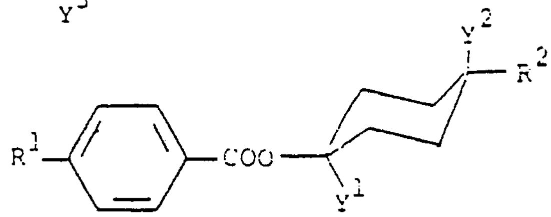
(Ie)

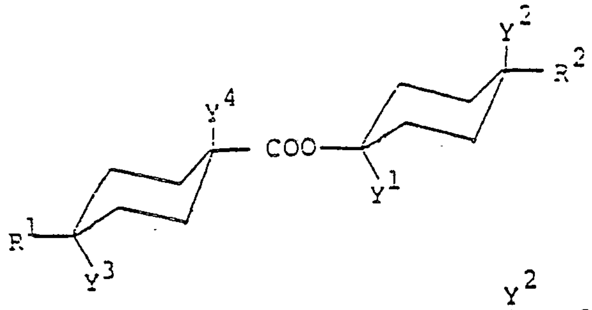
(If)

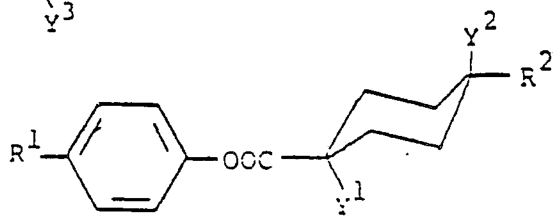
(Ig)

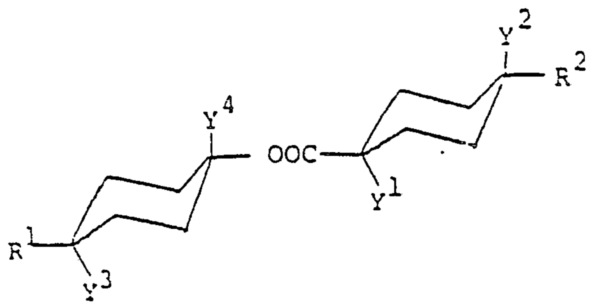
(Ih)

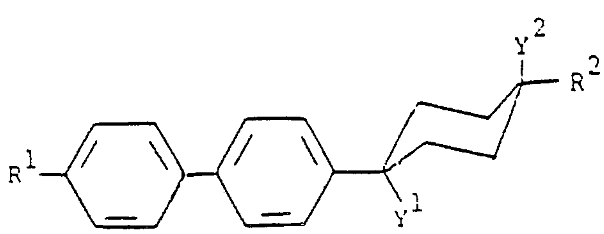
(Ii)

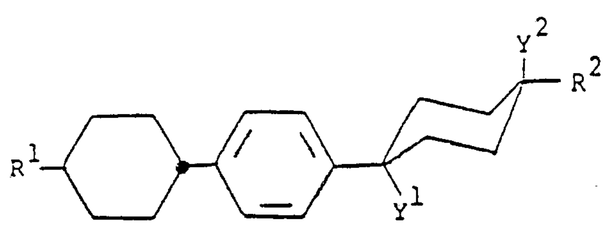
(Ij)

worin eines der Symbole $Y^1$ unds $Y^2$ Wasserstoff und das andere Cyano bedeutet ; $Y^3$ und $Y^4$ Wasserstoff bezeichnen oder eines der Symbole $Y^3$ und $Y^4$ auch Cyano bezeichnet ; und $R^1$ und $R^2$ die obigen Bedeutungen haben.

Bevorzugte Verbindungen der Formel I und der Formeln Ia-Ij sind diejenigen, worin $A^5$ eine einfache Kovalenzbindung darstellt. Besonders bevorzugt sind diejenigen Verbindungen, worin $A^4$ und $A^5$ einfache Kovalenzbindungen darstellen, d. h. $R^1$ für $R^{11}$ steht.

$R^{11}$ und $R^2$ stehen vorzugsweise für geradkettige Reste mit höchstens 12 Kohlenstoffatomen, insbesondere für geradkettiges trans-1-Alkenyl mit 2 bis 12 Kohlenstoffatomen, geradkettiges cis-2-Alkenyl mit 3 bis 12 Kohlenstoffatomen, geradkettiges trans-3-Alkenyl mit 4 bis 12 Kohlenstoffatomen, geradkettiges 4-Alkenyl mit 5 bis 12 Kohlenstoffatomen oder einer der Reste $R^{11}$ und $R^2$ auch für geradkettiges Alkyl mit 1 bis 12 Kohlenstoffatomen. Besonders bevorzugt sind Reste $R^{11}$ und $R^2$ mit höchstens 7 Kohlenstoffatomen.

Vorzugsweise bedeuten $R^{11}$ und $R^2$ unabhängig voneinander trans-1-Alkenyl, trans-3-Alkenyl oder 4-Alkenyl oder einer der Reste $R^{11}$ und $R^2$, vorzugsweise $R^{11}$, auch Alkyl. Besonders bevorzugt sind die Verbindungen der Formel I bzw. der Formeln Ia-Ij, worin einer der Reste $R^{11}$ und $R^2$ trans-3-Alkenyl und der andere trans-1-Alkenyl, trans-3-Alkenyl, 4-Alkenyl oder Alkyl bedeutet.

In den Verbindungen der Formel I, worin Ring $A^1$ 1,4-Phenylen darstellt, d. h. in den Verbindungen der Formeln Ia, Ic, Ie, Ig und Ii, bedeutet $R^1$ vorzugsweise trans-3-Alkenyl, 4-Alkenyl oder Alkyl.

In $R^1$ und/oder $R^2$ gegebenenfalls vorhandene 4-Alkenylreste und ein in $R^1$ gegebenenfalls vorhandener 3-Alkenyloxyrest liegen vorzugsweise in der Z-Form vor.

Besonders bevorzugte Alkenylreste sind 3-Butenyl, 4-Pentenyl, 3E-Pentenyl und 1E-Pentenyl.

Bevorzugte Verbindungen der Formel Id sind diejenigen, worin $Y^3$ und $Y^4$ Wasserstoff bedeuten, sowie diejenigen, worin $Y^4$ und zugleich $Y^2$ Cyano bedeuten. Von den übrigen Verbindungen der Formel I sind im allgemeinen diejenigen bevorzugt, worin $Y^2$ Cyano bedeutet und gegebenenfalls vorhandene Substituenten $Y^3$ und $Y^4$ Wasserstoff bedeuten.

Weiterhin sind im allgemeinen diejenigen Verbindungen der Formel I bevorzugt, worin Ring $A^1$ trans-1,4-Cyclohexylen darstellt, d. h. die Verbindungen der Formeln Ib und Ij und die Verbindungen der Formeln Id, If und Ih, worin $Y^3$ und $Y^4$ Wasserstoff bezeichnen.

Die Verbindungen der Formel I können erfindungsgemäss dadurch hergestellt werden, dass man

a) zur Herstellung der Verbindungen der Formel I, worin $X^1$ —COO- oder —OOC— darstellt, eine Verbindung der allgemeinen Formel

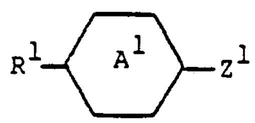

(III)

mit einer Verbindung der allgemeinen Formel

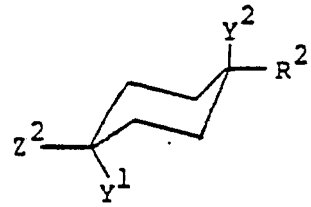

(IV)

worin eine der Gruppen $Z^1$ und $Z^2$ —COOH oder —COCl und die andere —OH bedeutet : Ring $A^1$ 1,4-Phenylen oder eine Gruppe der obigen Formel II darstellt ; $Y^3$ und $Y^4$ in Formel II Wasserstoff bezeichnen oder eines dieser Symbole auch Cyano bezeichnet ; und $R^1$, $R^2$, $Y^1$ und $Y^2$ die obigen Bedeutungen haben, verestert, oder

b) zur Herstellung der Verbindungen der Formel I, worin $X^1$ eine einfache Kovalenzbindung, $—CH_2CH_2—$ oder 1,4-Phenylen darstellt, $Y^2$ Cyano bezeichnet und $R^2$ cis-2-Alkenyl, trans-3-Alkenyl, 4-Alkenyl oder Alkyl bedeutet, eine Verbindung der allgemeinen Formel

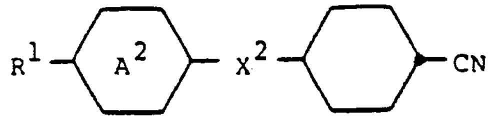

(V)

zuerst mit Base und dann mit einer Verbindung der allgemeinen Formel

$$Z^3—R^5 \quad \text{(VI)}$$

worin $X^2$ eine einfache Kovalenzbindung, $—CH_2CH_2—$ oder 1,4-Phenylen darstellt ; Ring $A^2$ 1,4-Phenylen

oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; $Z^3$ Halogen bezeichnet ; $R^5$ cis-2-Alkenyl, trans-3-Alkenyl, 4-Alkenyl oder Alkyl bedeutet ; und $R^1$ die obige Bedeutung hat, umsetzt, oder

c) zur Herstellung der Verbindungen der Formel I, worin $X^1$ eine einfache Kovalenzbindung oder $—CH_2CH_2—$ darstellt, Ring $A^1$ trans-1,4-Cyclohexylen bedeutet und $Y^1$ Cyano bezeichnet, eine Verbindung der allgemeinen Formel

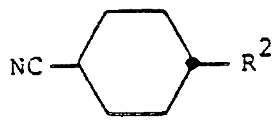

(VII)

zuerst mit Base und dann mit einer Verbindung der allgemeinen Formel

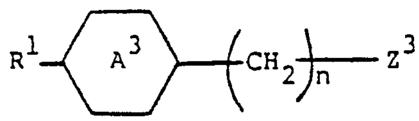

(VIII)

worin n für die Zahl O und Ring $A^3$ für cis-1,4-Cyclohexylen steht oder n für die Zahl 2 und Ring $A^3$ für trans-1,4-Cyclohexylen steht ; $Z^3$ Halogen bezeichnet ; und $R^1$ und $R^2$ die obigen Bedeutungen haben, umsetzt, oder

d) zur Herstellung der Verbindungen der Formel I, worin $X^1$ eine einfache Kovalenzbindung, $—CH_2CH_2—$ oder 1,4-Phenylen darstellt und $Y^1$ Cyano bezeichnet, eine Verbindung der allgemeinen Formel

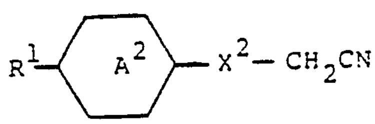

(IX)

zuerst mit Base und dann mit einer Verbindung der allgemeinen Formel

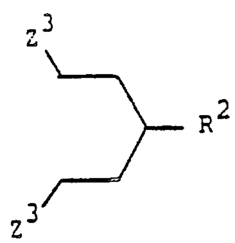

(X)

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten, oder, sofern $X^2$ $—CH_2CH_2—$ darstellt, eines der Symbole $Y^3$ und $Y^4$ auch Cyano bezeichnet ; $Z^3$ Halogen bezeichnet ; und $R^1$ und $R^2$ die obigen Bedeutungen haben, umsetzt, oder

e) zur Herstellung der Verbindungen der Formel I, worin $X^1$ eine einfache Kovalenzbindung, $—CH_2CH_2—$ oder 1,4-Phenylen darstellt : $Y^2$ Cyano bezeichnet und $R^2$ trans-1-Alkenyl bedeutet, eine Verbindung der allgemeinen Formel

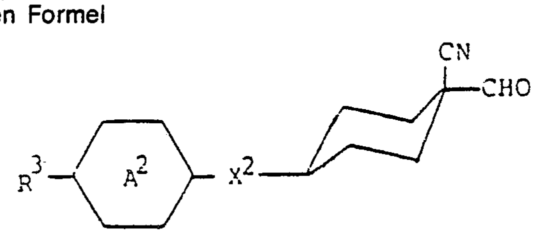

(XI)

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; und $R^3$ die für $R^1$ in Formel I gegebenen Bedeutungen hat, in Gegenwart einer Base mit Alkyl-triarylphosphoniumhalogenid umsetzt, oder

f) zur Herstellung der Verbindungen der Formel I, worin $X^1$ eine einfache Kovalenzbindung, $—CH_2CH_2—$ oder 1,4-Phenylen darstellt, an eine Verbindung der allgemeinen Formel

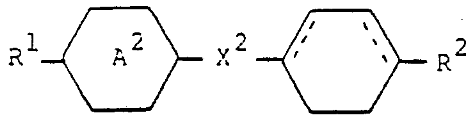

(XII)

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; eine der unterbrochenen Linien eine zusätzliche Bindung bezeichnet ; und $R^1$ und $R^2$ die obigen Bedeutungen haben, HCN anlagert, oder

g) zur Herstellung der Verbindungen der Formel I, worin $X^1$ eine einfache Kovalenzbindung, $—CH_2CH_2—$ oder 1,4-Phenylen darstellt, und $R^1$ trans-2-Alkenyloxy, 3-Alkenyloxy oder Alkoxy bedeutet, eine Verbindung der allgemeinen Formel

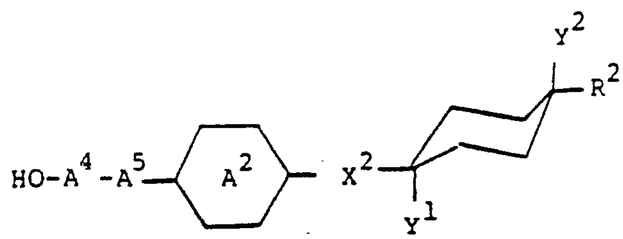

(LXXXI)

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; und $A^4$, $A^5$, $R^2$, $Y^1$ und $Y^2$ die in Formel I gegebenen Bedeutungen haben, mit trans-2-Alkenylhalogenid, 3-Alkenylhalogenid oder Alkylhalogenid veräthert.

Die Veresterung einer Verbindung der Formel III mit einer Verbindung der Formel IV (Verfahrensvariante a) kann in an sich bekannter Weise erfolgen. Die Veresterung der Säurechloride (welche aus den Carbonsäuren z. B. durch Erhitzen mit Thionylchlorid erhältlich sind) kann beispielsweise in Diäthyäther, Tetrahydrofuran, Dimethylformamid, Benzol, Toluol, Tetrachlorkohlenstoff, Pyridin und dergleichen erfolgen. Die Veresterung der Carbonsäuren erfolgt vorzugsweise in Gegenwart von 4-(Dimethylamino)-pyridin und Dicyclohexylcarbodiimid oder in Gegenwart von Oxalylchlorid und Dimethylformamid. Temperatur und Druck dieser Veresterungsreaktionen sind nicht kritisch. Im allgemeinen werden Atmosphärendruck und eine Temperatur zwischen etwa — 30 °C und der Siedetemperatur des Reaktionsgemisches angewendet.

Die Umsetzung eines Nitrils der Formel V, VII oder IX mit Base und anschliessend mit einem Halogenid der Formel VI, VIII oder X (Verfahrensvarianten b-d) kann ebenfalls in an sich bekannter Weise erfolgen. Bevorzugtes Halogen $Z^3$ ist Brom. Geeignete Basen sind beispielsweise Natriumhydrid, Natriumamid, Lithiumdiisopropylamid, Kalium-t-butylat und dergleichen. Bevorzugte Basen sind die Alkalimetall-dialkylamide, worin der Ausdruck « Alkyl » vorzugsweise geradkettige oder verzweigte Reste mit 1 bis 5 Kohlenstoffatomen bedeutet. Besonders bevorzugte Base ist Lithiumdiisopropylamid. Die Reaktion wird zweckmässigerweise in einem inerten organischen Lösungsmittel, beispielsweise einem Aether, einem aromatischen Kohlenwasserstoff, einem Amid oder einem Sulfoxid, wie Tetrahydrofuran, Dioxan, Diäthyläther, Toluol, Dimethylformamid oder Dimethylsulfoxid, durchgeführt. Temperatur und Druck sind nicht kritisch ; im allgemeinen werden jedoch Verfahrensvarianten b und c bei Atmosphärendruck und einer Temperatur von etwa — 80 °C bis Raumtemperatur und Verfahrensvariante d bei Atmosphärendruck und einer Temperatur von etwa 0-150 °C durchgeführt.

Die Umsetzung einer Verbindung der Formel XI mit Alkyl-triarylphosphoniumhalogenid, vorzugsweise Alkyl-triphenylphosphoniumchlorid oder Alkyl-triphenylphosphoniumbromid (Verfahrensvariante e) kann ebenfalls in an sich bekannter Weise erfolgen. Geeignete Basen sind die oben unter Verfahrensvariante b-d genannten Basen. Bevorzugte Basen sind jedoch die Alkalimetallcarbonate und insbesondere die Alkalimetallalkoholate mit 1 bis 5 Kohlenstoffatomen. Beispiele bevorzugter Basen sind Kaliumcarbonat, Natriummethylat und Kalium-t-butylat. Die Reaktion wird zweckmässig in einem inerten organischen Lösungsmittel, beispielsweise einem Aether, wie Diäthyläther, Tetrahydrofuran oder Dioxan, durchgeführt. Temperatur und Druck sind nicht kritisch ; im allgemeinen wird jedoch bei Atmosphärendruck und einer Temperatur von Raumtemperatur bis Rückflusstemperatur gearbeitet.

Die Anlagerung von HCN an eine Verbindung der Formel XII (Verfahrensvariante f) kann ebenfalls in an sich bekannter Weise erfolgen. Die Reaktion erfolgt zweckmässigerweise in einem inerten organischen Lösungsmittel, beispielsweise einem Nitril, Amid oder chlorierten Kohlenwasserstoff, wie Acetonitril, Dimethylformamid, Methylenchlorid oder Chloroform. Temperatur und Druck sind nicht kritisch ; im allgemeinen wird jedoch eine Temperatur von etwa — 10 °C bis 150 °C und ein Druck von etwa 1-100 bar angewendet. Die Reaktion kann gewünschtenfalls auch in Gegenwart eines Katalysators, wie Palladium-

bis-[2,3,0-isopropyliden-2,3-dihydroxy-1,4-bis-(diphenylphosphino) butan] und dergleichen, durchgeführt werden.

Die Verätherung einer Verbindung der Formel LXXXI (Verfahrensvariante g) kann ebenfalls in an sich bekannter Weise erfolgen. Zweckmässigerweise wird die Reaktion in einem inerten organischen Lösungsmittel in Gegenwart einer Base, wie Natriumhydrid, Natrium, Natriumcarbonat, Kaliumcarbonat und dergleichen, durchgeführt. Bevorzugte Halogenide sind die Bromide und insbesondere die Jodide. Vorzugsweise wird bei Atmosphärendruck und einer Temperatur von Raumtemperatur bis Rückflusstemperatur gearbeitet.

Die Verbindungen der Formeln III-V, VII-X und LXXXI, worin $R^1$ bzw. $R^2$ eine Alkyl- oder Alkoxygruppe aufweist, und die Verbindungen der Formel VI sind bekannte oder Analoge bekannter Verbindungen.

Die übrigen Ausgangsmaterialien, d. h. die Verbindungen der Formeln III-V, VII-X und LXXXI, worin $R^1$ bzw. $R^2$ eine Alkenyl- oder Alkenyloxygruppe aufweist, und die Verbindungen der Formeln XI und XII sind neu. Die Herstellung dieser Ausgangsmaterialien wird anhand der nachstehenden Reaktionsschemata 1-12 veranschaulicht, worin R Alkyl, $R^4$ Wasserstoff oder Alkyl und THP die Tetrahydropyranylgruppe bedeutet, $R^5$ cis-2-Alkenyl, trans-3-Alkenyl, 4-Alkenyl oder Alkyl bezeichnet und $R^1$, $R^2$, $R^3$, $X^2$ und Ring $A^2$ die obigen Bedeutungen haben.

Schema 1

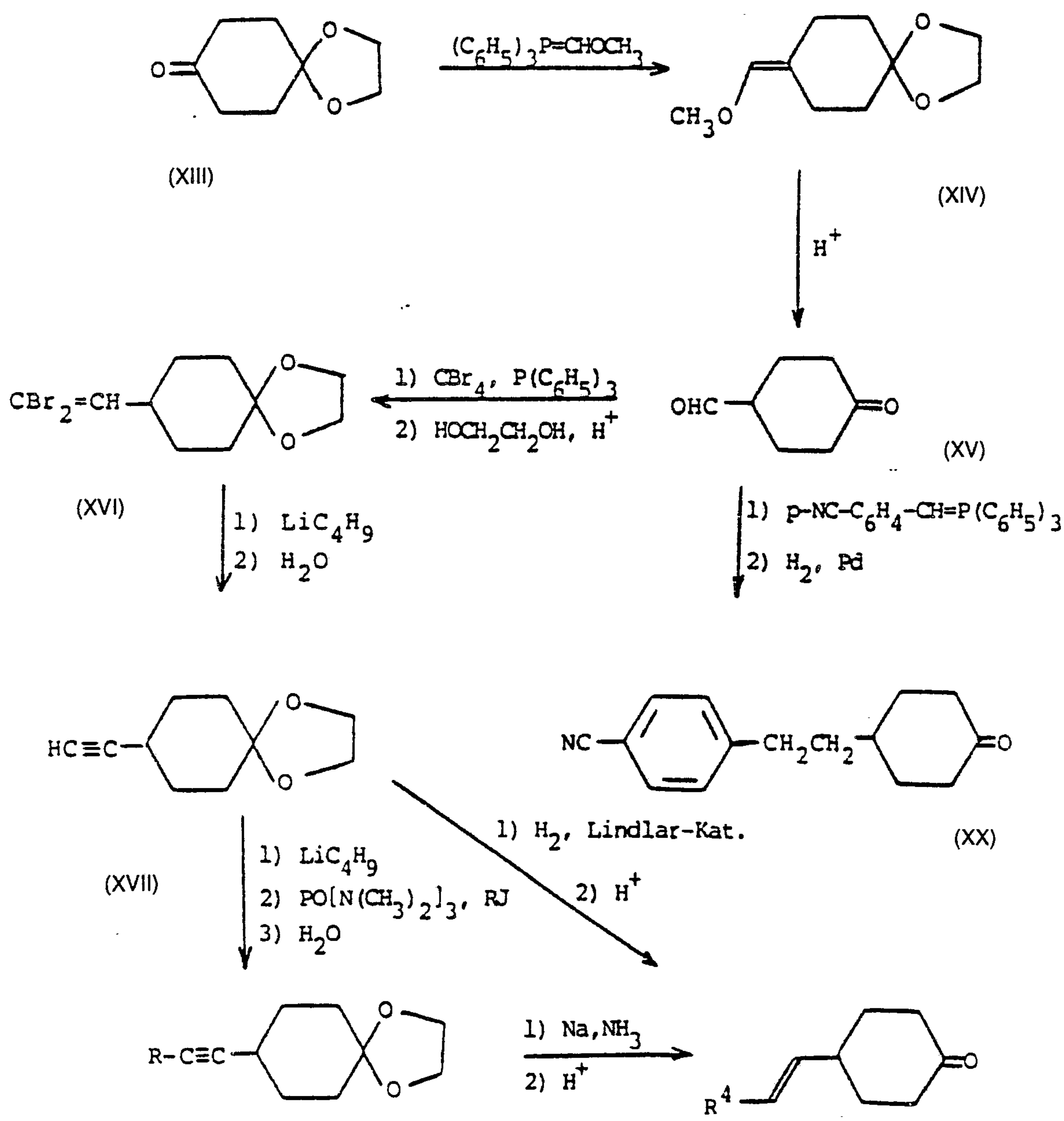


(XIII) (XIV) (XV) (XVI) (XVII) (XX) (XVIII) (XIXa)

Schema 2

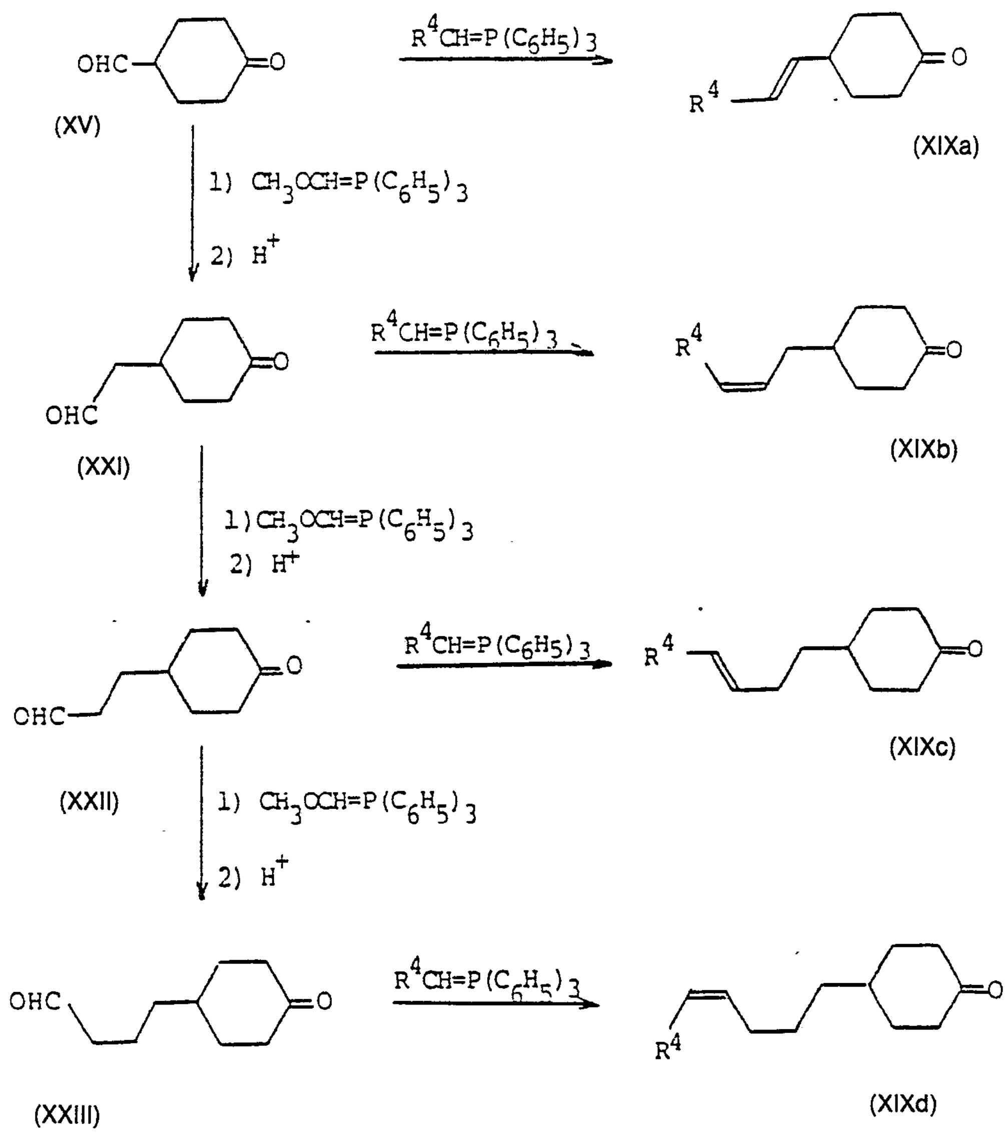
(XV)
$R^4CH=P(C_6H_5)_3$
(XIXa)
1) $CH_3OCH=P(C_6H_5)_3$
2) $H^+$
(XXI)
$R^4CH=P(C_6H_5)_3$
(XIXb)
1) $CH_3OCH=P(C_6H_5)_3$
2) $H^+$
(XXII)
$R^4CH=P(C_6H_5)_3$
(XIXc)
1) $CH_3OCH=P(C_6H_5)_3$
2) $H^+$
(XXIII)
$R^4CH=P(C_6H_5)_3$
(XIXd)

Schema 3

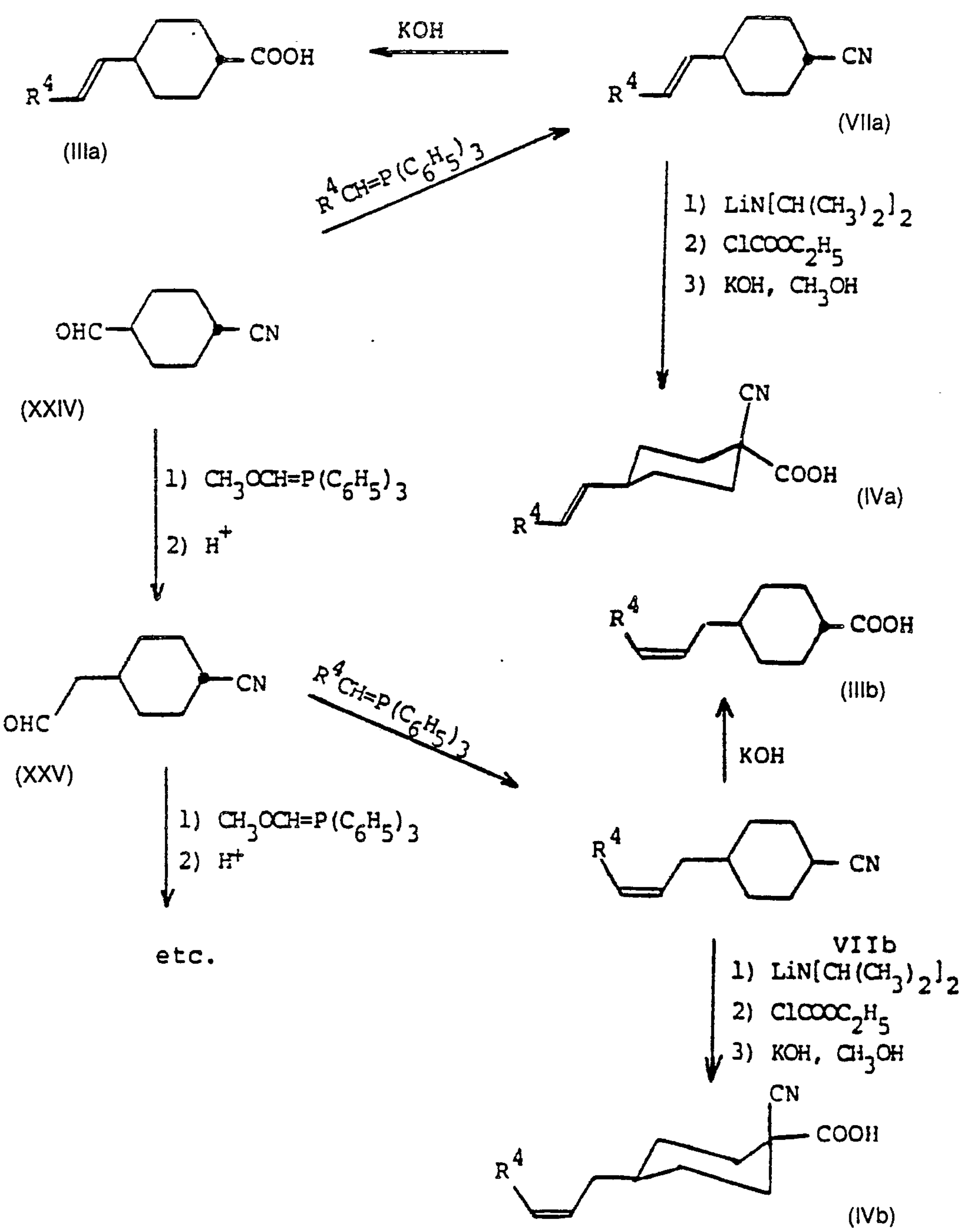


(IIIa) (VIIa) (XXIV) (IVa) (XXV) (IIIb) VIIb (IVb)

Schema 4

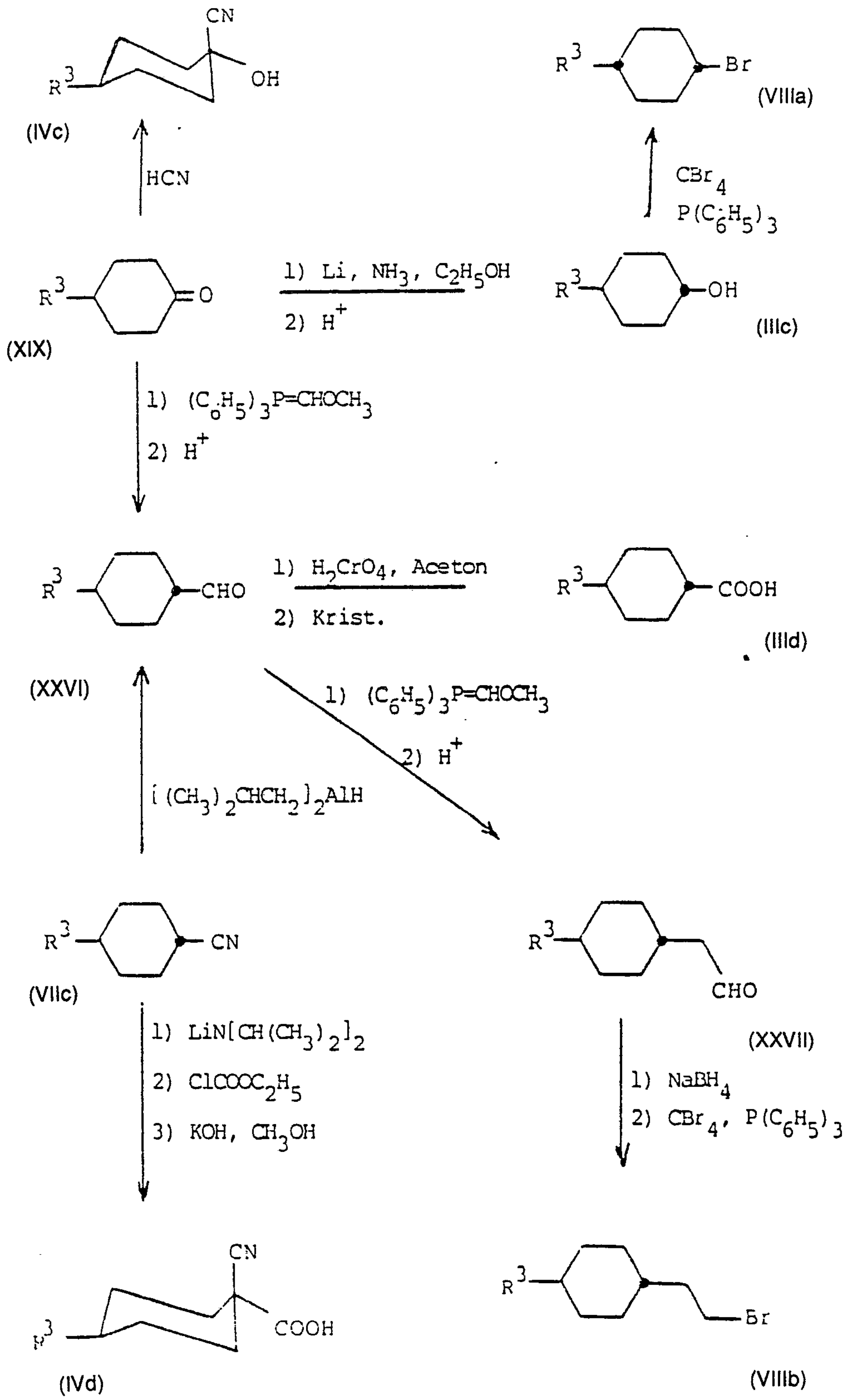
(IVc)
(VIIIa)
HCN
$CBr_4$
$P(C_6H_5)_3$
1) Li, $NH_3$, $C_2H_5OH$
2) $H^+$
(XIX)
(IIIc)
1) $(C_6H_5)_3P{=}CHOCH_3$
2) $H^+$
1) $H_2CrO_4$, Aceton
2) Krist.
(IIId)
(XXVI)
1) $(C_6H_5)_3P{=}CHOCH_3$
2) $H^+$
$[(CH_3)_2CHCH_2]_2AlH$
(VIIc)
(XXVII)
1) $LiN[CH(CH_3)_2]_2$
2) $ClCOOC_2H_5$
3) KOH, $CH_3OH$
1) $NaBH_4$
2) $CBr_4$, $P(C_6H_5)_3$
(IVd)
(VIIIb)

Schema 5

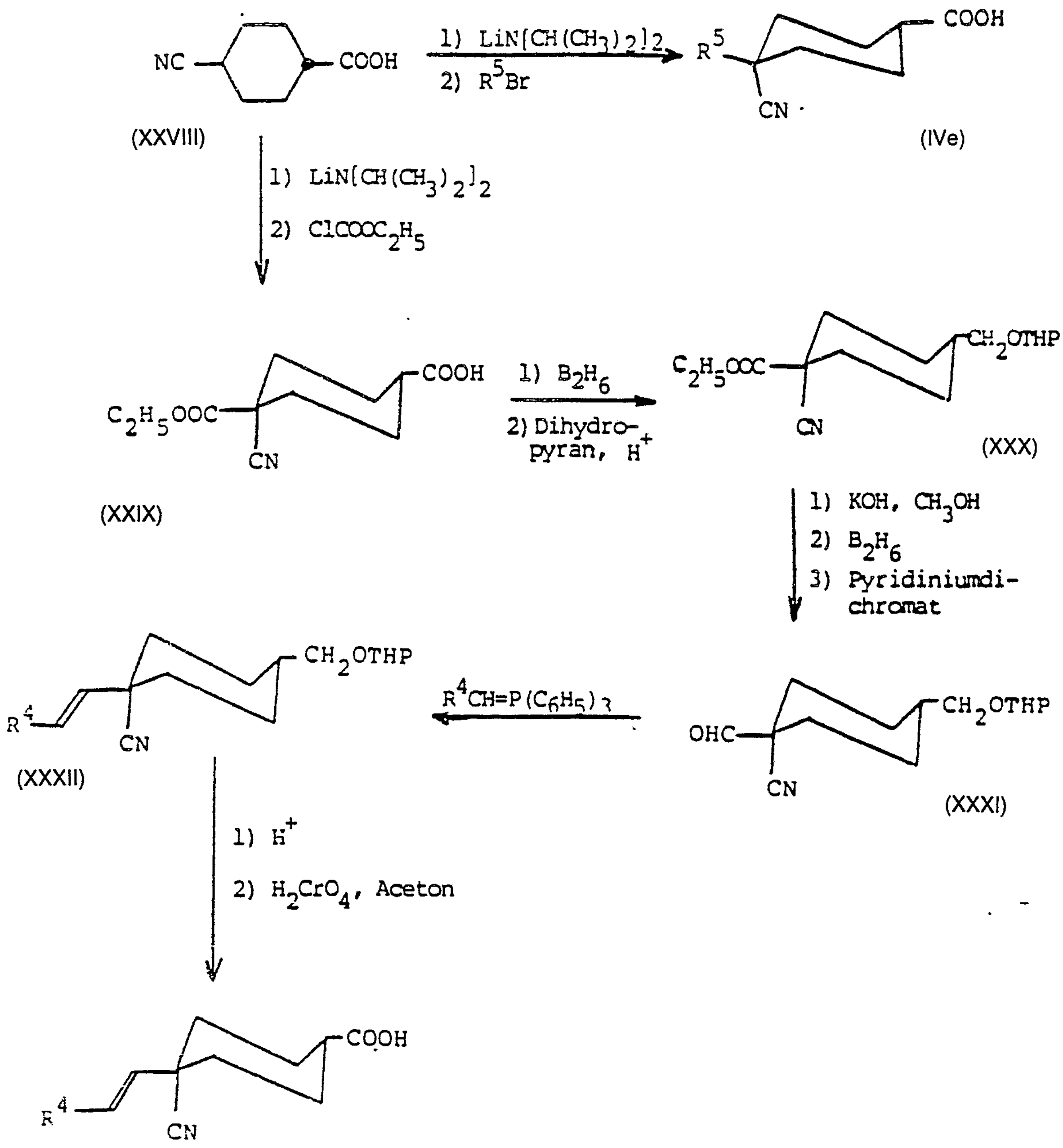


(XXVIII)

(IVe)

(XXIX)

(XXX)

(XXXI)

(XXXII)

(IVf)

Schema 6

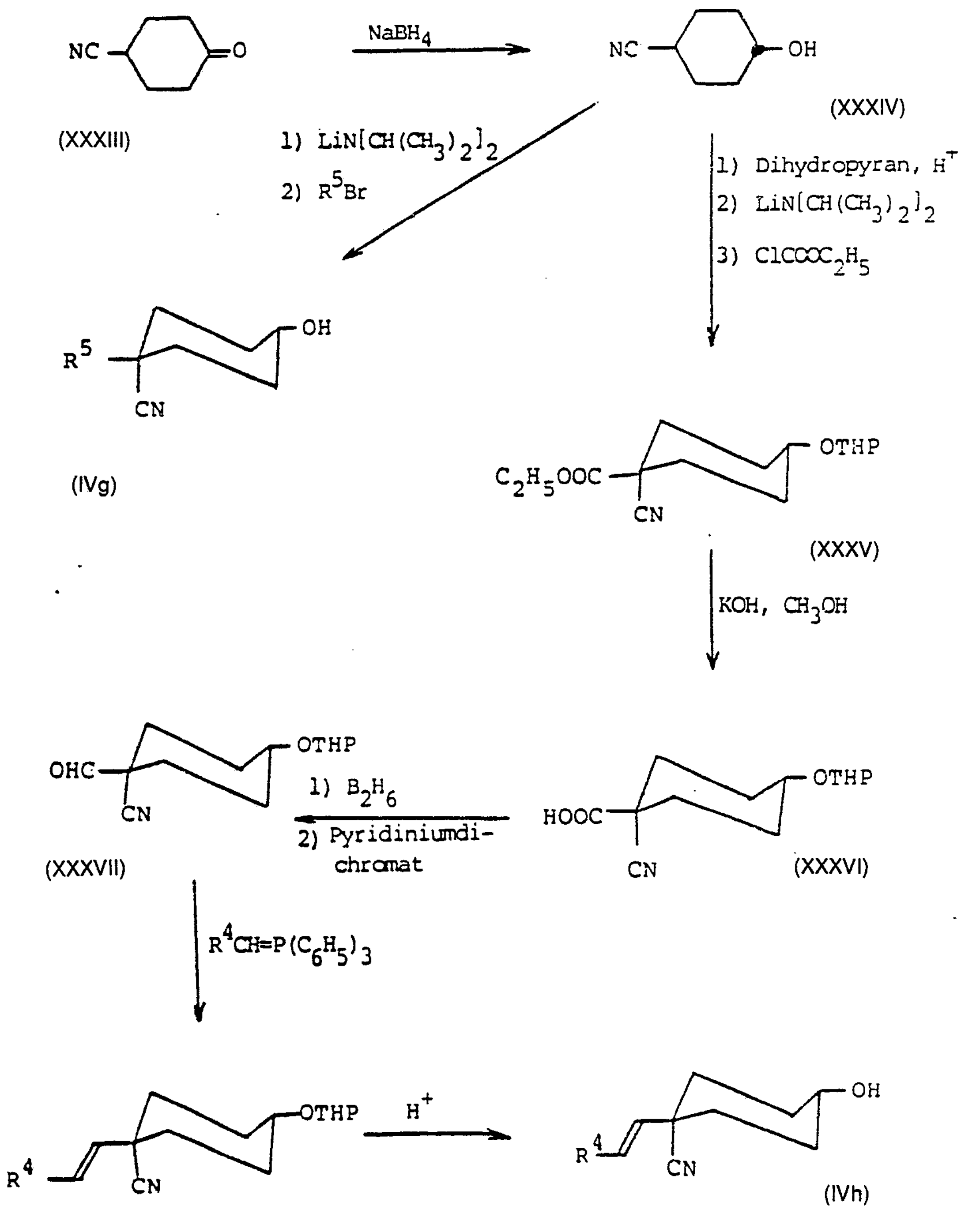


(XXXIII) (XXXIV) (IVg) (XXXV) (XXXVI) (XXXVII) (XXXVIII) (IVh)

Schema 7

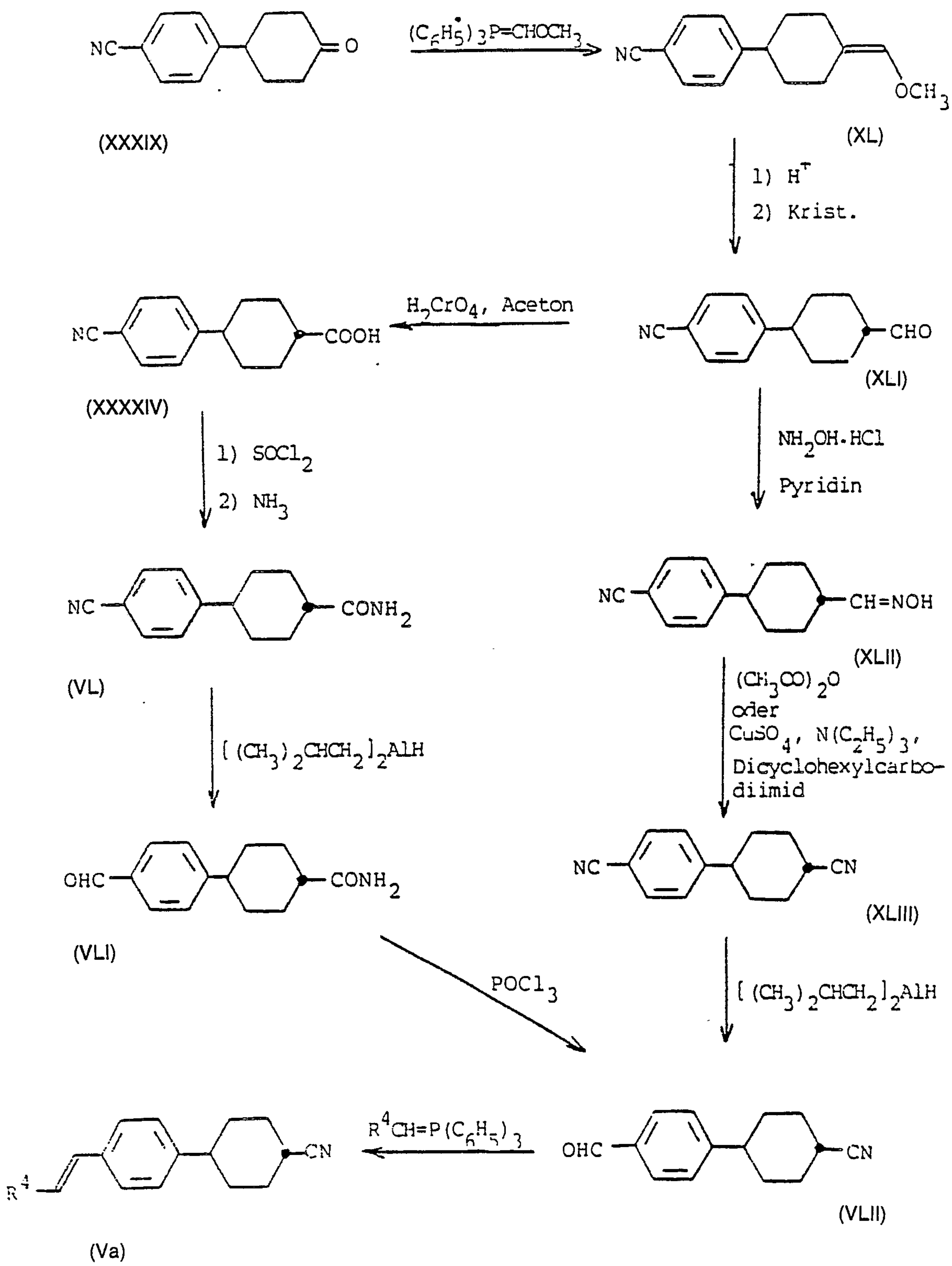


(XXXIX) (XL) (XLI) (XXXXIV) (XLII) (VL) (XLIII) (VLI) (VLII) (Va)

Schema 8

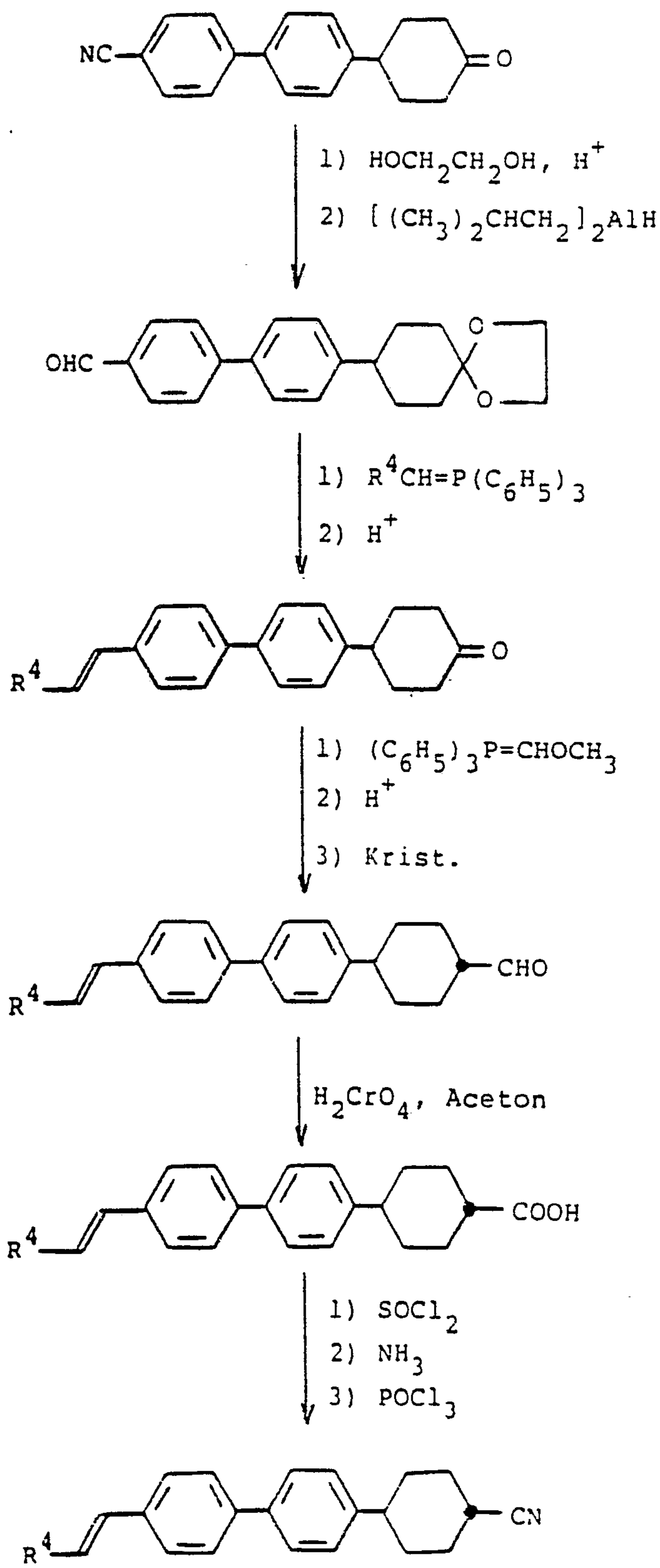


(VLIII)

(IL)

(L)

(LI)

(LII)

(Vb)

Schema 9

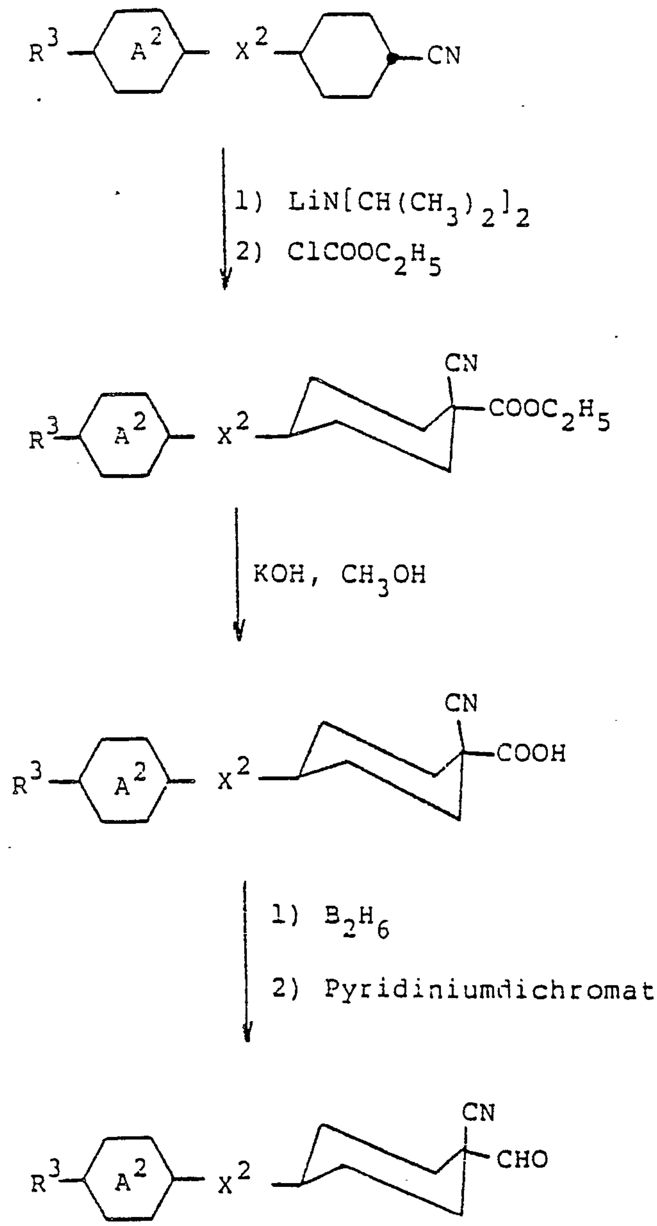


(LII)

(LIV)

(LV)

(XI)

Schema 10

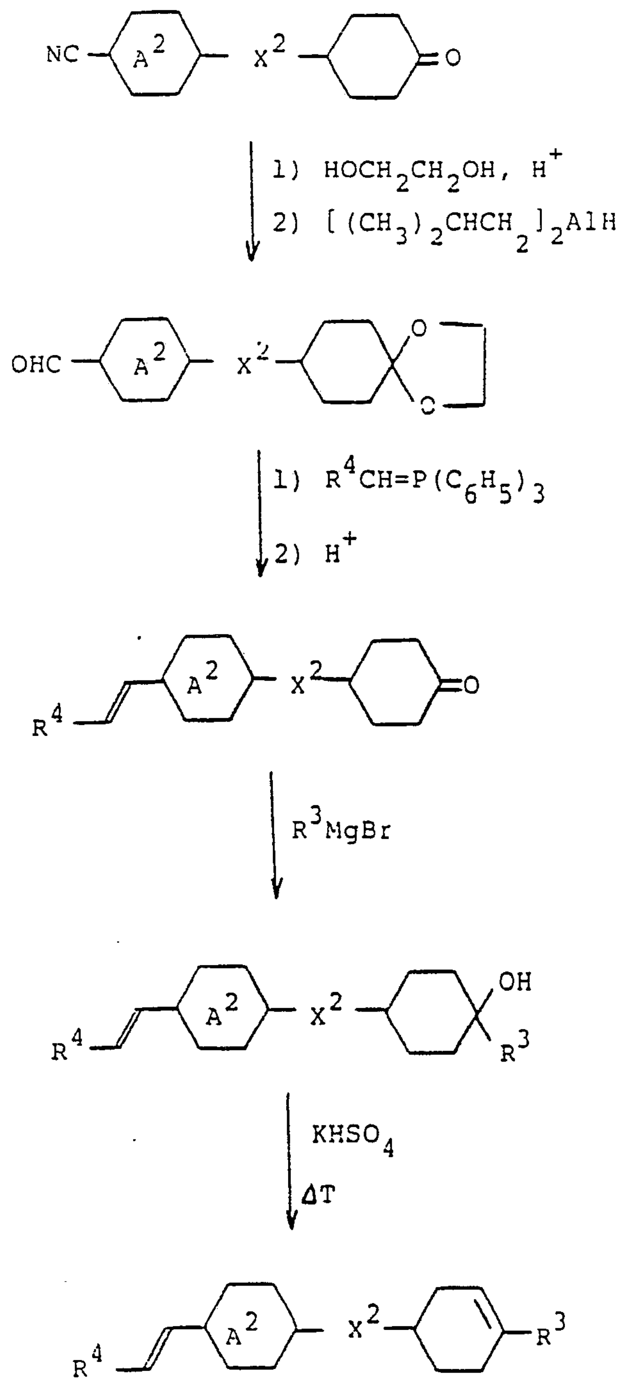


(LVI)

(LVII)

(LVIII)

(LIX)

(XIIa)

Schema 11

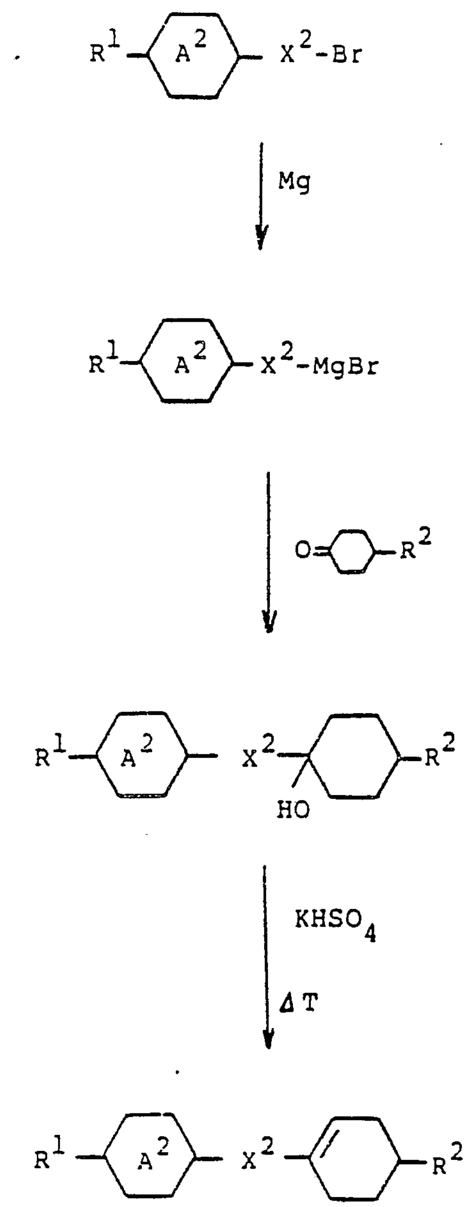


(LX)

(LXI)

(LXIII)

(XIIb)

Schema 12

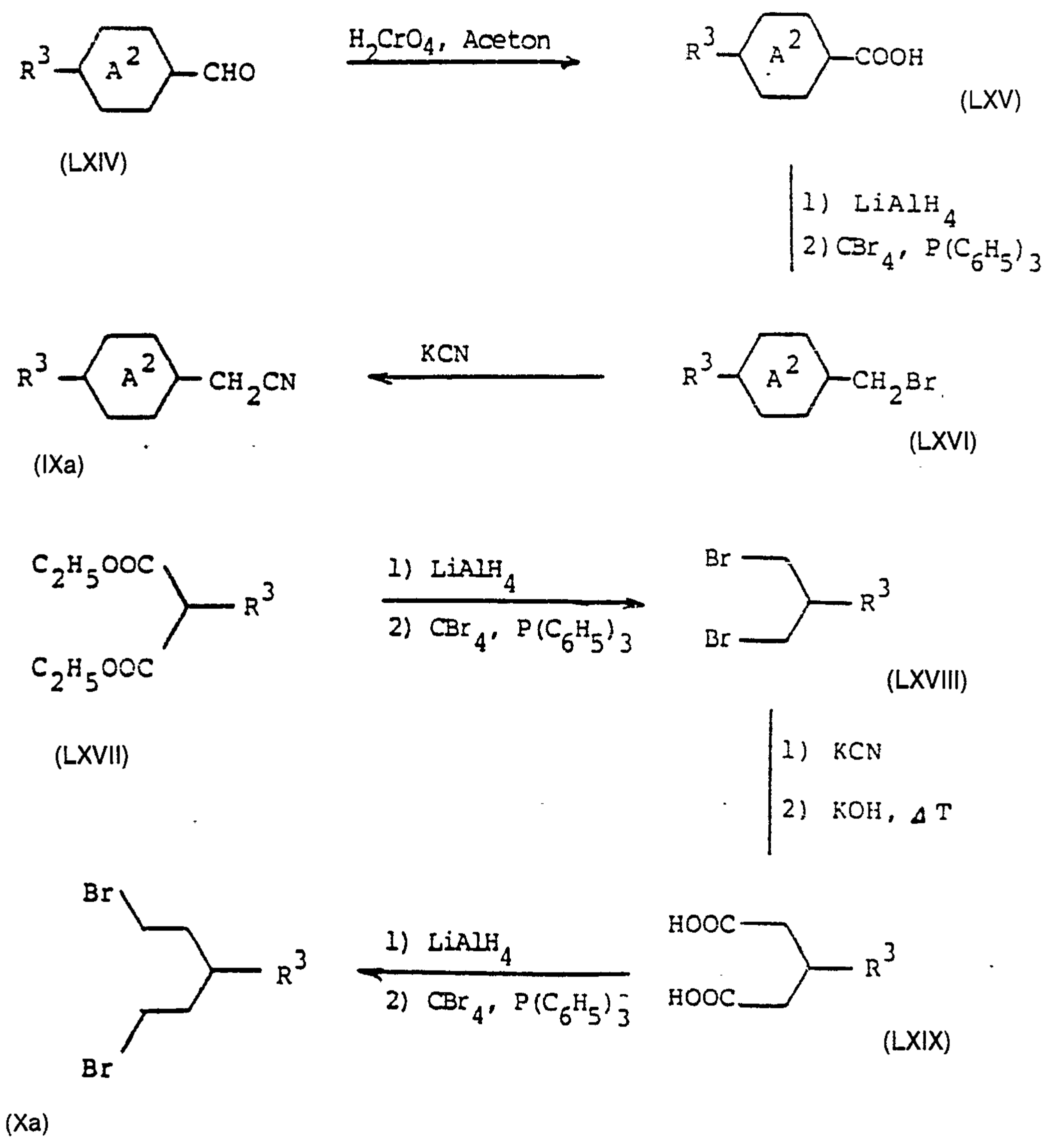


(LXIV) (LXV) (IXa) (LXVI) (LXVII) (LXVIII) (LXIX) (Xa)

In obigen Reaktionen werden in einigen Fällen Gemische von cis/trans-isomeren Cyclohexanderivaten erhalten, welche jedoch in an sich bekannter Weise, z. B. durch Chromatographie und/oder Kristallisation, getrennt werden können. Ferner werden in einigen Fällen Gemische von cis/trans-isomeren Alkenyl-Verbindungen erhalten, insbesondere bei der Einführung der Alkenylgruppe durch Wittig-Reaktion. Diese können ebenfalls nach an sich bekannten Methoden getrennt werden, z. B. durch Chromatographie. Hierbei hat sich insbesondere mit Silbernitrat imprägniertes Kieselgel als vorteilhaft erwiesen. Gewünschtenfalls kann das unerwünschte Alkenylisomere in das gewünschte Isomere übergeführt werden, beispielsweise durch Umsetzung mit p-Toluolsulfinsäure oder via das Epoxid, z. B. durch Umsetzung mit m-Chlorperbenzoesäure in Gegenwart von Kaliumcarbonat, Halogenierung des Epoxids mit Triphenylphosphin-Brom in Benzol und Reduktion des Halogenids mit Zink in Eisessig.

Weitere Alkenylderivate zu den Schemata 3, 7, 8 und 10 und weitere Verbindungen der Formel IX können nach Kettenverlängerung am Aldehyd nach der in Schema 2 dargestellten Methode erhalten werden.

Die unter Formel III fallenden Phenole, worin $R^1$ eine Alkenylgruppe bedeutet, können aus den entsprechenden Phenolen erhalten werden, welche anstelle des Restes $R^1$ eine Formylgruppe aufweisen, z. B. durch Einführung einer 2-Methoxyäthoxymethyl-Schutzgruppe, Einführung der Alkenylgruppe durch Wittig-Reaktion gegebenenfalls nach vorheriger Kettenverlängerung (in analoger Weise zu Schema 2) und schliesslich Abspaltung der Schutzgruppe mittels Zinkbromid.

Die Verbindungen der Formel LXVII (Schema 12), worin $R^3$ Alkyl oder trans-1-Alkenyl bedeutet, sind bekannte oder Analoge bekannter Verbindungen. Die übrigen Verbindungen der Formel LXVII können aus Malonsäurediäthylester z. B. durch Umsetzung mit Lithiumdiisopropylamid und Alkenylbromid erhalten werden.

Die erfindungsgemässen Verbindungen können in Form ihrer Gemische untereinander oder mit anderen Flüssigkristallen verwendet werden, wie z. B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Terphenyle, Phenylcyclohexane, Cyclohexylbiphenyle, Phenylpyrimidine, Diphenylpyrimidine, Cyclohexyl-phenylpyrimidine, Phenyldioxane, Phenylpyridazine, 2,3-Dicyano-1,4-phenylen-Derivate und dergleichen. Derartige Substanzen sind dem Fachmann bekannt und viele davon sind zudem im Handel erhältlich.

Die erfindungsgemässen Mischungen enthalten bevorzugt etwa 1-90 Gew.-% und besonders bevorzugt etwa 10-50 Gew.-% an Verbindungen der Formel I.

Vorzugsweise werden die erfindungsgemässen Verbindungen für Mischungen mit negativer dielektrischer Anisotropie oder für Mischungen, welche zur Zwei-Frequenz-Ansteuerung geeignet sind, verwendet. Die Herstellung derartiger Mischungen ist an sich bekannt.

Die erfindungsgemässen Mischungen für die Zwei-Frequenz-Matrix-Adressierung enthalten vorzugsweise eine Komponente A mit einer dielektrischen Anisotropie unterhalb —2 und eine Komponente B, welche eine dielektrische Anisotropie oberhalb + 10, einen Klärpunkt von mindestens 100 °C und eine Cross-over-Frequenz im Gesamtgemisch von höchstens 15 kHz bei 20 °C aufweist. Der Anteil an Komponente A beträgt vorzugsweise etwa 5-85 Gew.-% und der Anteil an Komponente B vorzugsweise etwa 3-30 Gew.-%. Die erfindungsgemässen Mischungen für die Zwei-Frequenz-Matrix-Adressierung können weitere Verbindungen enthalten, vorzugsweise eine Komponente C, welche eine Viskosität (bulk viscosity) von höchstens 40 cP, einen Klärpunkt von mindestens 40 °C und eine dielektrische Anisotropie zwischen —2 und + 2 aufweist. Der Anteil an Komponente C beträgt zweckmässig etwa 0-70 Gew.-% und bevorzugt etwa 10-50 Gew.-%. Komponente A, B und C bestehen jeweils aus einer oder mehreren Verbindungen und Komponente A enthält mindestens eine Verbindung der obigen Formel I.

Komponente A kann neben einer oder mehrerer Verbindungen der Formel I weitere Verbindungen enthalten, beispielsweise Verbindungen der allgemeinen Formel

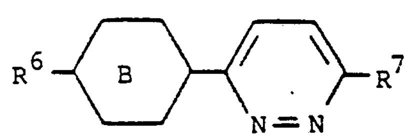

(LXX)

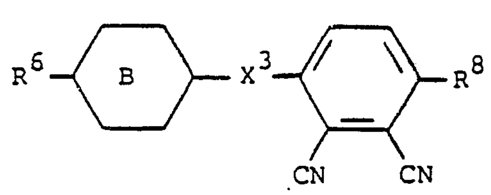

(LXXI)

worin Ring B 1,4-Phenylen oder trans-1,4-Cyclohexylen darstellt; $X^3$ eine einfache Kovalenzbindung, $—CH_2CH_2—$ oder —COO— bezeichnet; $R^6$ und $R^7$ geradkettiges $C_1$-$C_7$-Alkyl bedeuten; und $R^8$ geradkettiges $C_1$-$C_7$-Alkyl oder geradkettiges $C_1$-$C_6$-Alkoxy darstellt.

Komponente B enthält vorzugsweise mindestens eine Verbindung der allgemeinen Formel

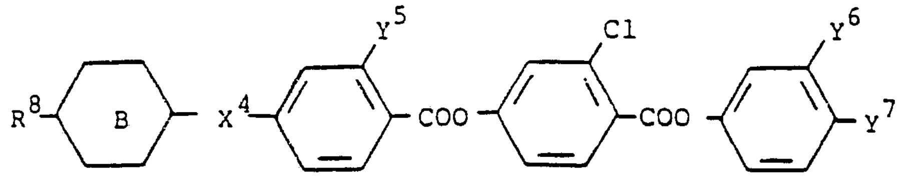

(LXXII)

worin Ring B 1,4-Phenylen oder trans-1,4-Cyclohexylen darstellt ; $X^4$ eine einfache Kovalenzbindung, $—CH_2CH_2—$, —COO— oder 1,4-Phenylen bezeichnet ; $R^8$ geradkettiges $C_1$-$C_7$-Alkyl oder an einem Benzolring auch geradkettiges $C_1$-$C_6$-Alkoxy bedeutet ; $Y^5$ für Wasserstoff oder Chlor steht ; und $Y^6$ und $Y^7$ Cyano, Nitro, Fluor, Chlor oder Brom bedeuten oder $Y^6$ Wasserstoff und $Y^7$ 2,2-Dicyanovinyl oder 2,2-Dicyano-1-methylvinyl bedeuten.

Verbindungen und Mischungen, die sich als Komponente C eignen, sind in grosser Zahl bekannt. vorzugsweise enthält Komponente C eine oder mehrere Verbindungen, der folgenden allgemeinen Formeln

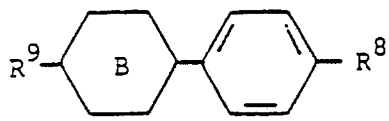

(LXXIII)

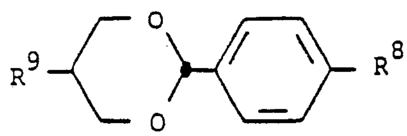

(LXXIV)

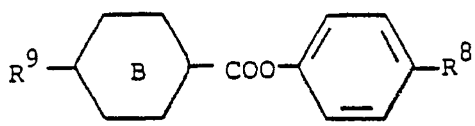

(LXXV)

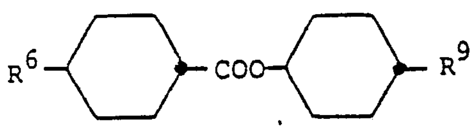

(LXXVI)

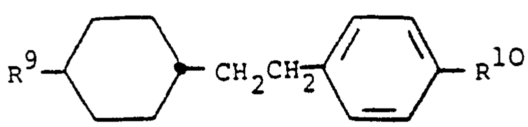

(LXXVII)

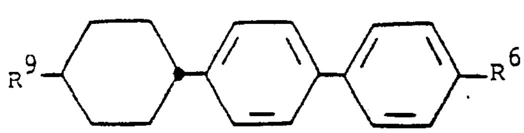

(LXXVIII)

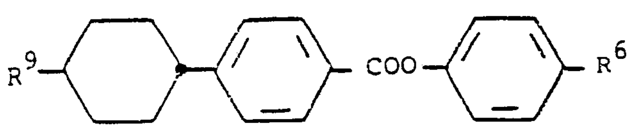

(LXXIX)

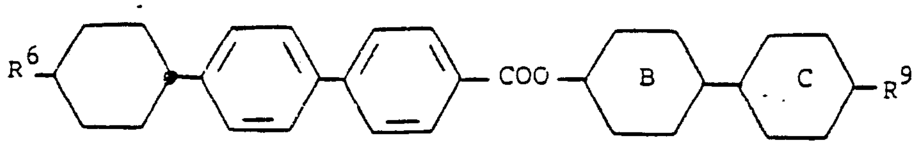

(LXXX)

worin Ring B und C 1,4-Phenylen oder trans-1,4-Cyclohexylen darstellen ; $R^6$ geradkettiges $C_1$-$C_7$-Alkyl bedeutet ; $R^8$ für geradkettiges $C_1$-$C_7$-Alkyl oder geradkettiges $C_1$-$C_6$-Alkoxy steht ; $R^9$ geradkettiges $C_1$-$C_7$-Alkyl, geradkettiges $C_4$-$C_7$-trans-3-Alkenyl oder an einem Cyclohexan- oder Dioxanring auch geradkettiges $C_2$-$C_7$-trans-1-Alkenyl bezeichnet ; $R^{10}$ Alkyl, Alkoxy, p-Alkylphenyl, p-Alkoxyphenyl, trans-4-Alkylcyclohexyl, 4'-Alkyl-4-biphenylyl, p-(trans-4-Alkylcyclohexyl) phenyl, 2-(trans-4-Alkylcyclohexyl) äthyl, p-[2-(trans-4-Alkylcyclohexyl) äthyl]-phenyl oder 2-[p-trans-4-Alkylcyclohexyl) phenyl] äthyl darstellt, und die Alkyl- und Alkoxyreste in $R^{10}$ geradkettige Reste mit 1 bis 7 Kohlenstoffatomen sind.

Die erfindungsgemässen Mischungen mit negativer dielektrischer Anisotropie enthalten neben einer oder mehreren Verbindungen der Formel I vorzugsweise eine oder mehrere Verbindungen der obigen Formeln LXX, LXXI und LXXIII-LXXX.

Die erfindungsgemässen Mischungen können ebenfalls optisch aktive Verbindungen (beispielsweise optisch aktive Biphenyle) und/oder dichroitische Farbstoffe (beispielsweise Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-% im Gesamtgemisch.

Die Herstellung der erfindungsgemässen Mischungen kann in an sich bekannter Weise erfolgen, z. B. durch Erhitzen einer Mischung der Bestandteile auf eine Temperatur knapp oberhalb des Klärpunktes und anschliessendes Abkühlen. Die Herstellung einer elektro-optischen Vorrichtung kann ebenfalls in an sich bekannter Weise erfolgen, z. B. durch Evakuieren einer geeigneten Zelle und Einbringen der Mischung in die evakuierte Zelle.

Die Verbindungen der Formeln LXXIII-LXXX, worin $R^9$ Alkenyl bedeutet, sind neu. Sie können in analoger Weise zu dem bei der Herstellung der Verbindungen der Formel I beschriebenen Methoden erhalten werden. Die übrigen Verbindungen der Formeln LXXIII-LXXX und die Verbindungen der Formeln LXX-LXXII sind bekannte oder analoge bekannter Verbindungen.

Die folgenden Mischungen 1-6 sind Beispiele bevorzugter erfindungsgemässer Mischungen. $V_0$ bezeichnet die homöotrope Schwellenspannung, $\Delta\varepsilon$ die dielektrische Anisotropie und $\Delta n$ die optische Anisotropie gemessen bei 22 °C.

Mischung 1

8,54 Gew.-% trans-4-Butylcyclohexencarbonsäure-p-äthoxyphenylester,
7,54 Gew.-% trans-4-Pentylcyclohexancarbonsäure-p-methoxyphenylester,
9,92 Gew.-% 4-Aethoxy-1-[2-(trans-4-pentylcyclohexyl) äthyl] benzol,
11,00 Gew.-% 1-[2-(trans-4-Butylcyclohexyl) äthyl]-4-(trans-4-pentylcyclohexyl) benzol,
7,00 Gew.-% 4-[2-(trans-4-Butylcyclohexyl) äthyl]-4'-(trans-4-pentylcyclohexyl)-1,1'-äthylendibenzol,
20,00 Gew.-% r-1-Cyan-1-(3-butenyl)-cis-4-(trans-4-butylcyclohexyl) cyclohexan,
10,00 Gew.-% r-1-Cyan-1-(3-butenyl)-cis-4-(trans-4-pentylcyclohexyl) cyclohexan,
26,00 Gew.-% r-1-Cyan-1-(3-butenyl)-cis-4-(trans-4-heptylcyclohexyl) cyclohexan ;
Smp. < — 20 °C, Klp. 73 °C, nematisch ; $\Delta\varepsilon = -4$, $V_0 = 1{,}75$ V ; $\Delta n = 0{,}06$.

Mischung 2

7,8 Gew.-% trans-4-Butylcyclohexancarbonsäure-p-äthoxyphenylester,
3,7 Gew.-% trans-4-Butylcyclohexancarbonsäure-p-pentyloxyphenylester,
7,7 Gew.-% trans-4-Pentylcyclohexancarbonsäure-p-methoxyphenylester,
13,0 Gew.-% 1-[2-(trans-4-Butylcyclohexyl) äthyl]-4-(trans-4-pentylcyclohexyl) benzol,
9,0 Gew.-% 4-[2-(trans-4-Butylcyclohexyl) äthyl]-4'-(trans-4-pentylcyclohexyl)-1,1'-äthylendibenzol,
17,7 Gew.-% r-1-Cyan-1-(3-butenyl)-cis-4-(trans-4-butylcyclohexyl) cyclohexan,
6,1 Gew.-% r-1-Cyan-1-(3-butenyl)-cis-4-(trans-4-pentylcyclohexyl) cyclohexan,
35,0 Gew.-% r-1-Cyan-1-(3-butenyl)-cis-4-[2-(trans-4-propylcyclohexyl) äthyl] cyclohexan ;
Smp. < — 20 °C, Klp. 71 °C, nematisch ; $\Delta\varepsilon = -3{,}8$, $V_0 = 1{,}85$ V , $\Delta n = 0{,}05$.

Mischung 3

11,0 Gew.-% trans-4-Butylcyclohexancarbonsäure-p-äthoxyphenylester,
10,0 Gew.-% trans-4-Butylcyclohexancarbonsäure-p-pentyloxyphenylester,
10,0 Gew.-% trans-4-Pentycyclohexancarbonsäure-p-methoxyphenylester,
9,5 Gew.-% trans-4-Pentylcyclohexancarbonsäure-p-propyloxyphenylester,
10,0 Gew.-% 1-[2-(trans-4-Butylcyclohexyl) äthyl]-4-(trans-4-pentylcyclohexyl) benzol,
4,0 Gew.-% 4-[2-trans-4-Butylcyiclohexyl) äthyl]-4'-(trans-4-pentylcyclohexyl) biphenyl,
5,0 Gew.-% 4-[2-(trans-4-Butylcyclohexyl) äthyl]-4'-(trans-4-pentylcyclohexyl)-1,1'-äthylendibenzol,
6,5 Gew.-% 4-Butyl-1-[2-(trans-4-butylcyclohexyl) äthyl]-2,3-dicyanobenzol,
7,5 Gew.-% 4-Butyl-1-[2-(trans-4-pentylcyclohexyl) äthyl]-2,3-dicyanobenzol,
26,5 Gew.-% r-1-Cyan-1-(3-butenyl)-cis-4-(trans-4-butylcyclohexyl) cyclohexan ;
Klp. 67 °C, nematisch.

Mischung 4

7,79 Gew.-% trans-4-Butylcyclohexancarbonsäure-p-äthoxyphenylester,
3,64 Gew.-% trans-4-Butylcyclohexancarbonsäure-p-pentyloxyphenylester,
7,77 Gew.-% trans-4-Pentylcyclohexancarbonsäure-p-methoxyphenylester,
13,00 Gew.-% 1-[2-(trans-4-Butylcyclohexyl) äthyl]-4-(trans-4-pentylcyclohexyl) benzol,
9,00 Gew.-% 4-[2-(trans-4-Butylcyclohexyl) äthyl]-4'-(trans-4-pentylcyclohexyl)-1,1'-äthylendibenzol,
14,00 Gew.-% r-1-Cyan-1-(3-butenyl)-cis-4-(trans-4-butylcyclohexyl) cyclohexan,
4,80 Gew.-% r-1-Cyano-1-(3-butenyl)-cis-4-trans-4-pentylcyclohexyl) cyclohexan,
40,00 Gew.-% r-1-Cyan-1-(3-butenyl)-cis-4-[2-(trans-4-propylcyclohexyl) äthyl] cyclohexan ;
Smp. <—20 °C, Klp. 70 °C, nematisch; $\Delta\varepsilon = -3{,}82$, $V_0 = 1{,}85$ V ; $\Delta n = 0{,}055$.

Mischung 5

2,80 Gew.-% trans-4-Butylcyclohexancarbonsäure-p-äthoxyphenylester,
2,59 Gew.-% trans-4-Pentylcyclohexancarbonsäure-p-methoxyphenylester,
9,00 Gew.-% 4-Aethoxy-1-[2-(trans-4-pentylcyclohexyl) äthyl] benzol,
14,20 Gew.-% 1-[2-(trans-4-Butylcyclohexyl) äthyl]-4-(trans-4-pentylcyclohexyl) benzol,
9,77 Gew.-% 4-[2-(trans-4-Butylcyclohexyl) äthyl]-4'-(trans-4-pentylcyclohexyl)-1,1'-äthyldibenzol,
14,00 Gew.-% r-1-Cyan-1-(3-butenyl)-cis-4-(trans-4-butylcyclohexyl) cyclohexan,
5,54 Gew.-% r-1-Cyan-1-(3-butenyl)-cis-4-(trans-4-pentylcyclohexyl) cyclohexan,
42,10 Gew.-% r-1-Cyan-1-[2-(trans-4-propylcyclohexyl) äthyl]-cis-4-(3-butenyl) cyclohexan ;
Smp. <—20 °C, Klp. 64 °C, nematisch; $\Delta\varepsilon = -4{,}04$, $V_0 = 1{,}8$ V ; $\Delta n = 0{,}05$.

Mischung 6

6,90 Gew.-% trans-4-Butylcyclohexancarbonsäure-p-äthoxyphenylester,
6,42 Gew.-% trans-5-Pentylcyclohexancarbonsäure-p-methoxyphenylester,
19,23 Gew.-% 4-Aethoxy-1-[2-(trans-4-pentylcyclohexyl) äthyl] benzol,
11,55 Gew.-% 1-[2-(trans-4-Butylcyclohexyl) äthyl]-4-(trans-4-pentylcyclohexyl) benzol,
6,79 Gew.-% 4-Butyl-1-[2-(trans-4-butylcyclohexyl) äthyl]-2,3-dicyanobenzol,
8,55 Gew.-% 4-Butyl-1-[2-(trans-4-pentylcyclohexyl) äthyl]-2,3-dicyanobenzol,
25,14 Gew.-% r-1-Cyan-1-(3-butenyl)-cis-4-(trans-4-butylcyclohexyl) cyclohexan,
15,42 Gew.-% r-1-Cyan-1-(3-butenyl)-cis-4-(4'-pentyl-4-biphenylyl) cyclohexan ;
Klp. 52 °C, nematisch.

Die Herstellung der Verbindungen der Formel I wird durch die folgenden Beispiele weiter veranschaulicht. C bedeutet eine kristalline, S eine smektische, N eine nematische und I die isotrope Phase.

## Beispiel 1

Eine Lösung von 1,157 g Diisopropylamin in 95 ml absolutem Tetrahydrofuran wurde unter Rühren und Stickstoffbegasung bei —74 °C bis —69 °C innert 2 Minuten tropfenweise mit 7,0 ml einer 1,6 M Lösung von Butyllithium in Hexan versetzt. Das Gemisch wurde 15 Minuten bei dieser Temperatur gerührt, dann bei —50 °C innert 3 Minuten tropfenweise mit einer Lösung von 2,5 g 4'-Pentyl-4-bicyclohexylcarbonitril in 8,5 ml absolutem Tetrahydrofuran versetzt und noch 30 Minuten bei —50 °C gerührt. Danach wurde das Gemisch innert 7 Minuten tropfenweise mit einer Lösung von 1,291 g 4-Brom-1-buten in 8,5 ml absolutem Tetrahydrofuran versetzt und noch 1,5 Stunden bei —50 °C und anschliessend 1,5 Stunden bei Raumtemperatur gerührt. Dann wurde das Reaktionsgemisch auf 190 ml gesättigte Ammoniumchlorid-Lösung gegossen und mit Diäthyläther extrahiert. Die organischen Phasen wurden mit Wasser gewaschen, über Natriumsulfat getrocknet und im Vakuum vom Lösungsmittel befreit. Das zurückbleibende, gelbe, flüssigkristalline Oel (2,982 g) wurde an 90 g Kieselgel in Hexan chromatographiert. Hexan/Toluol (Vol. 9 : 1, 8 : 2 und 7 : 3) eluierte r-1-Cyan-1-(3-butenyl)-cis-4-(trans-4-pentylcyclohexyl) cyclohexan als farbloses, flüssigkristallines Oel, welches beim Abkühlen kristallisierte. Umkristallisation aus Methanol ergab farblose Kristalle mit Smp. (C-N) 26,0 °C und Klp. (N-I) 61 °C.

In analoger Weise wurden folgende Vebindungen hergestellt :

r-1-Cyan-1-(3-butenyl)-cis-4-(trans-4-äthylcyclohexyl)-cyclohexan ; Smp. (C-I) 23,0 ° bzw. 25,6 °C (2 Modifikationen), Klp. (N-I) 13,8 °C,

r-1-Cyan-1-(3-butenyl)-cis-4-(trans-4-propylcyclohexyl)-cyclohexan ; Smp. (C-N) 31,4 °C, Klp. (N-I) 48 °C,

r-1-Cyan-1-(3-butenyl)-cis-4-(trans-4-butylcyclohexyl)-cyclohexan ; Smp. (C-N) 29,5 °C, Klp. (N-I) 51,2 °C,

r-1-Cyan-1-(3-butenyl)-cis-4-(trans-4-heptylcyclohexyl)-cyclohexan ; Smp. (C-S) 21,5 °C, Umwandlung (S-N) 27 °C, Klp. (N-I) 63,5 °C,

r-1-Cyan-1-(3E-pentenyl)-cis-4-(trans-4-pentylcyclohexyl) cyclohexan ; Smp. (C-N) 43,0 °C, Klp. (N-I) 68,6 °C,

r-1-Cyano-1-(4-pentenyl)-cis-4-(trans-4-pentylcyclohexyl) cyclohexan ; Smp. (C-S) 4,6 °C, Klp. (S-I) 21,9 °C,

r-1-Cyan-1-(3-butenyl)-cis-4-[2-(trans-4-butylcyclohexyl) äthyl] cyclohexan ; Smp. (C-N) 30,5 °C, Klp. (N-I) 44,5 °C,

r-1-Cyan-1-(3-butenyl)-cis-4-[2-(trans-4-propylcyclohexyl) äthyl] cyclohexan ; Smp. (C-N) 10,1 °C bzw. 12,3 °C (2 Modifikationen), Klp. (N-I) 40,1 °C,

r-1-Cyan-1-(3-butenyl)-cis-4-[2-(trans-4-pentylcyclohexyl) äthyl] cyclohexan ; Smp. (C-N) 27,1 °C, Klp. (N-I) 54,1 °C,

r-1-Cyan-1-(3-butenyl)-cis-4-(4-pentylphenyl) cyclohexan ; Smp. <20 °C, Sdp. (0,1 mbar) 155-160 °C,

r-1-Cyan-1-(3-butenyl)-cis-4-(4'-pentyl-4-biphenylyl)-cyclohexan ; Smp. (C-N) 55,6 °C, Klp. (N-I) 134,7 °C.

## Beispiel 2

Eine Lösung von 5,933 g Diisopropylamin in 480 ml absolutem Tetrahydrofuran wurde unter Rühren und Stickstoffbegasung bei — 72 °C bis — 70 °C innert 11 Minuten tropfenweise mit 35,5 ml einer 1,6 M Lösung von Butyllithium in Hexan versetzt. Das Gemisch wurde 15 Minuten bei dieser Temperatur gerührt, dann bei — 50 °C innert 8 Minuten tropfenweise mit einer Lösung von 8,0 g 4-(3-Butenyl) cyclohexancarbonitril in 45 ml absolutem Tetrahydrofuran versetzt (Nachspülen mit 6 ml absolutem Tetrahydrofuran) und noch 15 Minuten bei — 50 °C gerührt. Danach wurde das Gemisch innert 10 Minuten tropfenweise mit einer Lösung von 11,42 g trans-4-Propyl-1-(2-bromäthyl) cyclohexan in 45 ml absolutem Tetrahydrofuran versetzt und noch 1,5 Stunden bei — 50 °C und anschliessend 1,5 Stunden bei Raumtemperatur gerührt. Dann wurde das Reaktionsgemisch auf 1 l gesättigte Ammoniumchlorid-Lösung gegossen und mit Diäthyläther extrahiert. Die organischen Phasen wurden mit Wasser gewaschen, über Natriumsulfat getrocknet und am Vakuum vom Lösungsmittel befreit. Das zurückbleibende, gelbe Oel (16,1 g) wurde an 300 g Kieselgel in Hexan chromatographiert. Hexan/Toluol (Vol. 19 : 1, 9 : 1, 8 : 2 und 7 : 3) eluierte r-1-Cyan-1-[2-(trans-4-propylcyclohexyl) äthyl]-cis-4-(3-butenyl) cyclohexan als gelbliches Oel, welches beim Abkühlen flüssigkristallin wurde und später kristallisierte. Umkristallisation aus 2-Propanol ergab reines Produkt mit Smp. (C-N) 5,3 °C und Klp. (N-I) 29,3 °C.

Das als Ausgangsmaterial verwendete 4-(3-Butenyl) cyclohexancarbonitril wurde wie folgt hergestellt :

a) Eine Suspension von 133,7 g Methoxymethyl-triphenylphosphoniumchlorid in 1 200 ml absolutem Diäthyläther wurde unter Rühren und Stickstoffbegasung bei Raumtemperatur mit 46,6 g gestem Kalium-t-butylat versetzt und noch 1 Stunde bei Raumtemperatur gerührt. Dann wurde das Gemisch auf — 60 °C abgekühlt und innert 20 Minuten tropfenweise mit einer Lösung von 35,7 g 4-Cyanocyclohexancarboxaldehyd in 200 ml absolutem Tetrahydrofuran versetzt. Das Reaktionsgemisch wurde innert 2,5 Stunden auf Raumtemperatur erwärmen gelassen und dann am Rotationsverdampfer bei 50 °C vom Lösungsmittel befreit. Das zurückbleibende, rotbraune, zähflüssige Oel wurde in Methylenchlorid aufgenommen und zweimal mit Wasser gewaschen. Die wässrigen Phasen wurden zweimal mit Methylenchlorid nachextrahiert. Die organischen Phasen wurden über Natriumsulfat getrocknet und am Vakuum eingedampft. Der Rückstand wurde durch Umkristallisation aus Toluol/Hexan teilweise von Triphenylphosphinoxid befreit. Das Filtrat wurde an 600 g Kieselgel in Hexan/Toluol (Vol. 1 :1) chromatographiert. Toluol/Hexan (Vol. 1 : 1), Toluol und Toluol/Aceton (Vol. 97,5 : 2,5) eluierten 39,330 g 4-(2-Methoxyvinyl) cyclohexancarbonitril als gelbliche Flüssigkeit.

b) Ein Gemisch von 39,330 g 4-(2-Methoxyvinyl) cyclohexancarbonitril, 387 ml Tetrahydrofuran und 97 ml 2N Salzsäure wurde unter Rühren und Stickstoffbegasung 1 Stunde zum Rückfluss erhitzt. Nach dem Abkühlen wurde das Reaktionsgemisch auf 900 ml Wasser gegossen und dreimal mit Methylenchlorid extrahiert. Die organischen Phasen wurden zweimal mit Wasser gewaschen, über Natriumsulfat getrocknet und am Vakuum vom Lösungsmittel befreit. Es resultierten 34,9 g 4-Cyanocyclohexylacetaldehyd als gelbliche Flüssigkeit, welcher ohne zusätzliche Reinigung weiter umgesetzt wurde.

c) Eine Suspension von 118,5 g Methoxymethyl-triphenylphosphoniumchlorid in 1 000 ml absolutem Diäthyläther wurde unter Rühren und Stickstoffbegasung bei Raumtemperatur mit 41,3 g festem Kalium-t-butylat versetzt und noch 1 Stunde bei Raumtemperatur gerührt. Dann wurde das Gemisch auf — 30 °C abgekühlt und innert 18 Minuten tropfenweise mit einer Lösung von 34,9 g 4-Cyanocyclohexyl-acetaldehyd in 200 ml absolutem Diäthyläther versetzt. Das Reaktionsgemisch wurde innert 2 Stunden auf Raumtemperatur erwärmen gelassen und dann am Rotationsverdampfer bei 50 °C vom Lösungsmittel befreit. Das zurückbleibende, braune Oel wurde in Methylenchlorid aufgenommen und zweimal mit Wasser gewaschen. Die wässrigen Phasen wurden zweimal mit Methylenchlorid nachextrahiert. Die organischen Phasen wurden über Natriumsulfat getrocknet und am Vakuum eingedampft. Der Rückstand wurde durch Umkristallisation aus Toluol/Hexan teilweise von Triphenylphosphinoxid befreit. Das Filtrat wurde an 400 g Kieselgel in Hexan/Toluol (Vol. 1 : 1) chromatographiert. Toluol/Hexan (Vol. 1 : 1), Toluol und Toluol/Aceton (Vol. 97,5 : 2,5) eluierten 36,297 g 4-(3-Methoxy-2-propenyl) cyclohexancarbonitril als gelbliche Flüssigkeit.

d) Ein Gemisch von 36,297 g 4-(3-Methoxy-2-propenyl)-cyclohexancarbonitril, 330 ml Tetrahydrofuran und 83 ml 2N Salzsäure wurde unter Rühren und Stickstoffbegasung 30 Minuten zum Rückfluss erhitzt. Nach dem Abkühlen wurde das Reaktionsgemisch auf 765 ml Wasser gegossen und dreimal mit Methylenchlorid extrahiert. Die organischen Phasen wurden zweimal mit Wasser gewaschen, über Natriumsulfat getrocknet und am Vakuum von Lösungsmittel befreit. Es resultierten 32,7 g 3-(4-Cyanocyclohexyl) propionaldehyd als leicht bräunliches Oel, welcher ohne zusätzliche Reinigung weiter umgesetzt wurde.

e) Eine Suspension von 27,9 g Methyl-triphenylphosphonium bromid in 350 ml absolutem t-Butylmethyläther wurde unter Rühren und Stickstoffbegasung bei Raumtemperatur mit 8,8 g festem Kalium-t-butylat versetzt und noch 1 Stunde bei Raumtemperatur gerührt. Anschliessend wurde das Gemisch innert 16 Minuten tropfenweise mit einer Lösung von 8,6 g 3-(4-Cyanocyclohexyl) propionaldehyd in 90 ml absolutem t-Butylmethyläther versetzt und die gelbe Suspension noch 2 Stunden bei Raumtemperatur weiter gerührt. Dann wurde das Reaktionsgemisch am Rotationsverdampfer bei 50 °C vom Lösungsmittel befreit. Der Rückstand wurde in 200 ml Methanol/Wasser (Vol. 6 : 4) aufgenommen und einmal mit 200 ml Hexan und zweimal mit je 100 ml Hexan extrahiert. Die Extrakte wurden einmal mit 130 ml Methanol/Wasser (Vol. 6 : 4) und zweimal mit Wasser gewaschen, über Natriumsulfat getrocknet und am Vakuum vom Lösungsmittel befreit. Das erhaltene 4-(3-Butenyl) cyclohexancarbonitril wurde ohne weitere Reinigung verwendet.

Das ebenfalls als Ausgangsmaterial verwendete trans-4-Propyl-1-(2-bromäthyl) cyclohexan wurde in analoger Weise zum nachstehend beschriebenen Pentylderivat hergestellt :

f) Zu einer Suspension von 12,5 g Lithiumaluminiumhydrid in 750 ml absolutem Diäthyläther wurde eine Lösung von 76,1 g (trans-4-Pentylcylclohexyl) acetylchlorid in 500 ml absolutem Diäthyläther unter Rühren so zugetropft, dass das Reaktionsgemisch nicht zu stark siedete. Das Gemisch wurde noch 2 Stunden gerührt und dann tropfenweise zuerst mit 30 ml Aceton und danach mit 75 ml Wasser versetzt. Anschliessend wurde das Reaktionsgemisch mit 3N Salzsäure angesäuert, die organische Phase abgetrennt und die wässrige Phase mit 200 ml Diäthyläther nachextrahiert. Die vereinigte organische Phase wurde mit Wasser neutralgewaschen, über Natriumsulfat getrocknet und eingeengt, wobei 62,6 g 2-(trans-4-Pentylcyclohexyl) äthanol als gelbliche Flüssigkeit erhalten werden.

g) Zu einer Lösung von 49,6 g 2-(trans-4-Pentylcyclohexyl) äthanol und 71,9 g Triphenylphosphin in 160 ml Dichlormethan wurden unter Rühren und Kühlung mit einem Eisbad innert 30 Minuten 50,1 g N-Bromsuccinimid portionenweise so zugegeben, dass die Temperatur nicht über 30 °C anstieg. Anschliessend wurde das Gemisch noch 3 Stunden gerührt, dann die Suspension genutscht und das Filtrat am Rotationsverdampfer eingeengt. Der noch warme Rückstand (2 Phasen) wurde unter Schütteln langsam mit 200 ml Hexan versetzt. Der feinkörnige, orange Niederschlag wurde abgenutscht und mehrmals mit Hexan nachgewaschen. Das Filtrat wurde eingeengt und das erhaltene rohe trans-4-Pentyl-1-(2-bromäthyl) cyclohexan im Vakuum destilliert. Ausbeute 53,3 g Hauptdestillat ; Sdp. 93-99 °C/ca. 0,1 Torr; Reinheit 98,2 %.

In analoger Weise wurden folgende Verbindungen hergestellt :

r-1-Cyan-1-[2-(trans-4-propylcyclohexyl) äthyl]-cis-4-(1E-pentenyl) cyclohexan ; Smp. (C-I) 28,5 °C, Klp. (N-I) 22 °C,

r-1-Cyan-1-[2-(trans-4-propylcyclohexyl) äthyl)-cis-4-(3E-pentenyl) cyclohexan ; Smp. (C-N) 26,5 °C, Klp. (N-1) 41,8 °C,

r-1-Cyan-1-[2-(trans-4-propylcyclohexyl) äthyl]-cis-4-(4-pentenyl) cyclohexan ; Smp. (C-I) 25,3 °C.

## Beispiel 3

a) In einem Sulfierkolben mit Thermometer, mechanischem Rührer, Tropftrichter und Festsubstanzzugaberohr wurden unter Argonbegasung 10,4 g Triphenyl-methoxymethyl-phosphoniumchlorid in 60 ml t-Butylmethyläther aufgeschlämmt und bei — 10 °C innert 10 Minuten mit 3,6 g festem Kalium-t-butylat versetzt. Nach beendeter Zugabe wurde noch während 30 Minuten bei — 10 °C bis 0 °C gerührt und dann das tieforange, heterogene Reaktionsgemisch bei 0 °C tropfenweise mit einer Lösung von 4,2 g 4-(p-Cyanophenyl) cyclohexanon in 50 ml absolutem Tetrahydrofuran versetzt. Anschliessend wurde das Reaktionsgemisch noch 2 Stunden bei Raumtemperatur gerührt, dann auf 500 ml Hexan gegossen und filtriert. Niederdruckchromatographie (0,5 bar) des eingeengten Rückstandes (7,1 g) an Kieselgel mit Essigester/Petroläther (Vol. 5 : 95) ergab 4,5 g (94 %) p-[4-(Methoxymethylen) cyclohexyl] benzonitril als farbloses Oel; Reinheit 95 %, Rf-Wert (Essigester/Petroläther Vol. 1 : 9) 0,30.

b) In einem Rundkolben wurde ein Gemisch von 4,2 g p-[4-Methoxymethylen) cyclohexyl] benzonitril und 100 ml Tetrahydrofuran/2N Salzsäure (Vol. 4 : 1) während 30 Minuten zum Rückfluss erhitzt. Anschliessend wurde das Reaktionsgemisch auf 100 ml Wasser gegossen und dreimal mit je 100 ml Diäthyläther extrahiert. Die organischen Phasen wurden noch einmal mit 100 ml Wasser gewaschen, über Magnesiumsulfat getrocknet und eingeengt. Es resultierten 3,9 g (100 %) 4-(p-Cyanophenyl) cyclohexancarboxaldehyd als farbloses Oel, welches ohne weitere Reinigung in der nächsten Stufe eingesetzt wurde; trans/cis-Verhältnis ca. 3 : 1, Rf-Wert (Essigester/Petroläther Vol. 3 : 7) 0,41. Durch Kristallisation aus Hexan konnte reiner trans-4-(p-Cyanophenyl) cyclohexancarboxaldehyd erhalten werden; Smp. 57,1 °C.

Beispiel 4

a) In einem Sulfierkolben wurden unter Rühren und Stickstoffbegasung 149 g Methoxymethyl-triphenylphosphoniumchlorid und 860 ml t-Butylmethyläther bei Raumtemperatur vorgelegt, die Suspension auf — 10 °C gekühlt und innert 10 Minuten mit 51,6 g Kalium-t-butylat versetzt. Die Suspension wurde noch 30 Minuten bei — 10 °C bis 0 °C gerührt und dann innert 45 Minuten bei 0 °C tropfenweise mit einer Lösung von 47,3 g 4,4-Aethylendioxycyclohexanon in 720 ml Tetrahydrofuran versetzt. Die orange Suspension wurde noch 2 Stunden bei Raumtemperatur weiter gerührt, dann auf 5 l Hexan gegossen, 10 Minuten gerührt und genutscht. Das Filtrat wurde im Vakuum eingeengt und das erhaltene gelbräunliche Oel (104,1 g) mit 500 ml Hexan versetzt und genutscht. Das Filtrat wurde im Vakuum eingeengt, wobei 61,7 g gelbbräunliches Oel erhalten wurden. Chromatographische Trennung dieses Rohproduktes an Kieselgel mit Methylenchlorid/Aceton (Vol. 98 : 2 und 95 : 5) ergab schliesslich 53,5 g 1,1-Aethylendioxy-4-(methoxymethylen)-cyclohexan als farbloses Oel.

b) In einem Rundkolben wurde unter Stickstoffbegasung ein Gemisch von 28,2 g 1,1-Aethylendioxy-4-(methoxymethylen)-cyclohexan, 770 ml Eisessig und 385 ml Wasser 1 Stunde zum Rückfluss erhitzt. Danach wurde die gelbliche klare Lösung auf Raumtemperatur gekühlt, mit 800 ml Wasser verdünnt und dreimal mit je 700 ml Methylenchlorid extrahiert. Die organischen Phasen wurden zweimal mit je 500 ml 10 %-iger (Gew.-/Vol.- Natriumcarbonat-Lösung gewaschen, über Natriumsulfat getrocknet, filtriert und eingeengt. Chromatographische Tennung der erhaltenen bräunlichen Flüssigkeit (18,5 g) an Kieselgel mit Methylenchlorid als Eluens ergab schliesslich 16,7 g 4-Formylcyclohexanon als bräunliche Flüssigkeit.

c) In einem Sulfierkolben wurden unter Rühren und Stickstoffbegasung 63,3 g p-Cyanobenzyltriphenylphosphonium-chlorid, 17,2 g Kalium-t-butylat und 195 ml Aethylenglykoldimethyläther vorgelegt, wobei die Innentemperatur bis auf 44 °C anstieg. Die braune Suspension wurde auf 0 °C gekühlt und innert 2 Minuten mit einer Lösung von 16,7 g 4-Formylcyclohexanon in 100 ml Aethylenglykoldimethyläther versetzt. Danach wurde das Kühlbad entfernt und das Reaktionsgemisch noch 3,5 Stunden bei Raumtemperatur gerührt. Anschliessend wurde die Suspension auf 500 ml Wasser gegossen und dreimal mit je 600 ml Methylenchlorid extrahiert. Die organischen Phasen wurden zweimal mit je 500 ml 10 %-iger (Gew./Vol.) Kochsalzlösung gewaschen, über Natriumsulfat getrocknet, filtriert und eingeengt, wobei 76,9 g einer bräunlichen Paste zurückblieben. Chromatographische Trennung dieses Rohproduktes an Kieselgel mit Methylenchlorid als Eluens ergab 33,0 g 4-[2-(p-Cyanophenyl) vinyl] cyclohexanon als gelbbräunliches Oel.

d) In einem Rundkolben mit Magnetrührer wurde ein Gemisch von 33,0 g 4-[2-(p-Cyanophenyl) vinyl] cyclohexanon, 520 ml Toluol, 260 ml Aethanol und 3,2 g Palladium/Kohle (5 %) bei Raumtemperatur vorgelegt und das Gemisch bis zum Stillstand der Wasserstoffaufnahme hydriert. Anschliessend wurde die schwarze Suspension genutscht (Nachwaschen mit Toluol) und das Filtrat im Vakuum eingeengt. Das erhaltene, leicht trübe, gelbliche Oel (34,1 g) wurde an Kieselgel chromatographisch getrennt. Methylenchlorid/Hexan (Vol. 1 : 1), Methylenchlorid/Hexan (Vol. 8 : 2) und Methylenchlorid eluierten 25,6 g gelbliches Oel, welches aus t-Butylmethyläther kristallisiert wurde. Hierbei wurden 22,6 g 4-[2-(p-Cyanophenyl) äthyl] cyclohexanon als farblose Kristalle mit Smp. 62,5-64,3 °C erhalten.

Beispiel 5

a) 261,2 g Methoxymethyl-triphenylphosphoniumchlorid wurden in 550 ml t-Butylmethyläther aufgeschlämmt und bei — 10 °C mit 90,53 g Kalium-t-butylat versetzt. Das Kühlbad wurde entfernt und das Gemisch noch 1 Stunde bei Raumtemperatur gerührt. Anschliessend wurde die Suspension bei — 10 °C langsam mit einer Lösung von 70 g 4,4-Aethylendioxycyclohexanon in 350 ml Tetrahydrofuran versetzt, noch 1 Stunde bei Raumtemperatur gerührt, dann mit Wasser versetzt und dreimal mit Diäthyläther extrahiert. Die organischen Phasen wurden zweimal mit Wasser gewaschen und die wässrigen Phasen mit Diäthyläther nachextrahiert. Die vereinigten organischen Phasen wurden über Magnesiumsulfat getrocknet und eingeengt. Der erhaltene, kristalline Rückstand wurd in Essigester gelöst, mit Petroläther verdünnt, filtriert und vom Lösungsmittel befreit. Destillation des erhaltenen, gelben Oeles (100 g) ergab im Hauptlauf (71 °C/0,20-0,17 Torr) 75,28 g (91,17 %) 1,1-Aethylendioxy-4-(methoxymethylen) cyclohexan als klare, farblose Flüssigkeit.

b) Ein Gemisch von 10,55 g 1,1-Aethylendioxy-4-(methoxymethylen) cyclohexan, 130 ml Wasser und 200 ml Eisessig wurde 1 Stunde zum Rückfluss erhitzt. Anschliessend wurde am Rotationsverdampfer das Lösungsmittel abdestilliert und das Destillat zweimal mit Methylenchlorid extrahiert. Der Destillationsrückstand (gelbes Oel) wurde mit 200 ml Wasser verdünnt, mit Natriumcarbonat-Lösung neutralisiert und dreimal mit Methylenchlorid extrahiert. Die organischen Phasen wurden mit gesättigter Natriumcarbonat-Lösung gewaschen und die wässrigen Phasen mit Methylenchlorid nachextrahiert. Die organischen Phasen wurden über Magnesiumsulfat getrocknet und eingedampft. Destillation des zurückbleibenden, gelben Oeles ergab bei 70 °C/0,15 Torr 6,7 g (93 %) 4-Formylcyclohexanon.

c) Eine Lösung von 36,72 g Triphenylphosphin in 200 ml Methylenchlorid wurde bei — 20 °C langsam mit 23,22 g Tetrabromkohlenstoff versetzt und noch 10 Minuten gerührt. Anschliessend wurde das Gemisch mittels einer Kanüle zu einer auf — 60 °C gekühlten Lösung von 6,30 g 4-Formylcyclohexanon in 100 ml Methylenchlorid zugetropft. Das Reaktionsgemisch wurde noch 15 Minuten bei — 60 °C gerührt und dann in Wasser/Methylenchlorid verteilt. Die wässrigen Phasen wurden noch zweimal mit Methylenchlorid extrahiert. Die organischen Phasen wurden zweimal mit Wasser gewaschen, über Magnesiumsulfat getrocknet und eingedampft. Niederdruckchromatographie des erhaltenen hellgelben Oeles (16 g) an Kieselgel mit Essigester/Petroläther (Vol. 10 : 90) ergab 12,08 g (85,8 %) 4-(2,2-Dibromvinyl) cyclohexanon als hellgelbe Flüssigkeit.

d) Ein Gemisch von 2 g 4-(2,2-Dibromvinyl) cyclohexanon, 3,43 g Aethylenglykol, 0,202 g p-Toluolsulfonsäure und 240 ml Benzol wurde unter Wasserabscheidung 5 Stunden am Rückfluss gekocht. Anschliessend wurde das Reaktionsgemisch mit Kaliumcarbonat versetzt, kurz gerührt und über Nacht stehen gelassen. Danach wurde das Gemisch filtriert und das Filtrat am Rotationsverdampfer vom Lösungsmittel befreit. Der Rückstand wurde in 200 ml Methylenchlorid aufgenommen und zweimal mit verdünnter Natronlauge und einmal mit Wasser gewaschen. Die wässrigen Phasen wurden mit Methylenchlorid nachextrahiert. Die organischen Phasen wurden über Magnesiumsulfat getrocknet, filtriert und eingedampft. Hierbei wurden 14 g 1,1-Aethylendioxy-4-(2,2-dibromvinyl)-cyclohexan als leicht gelbe, kristallisierende Flüssigkeit erhalten.

e) Eine Lösung von 14 g 1,1-Aethylendioxy-4-(2,2-dibromvinyl) cyclohexan in 70 ml Tetrahydrofuran wurde auf — 20 °C gekühlt und bei dieser Temperatur langsam mit 76,62 ml einer 1,4 M Lösung von Butyllithium in Hexan versetzt (exotherme Reaktion). Das Kühlbad wurde entfernt und das Gemisch innert etwa 20 Minuten auf 20 °C erwärmen gelassen. Anschliessend wurde das Reaktionsgemisch mit 150 ml Wasser versetzt und dreimal mit Diäthyläther extrahiert. Die organischen Phasen wurden zweimal mit Wasser gewaschen und die Waschwasser mit Diäthyläther nachextrahiert. Die organischen Phasen wurden über Magnesiumsulfat getrocknet, filtriert und eingedampft. Niederdruckchromatographie der zurückbleibenden, gelben Flüssigkeit (8 g) an Kieselgel mit Essigester/Petroläther (Vol. 7 : 93) ergab 6,5 g (91 %) 4-Aethinyl-1,1-äthylendioxycyclohexan als klare Flüssigkeit.

f) Eine Lösung von 6,5 g 4-Aethinyl-1,1-äthylendioxycyclohexan in 40 ml Tetrahydrofuran wurde bei — 20 °C mit 39,1 ml einer 1,4 M Lösung von Butyllithium in Hexan versetzt. Anschliessend wurde das Gemisch bei 0 °C mit 60 ml Hexamethylphosphorsäuretriamid (kurzer Temperaturanstieg auf 26 °C) und dann tropfenweise mit 6,5 ml Propyljodid versetzt. Das Kühlbad wurde entfernt und das Gemisch auf Raumtemperatur erwärmen gelassen. Hierbei bildete sich ein weisser Niederschlag. Nach 30 Minuten wurde das Reaktionsgemisch mit 150 ml Wasser versetzt und dreimal mit Hexan extrahiert. Die organischen Phasen wurden dreimal mit Wasser gewaschen und die Waschwasser mit Hexan nachextrahiert. Die organischen Phasen wurden über Magnesiumsulfat getrocknet, filtriert und am Rotationsverdampfer vom Lösungsmittel befreit. Niederdruckchromatographie der erhaltenen gelben Flüssigkeit (9 g) an Kieselgel mit Essigester/Petroläther (Vol. 10 : 90) und Behandlung mit Aktivkohle ergab 6,23 g (76,5 %) 1,1-Aethylendioxy-4-(1-pentinyl) cyclohexan als leicht hellgelbe Flüssigkeit.

g) In einem Sulfierkolben mit Magnetrührer wurde eine Lösung von 4,5 g 1,1-Aethylendioxy-4-(1-pentinyl) cyclohexan in 54 ml Tetrahydrofuran tropfenweise mit ca. 50 ml vorkondensiertem Ammoniak versetzt. Anschliessend wurde das Gemisch bei — 78 °C innert 7 Stunden portionenweise mit 1,3 g Natrium versetzt. 1,5 Stunden nach der letzten Zugabe wurde das Ammoniak abgedampft und das Reaktionsgemisch mit 25 %-iger Salzsäure neutralisiert und über Nacht stehen gelassen. Danach wurde das Reaktionsgemisch dreimal in Wasser/Diäthyläther verteilt. Die wässrigen Phasen wurden mit Diäthyläther nachextrahiert. Die organischen Phasen wurden über Magnesiumsulfat getrocknet und am Rotationsverdampfer vom Lösungsmittel befreit. Die erhaltene, hellgelbe Flüssigkeit (3,8 g) wurde mit 200 ml Aceton und 0,1 ml konzentrierter Schwefelsäure versetzt. Das Gemisch wurde 10 Minuten zum Rückfluss erhitzt, dann mit Wasser versetzt und am Rotationsverdampfer vom Aceton befreit. Der Rückstand wurde dreimal in Methylenchlorid/Wasser verteilt. Die wässrigen Phasen wurden mit Methylenchlorid nachextrahiert. Die organischen Phasen wurden über Magnesiumsulfat getrocknet und eingedampft. Chromatographische Trennung der zurückbleibenden, gelben Flüssigkeit (3,5 g) an Kieselgel mit Essigester/Petroläther (Vol. 7 : 93) ergab 3,0 g (83,5 %) 4-(trans-1-Pentenyl) cyclohexanon als hellgelbe Flüssigkeit.

h) 1,63 g 4-(trans-1-Pentenyl) cyclohexanon wurden in 8 ml Diäthyläther und 14 ml Aethanol gelöst. Anschliessend wurde die Lösung tropfenweise mit ca. 70 ml Ammoniak versetzt und portionenweise solange mit Lithiumdraht versetzt, bis die Farbe des Reaktionsgemisches 1,5 Stunden lang konstant blieb (ca. 1,3 g Lithium). Danach wurde das Ammoniak abgedampft und das Gemisch mit Ammoniumchlorid und Salzsäure sauer gestellt und 3 Tage stehengelassen. Dann wurde das Reaktionsgemisch in Diäthyläther/Wasser verteilt und die wässrigen Phasen mit Diäthyläther nachextrahiert. Die organischen Phasen wurden über Magnesiumsulfat getrocknet, filtriert und am Rotationsverdampfer vom Lösungsmittel befreit. Niederdruckchromatographie des zurückbleibenden, gelben Oeles an Kieselgel mit Essigester/Petroläther (Vol. 10 : 90) ergab 1,47 g (89,1 %) trans-4-(trans-1-Pentenyl)-cyclohexanol als leicht gelbes, viskoses Oel.

Beispiel 6

a) 7 g Methoxymethyl-triphenylphosphoniumchlorid wurden in 35 ml t-Butylmethyläther aufgeschlämmt und bei — 20 °C mit 2,43 g Kalium-t-butylat versetzt. Das Gemisch wurde noch 1 Stunde bei

Raumtemperatur gerührt, dann bei —20 °C tropfenweise mit einer Lösung von 2 g 4-(trans-1-Pentenyl)-cyclohexanon in 18 ml Tetrahydrofuran versetzt und nochmals 1 Stunde bei Raumtemperatur gerührt. Anschliessend wurde das Reaktionsgemisch mit Wasser versetzt und dreimal mit je 50 ml Diäthyläther extrahiert. Die Extrakte wurden mit Wasser gewaschen und die Waschwasser mit Diäthyläther nachextrahiert. Die organischen Phasen wurden über Magnesiumsulfat getrocknet und vom Lösungsmittel befreit. Der Rückstand wurde in Essigester gelöst und die Lösung mit Petroläther verdünnt, durch Filtration vom ausgefallenen Triphenylphosphinoxid befreit und erneut eingeengt. Dieser Vorgang zur Abtrennung von Triphenylphosphinoxid wurde noch zweimal wiederholt und das erhaltene Rohprodukt von 1-(Methoxymethylen)-4-(trans-1-pentenyl) cyclohexan ohne zusätzliche Reinigung weiterverwendet.

b) 2,75 g 1-(Methoxymethylen)-4-(trans-1-pentenyl) cyclohexan (Rohprodukt aus Absatz a) wurden mit 100 ml Tetrahydrofuran/2N Salzsäure (Vol. 4 : 1) 30 Minuten zum Rückfluss erhitzt und dann über Nacht bei Raumtemperatur gerührt. Anschliessend wurde das Gemisch mit 100 ml Wasser versetzt und dreimal mit je 100 ml Diäthyläther extrahiert. Die Extrakte wurden mit verdünnter Natriumhydrogencarbonat-Lösung und Wasser gewaschen und die wässrigen Phasen mit Diäthyläther nachextrahiert. Die organischen Phasen wurden über Magnesiumsulfat getrocknet und eingedampft. Niederdruckchromatographie der zurückbleibenden gelblichen Flüssigkeit (2,5 g) an Kieselgel mit Petroläther und Essigester/Petroläther (Vol. 3 : 97) ergab 1,65 g (76 %) 4-(trans-1-Pentenyl) cyclohexancarboxaldehyd.

c) Eine Lösung von 1,6 g 4-(trans-1-Pentenyl) cyclohexancarboxyldehyd in 120 ml Aceton wurde auf —10 °C gekühlt, tropfenweise mit 8N Chromsäure (ca. 10 ml) versetzt, bis die Farbe des Reaktionsgemisches braunorange blieb, und noch 1 Stunde gerührt. Ueberschüssige Chromsäure wurde durch Zugabe von Isopropanol reduziert. Anschliessend wurde die grüne Lösung dreimal in Wasser/Methylenchlorid verteilt. Die organischen Extrakte wurden zweimal mit Wasser gewaschen und die Waschwasser mit Methylenchlorid nachextrahiert. Die organischen Phasen wurden über Magnesiumsulfat getrocknet und eingedampft. Der hellbraune kristalline Rückstand (2,01 g) wurde in 20 ml Petroläther teilweise gelöst. Ungelöster Rückstand wurde abfiltriert und das Filtrat eingedampft. Umkristallisation des erhaltenen Rückstandes aus 60 ml Petroläther bei —78 °C ergab 866 mg (49,5 %) 4-(trans-1-Pentenyl) cyclohexancarbonsäure als weisse Kristalle.

d) Ein Gemisch von 1,29 g 4-(trans-1-Pentenyl) cyclohexancarbonsäure und 20 ml einer 11 %-igen (Gew./Vol.) Lösung von Kaliumhydroxid in Diäthylenglykol wurde unter Argonbegasung 20 Stunden am Rückfluss gekocht. Anschliessend wurde das Reaktionsgemisch mit 25 %-iger Salzsäure schwach sauer gestellt und dreimal in Wasser/Methylenchlorid verteilt. Die organischen Extrakte wurden zweimal mit Wasser gewaschen und die Waschwasser mit Methylenchlorid nachextrahiert. Die organischen Phasen wurden über Magnesiumsulfat getrocknet und am Rotationsverdampfer vom Lösungsmittel befreit. Umkristallisation des erhaltenen, dunkelbraunen, kristallisierenden Oeles (1,17 g) aus 50 ml Petroläther bei —78 °C ergab 0,44 g trans-4-(trans-1-Pentenyl) cyclohexancarbonsäure als hellbraune Kristalle. Die Mutterlauge enthaltend 0,695 g rohe cis/trans-4-(trans-1-Pentenyl) cyclohexancarbonsäure wurde nicht aufgearbeitet.

## Beispiel 7

Zu einer Suspension von 5,3 g Lithiumaluminiumhydrid in 200 ml trockenem Tetrahydrofuran wurde unter Rühren in Inertgasatmosphäre innert 1 Stunde bei 5 °C eine Lösung von 16,1 g 2-(trans-1-Pentenyl) malonsäurediäthylester (Tetrahedron Lett. 1979, 861) in 75 ml Tetrahydrofuran zugetropft. Das Gemisch wurde noch 3,5 Stunden bei Raumtemperatur gerührt und dann nacheinander tropfenweise mit 15 ml Aceton und 20 ml gesättigter Natriumhydrogencarbonat-Lösung versetzt. Das Reaktionsgemisch wurde filtriert, das Filtrat eingeengt und der Rückstand (8,2 g) im Kugelrohr bei 150 °C/ca. 1 Torr destilliert. Hierbei wurden 6,5 g 2-(trans-1-Pentenyl)-1,3-propandiol als farbloses Oel erhalten.

## Beispiel 8

Eine Lösung von 1,692 g Diisopropylamin in 138 ml absolutem Tetrahydrofuran wurde unter Rühren und Stickstoffbegasung bei —73 °C bis —70 °C innert 3 Minuten tropfenweise mit 10,2 ml einer 1,6 M Lösung von Butyllithium in Hexan versetzt. Das Gemisch wurde 15 Minuten bei dieser Temperatur gerührt und dann bei —50 °C innert 3 Minuten tropfenweise mit einer Lösung von 4,40 g 4-[2r-(trans-4-Pentyl-cis-1-cyanocyclohexyl) äthyl]-1-cyclohexancarbonitril (cis/trans-Gemisch) in 12,5 ml absolutem Tetrahydrofuran versetzt (Nachspülen mit 7 ml absolutem Tetrahydrofuran). Die gelbe Lösung wurde 15 Minuten bei —50 °C gerührt, innert 7 Minuten tropfenweise mit einer Lösung von 1,889 g 4-Brom-1-buten in 12,5 ml absolutem Tetrahydrofuran versetzt und noch 1,5 Stunden bei —50 °C und dann 1,5 Stunden bei Raumtemperatur gerührt. Das Reaktionsgemisch wurde auf 280 ml gesättigte Ammoniumchlorid-Lösung gegossen und mit Diäthyläther extrahiert. Die organischen Phasen wurden mit Wasser gewaschen, über Natriumsulfat getrocknet und am Vakuum vom Lösungsmittel befreit. Der gelbe, feste Rückstand (5,41 g) wurde an Kieselgel in Toluol chromatographiert. Toluol und Toluol/1 % Aceton eluierten 3,16 g farbloses, kristallines 1'-(3-Butenyl)-4α-pentyl-1,4'α-äthylendi-1α-cyclohexancarbonitril. Umkristallisation aus Diäthyläther/Hexan ergab reines Produkt mit Smp. 110,2 °C.

Das als Ausgangsmaterial verwendete 4-[2r-(trans-4-Pentyl-cis-1-cyanocyclohexyl) äthyl]-1-cyclohexancarbonitril wurde wie folgt hergestellt:

a) Eine Lösung von 38,7 g 4-Cyanocyclohexyl-acetaldehyd in 400 ml Methanol wurde bei 0 °C unter Rühren und Stickstoffbegasung innert 20 Minuten portionenweise mit 9,67 g festem Natriumborhydrid versetzt. Nach Abklingen der exothermen Reaktion wurde das gelbliche Reaktionsgemisch 30 Minuten bei 0 °C gerührt, auf 1 l Eiswasser gegossen und mit Methylenchlorid extrahiert. Die organischen Phasen wurden mit Wasser gewaschen und über Natriumsulfat getrocknet. Nach Entfernen des Lösungsmittels im Vakuum wurden 38,4 g 2-(4-Cyanocyclohexyl) äthanol erhalten.

b) Eine Lösung von 38,4 g 2-(4-Cyanocyclohexyl) äthanol und 69,1 g Triphenylphosphin in 850 ml absolutem Methylenchlorid wurde unter Rühren und Stickstoffbegasung bei —10 °C innert 1 Stunde portionenweise mit 87,3 g festem Tetrabromethan versetzt. Die gelbliche, klare Lösung wurde noch 30 Minuten bei —10 °C und 30 Minuten bei 15 °C gerührt und im Vakuum vom Lösungsmittel befreit. Das zurückbleibende bräunliche Oel wurde in Methylenchlorid gelöst und durch eine Säule mit Kieselgel in Methylenchlorid filtriert. Hierbei wurden 62,6 g rohes 4-(2-Bromäthyl) cyclohexancarbonitril (cis/trans-Gemisch) erhalten. Destillation ergab eine farblose Flussigkeit mit Sdp. (0,6 mbar) 111-112 °C.

c) Eine Lösung von 6,176 g Diisopropylamin in 500 ml absolutem Tetrahydrofuran wurde unter Rühren und Stickstoffbegasung bei —72 °C bis —70 °C innert 4 Minuten tropfenweise mit 37 ml einer 1,7 M Lösung von Butyllithium in Hexan versetzt und noch 15 Minuten gerührt. Dann wurde das Gemisch bei —50 °C innert 4 Minuten tropfenweise mit einer Lösung von 9,147 g trans-4-Pentylcyclohexancarbonitril in 45 ml absolutem Tetrahydrofuran versetzt (Nachspülen mit 6 ml absolutem Tetrahydrofuran) und noch 15 Minuten gerührt. Danach wurde das Gemisch bei —50 °C innert 10 Minuten tropfenweise mit einer Lösung von 11,022 g 4-(2-Bromäthyl)-cyclohexancarbonitril in 45 ml absolutem Tetrahydrofuran versetzt. Die erhaltene gelbe Lösung wurde noch 1,5 Stunden bei —50 °C und 1,5 Stunden bei Raumtemperatur gerührt und dann auf 1 l gesättigte Ammoniumchlorid-Lösung gegossen und mit Diäthyläther extrahiert. Die organischen Phasen wurden mit Wasser gewaschen, über Natriumsulfat getrocknet und im Vakuum vom Lösungsmittel befreit. Der gelbe, halbfeste Rückstand (16,5 g) wurde in Hexan/Methylenchlorid (Vol. 9 : 1) gelöst und an Kieselgel mit Hexan/Methylenchlorid (mit steigendem Anteil Methylenchlorid) chromatographiert. Hierbei wurden 8,870 g 4-[2r-(trans-4-Pentyl-cis-1-cyanocyclohexyl) äthyl]-1-cyclohexancarbonitril als gelbliches Oel erhalten. Anschliessend wurden mit Methylenchlorid 3,415 g 1-[2-(trans-4-Cyanocyclohexyl) äthyl]-4′α-pentyl-1,1′-äthylendi-1α-cyclohexancarbonitril eluiert, welches nach Umkristallisation aus Diäthyläther/Hexan einen Smp. von 158,9-159,5 °C aufwies.

In analoger Weise wurde folgende Verbindung hergestellt:

1′-(4′-Pentenyl)-4α-pentyl-1,4′α-äthylendi-1α-cyclohexancarbonitril; Smp. 109,7 °C.

## Beispiel 9

Eine Lösung von 1,214 g Diisopropylamin in 99 ml absolutem Tetrahydrofuran wurde unter Rühren und Stickstoffbegasung bei —73 °C bis —70 °C innert 2 Minuten tropfenweise mit 7,33 ml einer 1,6 M Lösung von Butyllithium in Hexan versetzt. Das Gemisch wurde noch 15 Minuten bei dieser Temperatur gerührt und dann bei —50 °C innert 3 Minuten tropfenweise mit einer Lösung von 3,144 g 4-[2r-(trans-4-(4-Pentenyl)-cis-1-cyanocyclohexyl) äthyl]-1-cyclohexancarbonitril (cis/trans-Gemisch) in 9 ml absolutem Tetrahydrofuran versetzt (Nachspülen mit 5 ml absolutem Tetrahydrofuran). Die gelbliche Lösung wurde noch 15 Minuten bei —50 °C gerührt, innert 4 Minuten tropfenweise mit einer Lösung von 1,358 g 4-Brom-1-buten in 9 ml absolutem Tetrahydrofuran versetzt und die erhaltene rötliche Lösung noch 1,5 Stunden bei —50 °C und dann 1,5 Stunden bei Raumtemperatur gerührt. Das Reaktionsgemisch wurde auf 200 ml gesättigte Ammoniumchlorid-Lösung gegossen und mit Diäthyläther extrahiert. Die organischen Phasen wurden mit Wasser gewaschen, über Natriumsulfat getrocknet und im Vakuum vom Lösungsmittel befreit. Der gelbe, feste Rückstand (3,805 g) wurden an Kieselgel in Toluol chromatographiert. Toluol eluierte 2,36 g farbloses, kristallines 1′-(3-Butenyl)-4α-(4-pentenyl)-1,4′α-äthylendi-1α-cyclohexancarbonitril. Umkristallisation aus Diäthyläther/Hexan ergab reines Produkt mit Smp. 106,1 °C.

Das als Ausgangsmaterial verwendete 4-[2r-(trans-4-(4-Pentenyl)-cis-1-cyanocyclohexyl) äthyl]-1-cyclohexancarbonitril wurde wie folgt hergestellt:

a) Eine Suspension von 101,9 g Methoxymethyl-triphenylphosphoniumchlorid in 910 ml absolutem Diäthyläther wurde unter Rühren und Stickstoffbegasung bei Raumtemperatur mit 35,5 g festem Kalium-t-butylat versetzt und noch 1 Stunde gerührt. Dann wurde das Gemisch bei 2 °C innert 18 Minuten tropfenweise mit einer Lösung von 32,7 g 3-(4-Cyanocyclohexyl) propionaldehyd in 250 ml absolutem Diäthyläther versetzt, noch 1,5 Stunden bei Raumtemperatur gerührt und im Vakuum vom Lösungsmittel befreit. Der braune Rückstand wurde in Methylenchlorid aufgenommen und die Lösung mit Wasser gewaschen, über Natriumsulfat getrocknet und im Vakuum eingedampft. Der braune, feste Rückstand wurde durch Umkristallisation aus Toluol/Hexan teilweise von Triphenylphosphinoxid befreit und das Filtrat an Kieselgel in Hexan/Toluol chromatographiert. Hexan/Toluol, Toluol und Toluol/Aceton eluierten 33,80 g 4-(4-Methoxy-3-butenyl) cyclohexancarbonitril als gelbliche Flüssigkeit.

b) Ein Gemisch von 16,178 g 4-(4-Methoxy-3-butenyl) cyclohexancarbonitril, 125 ml Tetrahydrofuran und 31 ml 2N Salzsäure wurde unter Rühren und Stickstoffbegasung 30 Minuten zum Rückfluss erhitzt.

Nach dem Abkühlen wurde das Reaktionsgemisch auf 290 ml Wasser gegossen und mit methylenchlorid extrahiert. Die organischen Phasen wurden mit Wasser gewaschen, über Natriumsulfat getrocknet und im Vakuum vom Lösungsmittel befreit. Es resultierten 14,3 g 4-(4-Cyanocyclohexyl) butylraldehyd als gelbes Oel.

c) Eine Suspension von 42,8 g Methyl-triphenylphosphoniumbromid in 530 ml absolutem t-Butylmethyläther wurde unter Rühren und Stickstoffbegasung bei Raumtemperatur mit 13,5 g festem Kalium-t-butylat versetzt und noch 1 Stunde gerührt. Dann wurde das gelbe Reaktionsgemisch innert 35 Minuten tropfenweise mit einer Lösung von 14,3 g 4-(4-Cyanocyclohexyl) butyraldehyd in 133 ml absolutem t-Butylmethyläther versetzt und noch 2,5 Stunden bei Raumtemperatur gerührt. Anschliessend wurde das Reaktionsgemisch im Vakuum bei 50 °C vom Lösungsmittel befreit. Der Rückstand wurde in 300 ml Methanol/Wasser (Vol. 6 : 4) aufgenommen und einmal mit 310 ml und zweimal mit je 150 ml Hexan extrahiert. Die Hexan-Extrakte wurden einmal mit Methanol/Wasser (Vol. 6 : 4) und zweimal mit Wasser gewaschen und über Natriumsulfat getrocknet. Nach Entfernen des Lösungsmittels im Vakuum wurde 4-(4-Pentenyl) cyclohexancarbonitril erhalten.

d) Eine Lösung von 4,712 g Diisopropylamin in 382 ml absolutem Tetrahydrofuran wurde unter Rühren und Stickstoffbegasung bei —73 °C bis —70 °C innert 3 Minuten tropfenweise mit 28,2 ml einer 1,6 M Lösung von Butyllithium in Hexan versetzt und das Gemisch noch 15 Minuten bei dieser Temperatur gerührt. Dann wurde das Gemisch bei —50 °C innert 5 Minuten tropfenweise mit einer Lösung von 6,9 g 4-(4-Pentenylcyclo) hexancarbonitril in 34 ml absolutem Tetrahydrofuran versetzt (Nachspülen mit 5 ml absolutem Tetrahydrofuran und noch 15 Minuten gerührt. Anschliessend wurde das Gemisch bei —50 °C innert 9 Minuten tropfenweise mit einer Lösung von 8,411 g 4-(2-Bromäthyl) cyclohexancarbonitril in 34 ml absolutem Tetrahydrofuran versetzt. Das Reaktionsgemisch wurde noch 1,5 Stunden bei —50 °C und 1,5 Stunden bei Raumtemperatur gerührt und dann auf 750 ml gesättigte Ammoniumchlorid-Lösung gegossen und mit Diäthyläther extrahiert. Die organischen Phasen wurden mit Wasser gewaschen, über Natriumsulfat getrocknet und im Vakuum vom Lösungsmittel befreit. Der gelbe ölige Rückstand (13,2 g) wurde in Hexan/Methylenchlorid (Vol. 9 : 1) gelöst und an Kieselgel in Hexan/Methylenchlorid chromatographiert. Hexan/Methylenchlorid eluierte 6,360 g 4-[2r-(trans-4-(4-Pentenyl)-cis-1-cyanocyclohexyl) äthyl]-1-cyclohexancarbonitril als gelbes Oel. Anschliessend eluierte Methylenchlorid 2,925 g 1-[2-(trans-4-Cyanocyclohexyl) äthyl]-4'α-(4-pentenyl)-1,1'-äthylendi-1α-cyclohexancarbonitril ; Smp. 148,6-149,2 °C nach Umkristallisation aus Aceton/Hexan.

In analoger Weise wurde folgende Verbindung hergestellt :

1'-Butyl-4α-(4-pentenyl)1,4'α-äthylendi-1α-cyclohexancarbonitril ; Smp. 112,9 °C.

## Beispiel 10

Eine Lösung von 1,939 g 4-Butylphenol und 0,258 g 4-Dimethylamino) pyridin in 87 ml absolutem Tetrahydrofuran wurde unter Rühren mit einem Magnetrührer mit einer Lösung von 2,675 g 4-cis-Cyano-4-(3-butenyl) cyclohexancarbonsäure (enthaltend 11 % 4-trans-Cyano-Isomer) in 43 ml absolutem Methylenchlorid und anschliessend mit 3,197 g Dicyclohexylcarbodiimid versetzt. Das Reaktionsgemisch wurde 5 Stunden bei Raumtemperatur gerührt und dann genutscht. Der Niederschlag wurde mit Methylenchlorid nachgewaschen und das Filtrat im Vakuum eingeengt. Das zurückbleibende, gelbe Oel (5,488 g) wurde an Kieselgel in Hexan/Toluol (Vol. 1 : 1) chromatographiert. Toluol/Hexan (Vol. 1 : 1), Toluol und Toluol/Aceton (Vol. 99 : 1) eluierten 4-cis-Cyano-4-(3-butenyl) cyclohexancarbonsäure-4-butylphenylester als farblose Kristalle. Durch Umkristallisation aus Diäthyläther/Hexan wurde reines Produkt mit Smp. 59,1 °C erhalten.

Die als Ausgangsmaterial verwendete 4-cis-Cyano-4-(3-butenyl) cyclohexancarbonsäure wurde wie folgt erhalten :

Eine Lösung von 8,996 g Diisopropylamin in 360 ml absolutem Tetrahydrofuran wurde unter Rühren und Stickstoffbegasung bei —73 °C bis —65 °C innert 6 Minuten tropfenweise mit 54,5 ml einer 1,6 M Lösung von Butyllithium in Hexan versetzt und noch 15 Minuten bei —70 °C gerührt. Dann wurde das Gemisch bei —50 °C innert 7 Minuten tropfenweise mit einer Lösung von 5,7 g 4-Cyanocyclohexancarbonsäure in 40 ml absolutem Tetrahydrofuran versetzt (Nachspülen mit 10 ml absolutem Tetrahydrofuran). Die rötliche Suspension wurde noch 15 Minuten bei —50 °C gerührt und anschliessend innert 9 Minuten tropfenweise mit einer Lösung von 5,025 g 4-Brom-1-buten in 35 ml absolutem Tetrahydrofuran versetzt (Nachspülen mit 10 ml absolutem Tetrahydrofuran). Die rötliche, klare Lösung wurde noch 1,5 Stunden bei Raumtemperatur gerührt, dann auf 500 ml Wasser gegossen und mit Diäthyläther extrahiert. Die organischen Phasen wurden dreimal mit je 250 ml Wasser gewaschen und verworfen. Die wässrigen Phasen wurden vereinigt, mit 18 ml halbkonzentrierter Salzsäure kongosauer gestellt und mit Methylenchlorid extrahiert. Die Methylenchlorid-Phasen wurden mit Wasser gewaschen und über Natriumsulfat getrocknet. Nach Entfernen des Lösungsmittels im Vakuum bei 50 °C wurden 7,003 g 4-cis-Cyano-4-(3-butenyl) cyclohexancarbonsäure (enthaltend 11 % 4-trans-Cyano-Isomer) als gelbes, festes Produkt erhalten.

In analoger Weise wurden folgende Verbindungen hergestellt :

4-cis-Cyano-4-(3-butenyl) cyclohexancarbonsäure-4-äthoxyphenylester ; Smp. (C-N) 57,2 °C, Klp. (N-I) 68,1 °C,

4-cis-Cyano-4-butylcyclohexancarbonsäure-4-allyloxyphenylester ; Smp. (C-I) 77,2 °C, Klp. (N-I) 61,0 °C,

4-cis-Cyano-4-(3-butenyl) cyclohexancarbonsäure-4-trans-(4-butylphenyl) cyclohexylester ; Smp. (C-N) 67,4 °C, Klp. (N-I) 102,5 °C.

4-cis-Cyano-4-(3-butenyl) cyclohexancarbonsäure-4-transbutylcyclohexylester ; Smp. (C-I) 20,5 °C, Klp. (N-I) — 1,5 °C.

Beispiel 11

Eine Lösung von 2,277 g 4-cis-Cyano-4-(3-butenyl) cyclohexanol und 0,254 g 4-(Dimethylamino) pyridin in 50 ml absolutem Methylenchlorid wurde unter Rühren mit einem Magnetrührer mit einer Suspension von 2,110 g 4-Aethoxybenzoesäure in 100 ml absolutem Methylenchlorid und anschliessend mit 3,145 g Dicyclohexylcarbodiimid versetzt. Das Reaktionsgemisch wurde noch 2,5 Stunden bei Raumtemperatur gerührt und über Nacht stehengelassen. Der Niederschlag wurde abgenutscht und mit Methylenchlorid nachgewaschen. Das Filtrat wurde im Vakuum eingeengt. Das zurückbleibende, gelbe, halbfeste Produkt (6,8 g) wurde in Methylenchlorid gelöst und die Lösung mit 30 g Kieselgel versetzt und eingedampft. Der Rückstand wurde in Toluol/Hexan (Vol. 1 : 1) suspendiert und auf eine Säule mit Kieselgel in Toluol/Hexan (Vol. 1 : 1) gegeben. Toluol/Hexan, Toluol und Toluol/Aceton eluierten 4-Aethoxybenzoesäure-4-cis-cyano-4-(3-butenyl) cyclohexylester als farbloses, kristallisierendes Oel. Umkristallisation aus Diäthyläther/Hexan ergab reines Produkt mit Smp. 85,4 °C.

Das als Ausgangsmaterial verwendete 4-cis-Cyano-4-(3-butenyl) cyclohexanol wurde wie folgt hergestellt :

a) Ein Gemisch von 113 g 4-Acetoxycyclohexancarbonsäure und 250 ml Thionylchlorid wurde 1 Stunde bei Raumtemperatur stehengelassen und dann 2 Stunden unter Rückfluss gekocht. Ueberschüssiges Thionylchlorid wurde abdestilliert und der Rückstand im Vakuum vom restlichen Thionylchlorid befreit. Das erhaltene 4-Acetoxycyclohexancarbonsäurechlorid (139,1 g) wurde mit 500 ml absolutem Methylenchlorid versetzt und das Gemisch zu 2,5 l auf — 10 °C gekühltem, mit Ammoniakgas gesättigtem Methylenchlorid zugetropft. In die rötliche Suspension wurde unter Rühren noch 2 Stunden Ammoniakgas eingeleitet. Anschliessend wurde das Reaktionsgemisch im Vakuum bei 50 °C möglicht weitgehend vom Methylenchlorid befreit. Der rötliche Rückstand wurde mit 500 ml Wasser versetzt und mit Methylenchlorid extrahiert. Die organischen Extrakte wurden mit gesättigter Kochsalzlösung gewaschen, über Natriumsulfat getrocknet und im Vakuum eingedampft. Der Rückstand (109 g) wurde in 500 ml Diäthyläther suspendiert. Die Suspension wurde 30 Minuten bei Raumtemperatur gerührt und dann genutscht. Der Rückstand wurde mit Diäthyläther gewaschen und getrocknet, wobei 94 g rohes 4-Acetoxycyclohexancarbonsäureamid erhalten wurden.

b) Eine Suspension von 68,8 g rohem 4-Acetoxycyclohexancarbonsäureamid in 200 ml absolutem Pyridin wurde unter Rühren mit 78,7 g Benzolsulfonylchlorid versetzt und über Nacht stehengelassen. Danach wurde das Reaktionsgemisch auf 830 ml Eiswasser und 415 ml konzentrierte Salzsäure gegossen und mit Diäthyläther extrahiert. Der Aetherextrakt wurde mit Wasser gewaschen, über Natriumsulfat getrocknet und im Vakuum eingedampft, wobei 59,5 g rohes 4-Acetoxycyclohexancarbonitril als bräunliche Flüssigkeit erhalten wurden.

c) Eine Lösung von 18,74 g rohem 4-Acetoxycyclohexancarbonitril in 52 ml Toluol wurde unter Rühren und Stickstoffbegasung mit einer Lösung von 6,65 g Natriumhydroxid in 45 ml Wasser versetzt und noch 5 Stunden gerührt. Anschliessend wurde das Reaktionsgemisch mit Methylenchlorid extrahiert und die organischen Phasen mit gesättigter Kochsalzlösung gewaschen, über Natriumsulfat getrocknet und eingedampft. Das zurückbleibende, bräunliche Oel (14,6 g) wurde in Toluol gelöst und an Kieselgel chromatographisch getrennt. Toluol/Aceton-Gemische eluierten neben 6,9 g apolaren Nebenprodukten 7,5 g 4-Hydroxycyclohexancarbonitril (cis/trans-Gemisch) als farbloses Oel.

d) Eine Lösung von 19,1 g 4-Hydroxycyclohexancarbonitril in 250 ml absolutem Diäthyläther wurde mit 21,2 g Dihydropyran und 1,5 g p-Toluolsulfonsäure versetzt und über Nacht stehengelassen. Anschliessend wurde das Reaktionsgemisch auf ein Gemisch von 100 ml 3N Natronlauge und 100 g Eis gegossen, die Aetherphase abgetrennt und die wässrige Phase mit Methylenchlorid extrahiert. Die vereinigten organischen Phasen wurden mit gesättigter Kochsalzlösung gewaschen, über Natriumsulfat getrocknet und im Vakuum vom Lösungsmittel befreit. Der erhaltene, rohe Tetrahydropyranyläther (39,5 g) wurde in Toluol gelöst und durch eine Säule mit Aluminiumoxid in Toluol filtriert (Nachspülen mit Toluol/Diäthyläther). Es resultierten 32,5 g Tetrahydropyranyläther des 4-Hydroxycyclohexancarbonitrils (cis/trans-Gemisch).

e) Eine Lösung von 7,111 g Diisopropylamin in 575 ml absolutem Tetrahydrofuran wurde unter Rühren und Stickstoffbegasung bei — 73 °C innert 5 Minuten tropfenweise mit 42,7 ml einer 1,6 M Lösung von Butyllithium in Hexan versetzt und das Gemisch noch 15 Minuten bei — 70 °C gerührt. Dann wurde das Gemisch bei — 50 °C innert 4 Minuten tropfenweise mit einer Lösung von 12,238 g des Tetrahydrohydropyranyläthers von 4-Hydroxycyclohexancarbonitril in 43 ml absolutem Tetrahydrofuran versetzt (Nachspülen mit 14 ml absolutem Tetrahydrofuran) und die gelbe Lösung noch 15 Minuten bei — 50 °C gerührt. Anschliessend wurde das Reaktionsgemisch bei — 50 °C innert 8 Minuten tropfenweise mit einer Lösung von 7,898 g 4-Brom-1-buten in 43 ml absolutem Tetrahydrofuran versetzt (Nachspülen

mit 14 ml absolutem Tetrahydrofuran). Die gelbe Reaktionslösung wurde noch 1,5 Stunden bei —50 °C und 1,5 Stunden bei Raumtemperatur gerührt, dann auf 640 ml gesättigte Ammoniumchlorid-Lösung gegossen und mit Diäthyläther extrahiert. Die organischen Phasen wurden mit Wasser gewaschen, über Natriumsulfat getrocknet und im Vakuum vom Lösungsmittel befreit. Das zurückbleibende gelbe Oel (15,2 g) wurde in 145 ml Tetrahydrofuran gelöst, mit 50 ml 1N Salzsäure versetzt und über Nacht stehengelassen. Anschliessend wurde das Gemisch mit 47,5 ml 1N Natronlauge versetzt und im Vakuum vom Tetrahydrofuran befreit. Die erhaltene Emulsion wurde mit Methylenchlorid extrahiert. Die organischen Phasen wurden mit gesättigter Kochsalzlösung gewaschen, über Natriumsulfat getrocknet und im Vakuum vom Lösungsmittel befreit. Das zurückbleibende gelbe Oel (13,9 g) wurde in Toluol gelöst und an Kieselgel in Toluol chromatographisch getrennt. Toluol/Aceton eluierten zuerst 2,78 g unpolare Verunreinigungen und dann 7,59 g 4-cis-Cyano-4-(3-butenyl) cyclohexanol als farblose Flüssigkeit mit Sdp. (0,3 mbar) 120 °C.

In analoger Weise wurden folgende Verbindungen hergestellt :

4″-Pentyl-p-terphenylcarbonsäure-4-cis-cyano-4-(3- butenyl) cyclohexylester ; Smp. (C-N) 154,4 °C, Klp. (N-I) 272 °C,

4-trans-(4′-Pentylbiphenylyl) cyclohexancarbonsäure-4-cis-cyano-4-(3-butenyl) cyclohexylester ; Smp. (C-N) 127, 8 °C, Klp. (N-I) 225 °C,

4-Pentylbenzosäure-4-cis-cyano-4-(3-butenyl) cyclohexylester ; Smp. (C-I) 71,2 °C.

4′-Pentyl-4-biphenylcarbonsaüre-4-cis-cyano-4-(3-butenyl) cyclohexylester ; Smp. (C-N) 89,8 °C, Klp. (N-I) 129,7 °C,

4-trans-Pentylcyclohexancarbonsäure-4-cis-cyano-4-(3-butenyl) cyclohexylester ; Smp. (C-I) 74,9 °C,

4-trans-(4-Pentylphenyl) cyclohexancarbonsäure-4-cis-cyano-4-(3-butenyl) cyclohexylester ; Smp. (C-I) 87,2 °C ; Klp. (N-I) 66,5 °C.

**Patentansprüche** (für die Vertragsstaaten : BE, CH, DE, FR, GB, IT, LI, NL, SE)

1. Verbindungen der allgemeinen Formel

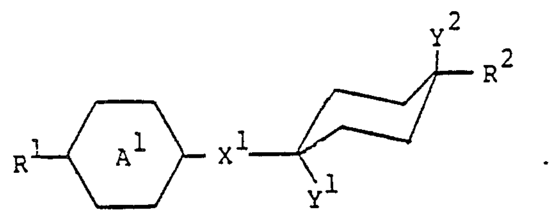

(I)

worin $X^1$ eine einfache Kovalenzbindung, $—CH_2CH_2—$, —COO—, —OOC— oder 1,4-Phenylen darstellt ; eines der Symbole $Y^1$ und $Y^2$ Wasserstoff unddas andere Cyano bezeichnet ; $R^2$ trans-1-Alkenyl, cis-2-Alkenyl, trans-3-Alkenyl, 4-Alkenyl oder Alkyl bedeutet ; $R^1$ für die Gruppe $R^{11}—A^4—A^5—$ steht und $A^4$ und $A^5$ unabhängig voneinander eine einfache Kovalenzbindung, 1,4-Phenylen oder trans-1,4-Cyclohexylen darstellen ; $R^{11}$ trans-1-Alkenyl, cis-2-Alkenyl, trans-3-Alkenyl, 4-Alkenyl, trans-2-Alkenyloxy, 3-Alkenyloxy oder, wenn $R^2$ für eine Alkenylgruppe steht, auch Alkyl oder Alkoxy bedeutet ; Ring $A^1$ 1,4-Phenylen oder eine Gruppe der allgemeinen Formel

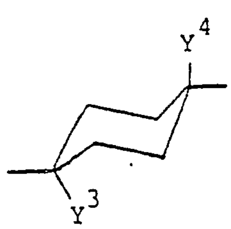

(II)

darstellt ; und $Y^3$ und $Y^4$ Wasserstoff bezeichnen oder, sofern $X^1$ $—CH_2CH_2—$, —COO— oder —OOC— darstellt, eines der Symbole $Y^3$ und $Y^4$ auch Cyano bezeichnet.

2. Verbindungen nach Anspruch 1, dadurch gekennzeichnet, dass $R^1$ und $R^2$ unabhängig voneinander trans-1-Alkenyl, cis-2-Alkenyl, trans-3-Alkenyl oder 4-Alkenyl bedeuten oder einer der Reste $R^1$ und $R^2$ auch Alkyl bedeutet, und $Y^3$ und $Y^4$ in Formel II Wasserstoff bezeichnen oder, sofern $X^1$ —COO— oder —OOC— darstellt, eines der Symbole $Y^3$ und $Y^4$ auch Cyano bezeichnet.

3. Verbindungen nach Anspruch 1, dadurch gekennzeichnet, dass $X^1$ $—CH_2CH_2—$ darstellt, $R^1$ und $R^2$ unabhängig voneinander trans-1-Alkenyl, cis-2-Alkenyl, trans-3-Alkenyl oder 4-Alkenyl bedeuten oder einer der Reste $R^1$ und $R^2$ auch Alkyl bedeutet, Ring $A^1$ eine Gruppe der Formel II darstellt, und eines der Symbole $Y^3$ und $Y^4$ Wasserstoff und das andere Cyano bezeichnet.

4. Verbindungen nach Anspruch 2 oder 3, dadurch gekennzeichnet, dass $R^1$ und $R^2$ Reste mit höchstens 12 Kohlenstoffatomen, vorzugsweise höchstens 7 Kohlenstoffatomen bedeuten.

5. Verbindungen nach einem der Ansprüche 2 bis 4, dadurch gekennzeichnet, dass $R^1$ und $R^2$ unabhängig voneinander trans-1-Alkenyl, trans-3-Alkenyl oder 4-Alkenyl bedeuten oder einer der Reste $R^1$ und $R^2$, vorzugsweise $R^1$, auch Alkyl bedeutet.

6. Verbindungen nach Anspruch 5, dadurch gekennzeichnet, dass einer der Reste $R^1$ und $R^2$ trans-3-Alkenyl und der andere trans-1-Alkenyl, trans-3-Alkenyl, 4-Alkenyl oder Alkyl bedeutet.

7. Verbindungen nach einem der Ansprüche 1 bis 6, dadurch gekennzeichnet, dass $Y^1$ und ein gegebenenfalls vorhandener Substituent $Y^3$ Wasserstoff bedeuten und $Y^2$ Cyano bezeichnet.

8. Flüssigkristallines Gemisch mit mindestens 2 Komponenten, dadurch gekennzeichnet, dass mindestens eine Komponente eine Verbindung der in Anspruch 1 definierten Formel I ist.

9. Verfahren zur Herstellung der Verbindungen der in Anspruch 1 definierten Formel I, dadurch gekennzeichnet, dass man

a) zur Herstellung der Verbindungen der Formel I, worin $X^1$ —COO— oder —OOC— darstellt, eine Verbindung der allgemeinen Formel

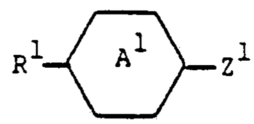

(III)

mit einer Verbindung der allgemeinen Formel

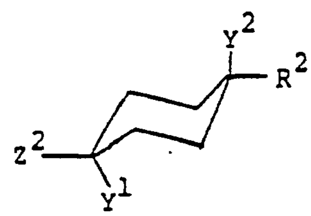

(IV)

worin eine der Gruppen $Z^1$ und $Z^2$ —COOH oder —COCl und die andere —OH bedeutet : Ring $A^1$ 1,4-Phenylen oder eine Gruppe der in Anspruch 1 definierten Formel II darstellt : $Y^3$ und $Y^4$ in Formel II Wasserstoff bezeichnen oder eines dieser Symbole auch Cyano bezeichnet ; und $R^1$, $R^2$, $Y^1$ und $Y^2$ die in Anspruch 1 gegebenen Bedeutungen haben, verestert, oder

b) zur Herstellung der Verbindungen der Formel I, worin $X^1$ eine einfache Kovalenzbindung, —$CH_2CH_2$ oder 1,4-Phenylen darstellt, $Y^2$ Cyano bezeichnet und $R^2$ cis-2-Alkenyl, trans-3-Alkenyl, 4-Alkenyl oder Alkyl bedeutet, eine Verbindung der allgemeinen Formel

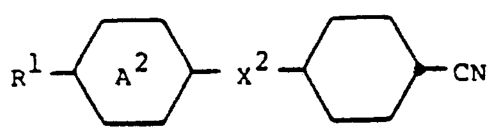

(V)

zuerst mit Base und dann mit einer Verbindung der allgemeinen Formel

$$Z^3\text{—}R^5 \qquad \text{(VI)}$$

worin $X^2$ eine einfache Kovalenzbindung, —$CH_2CH_2$— oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ —$CH_2CH_2$— darstellt, eines der Symbole $Y^3$ und $Y^4$ auch Cyano bezeichnet ; $Z^3$ Halogen bezeichnet ; $R^5$ cis-2-Alkenyl, trans-3-Alkenyl, 4-Alkenyl oder Alkyl bedeutet ; und $R^1$ die obige Bedeutung hat, umsetzt, oder

c) zur Herstellung der Verbindungen der Formel I, worin $X^1$ eine einfache Kovalenzbindung oder —$CH_2CH_2$— darstellt, Ring $A^1$ trans-1,4-Cyclohexylen bedeutet und $Y^1$ Cyano bezeichnet, eine Verbindung der allgemeinen Formel

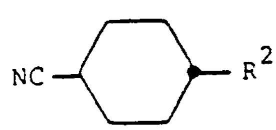

(VII)

zuerst mit Base und dann mit einer Verbindung der allgemeinen Formel

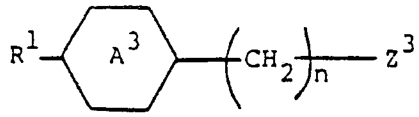

(VIII)

worin n für die Zahl 0 und Ring $A^3$ für cis-1,4-cyclohexylen steht oder n für die Zahl 2 und Ring $A^3$ für trans-1,4-Cyclohexylen steht ; $Z^3$ Halogen bezeichnet ; und $R^1$ und $R^2$ die in Anspruch 1 gegebenen Bedeutungen haben, umsetzt, oder

d) zur Herstellung der Verbindungen der Formel I, worin $X^1$ eine einfache Kovalenzbindung, $-CH_2CH_2-$ oder 1,4-Phenylen darstellt und $Y^1$ Cyano bezeichnet, eine Verbindung der allgemeinen Formel

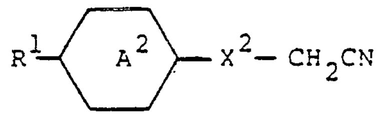

(IX)

zuerst mit Base und dann mit einer Verbindung der allgemeinen Formel

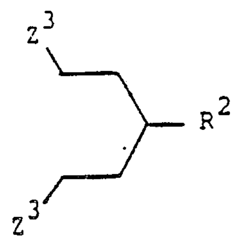

(X)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; $Z^3$ Halogen bezeichnet ; und $R^1$ und $R^2$ die in Anspruch 1 gegebenen Bedeutungen haben umsetzt, oder

e) zur Herstellung der Verbindungen der Formel I, worin $X^1$ eine einfache Kovalenzbindung, $-CH_2CH_2-$ oder 1,4-Phenylen darstellt ; $Y^2$ Cyano bezeichnet und $R^2$ trans-1-Alkenyl bedeutet, eine Verbindung der allgemeinen Formel

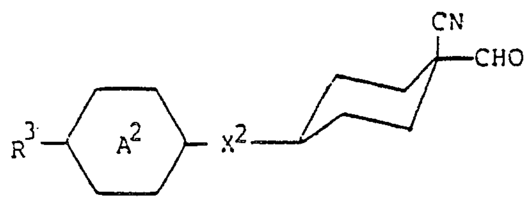

(XI)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; und $R^3$ die für $R^1$ in Formel I gegebenen Bedeutungen hat, in Gegenwart einer Base mit Alkyl-triarylphosphoniumhalogenid umsetzt, oder

f) zur Herstellung der Verbindungen der Formel I, worin $X^1$ eine einfache Kovalenzbindung, $-CH_2CH_2-$ oder 1,4-Phenylen darstellt, an eine Verbindung der allgemeinen Formel

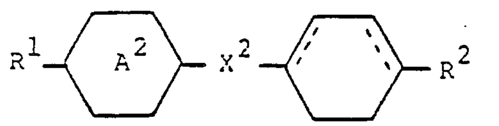

(XII)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; eine der unterbrochenen Linien eine zusätzliche Bindung bezeichnet ; und $R^1$ und $R^2$ die in Anspruch 1 gegebenen Bedeutungen haben, HCN anlagert, oder

g) zur Herstellung der Verbindungen der Formel I, worin $X^1$ eine einfache Kovalenzbindung, $-CH_2CH_2-$ oder 1,4-Phenylen darstellt, und $R^1$ trans-2-Alkenyloxy, 3-Alkenyloxy oder Alkoxy bedeutet, eine Verbindung der allgemeinen Formel

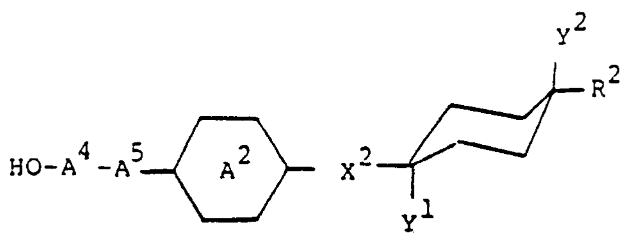

(LXXXI)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; und $A^4$, $A^5$, $R^2$, $Y^1$ und $Y^2$ die in Formel I gegebenen Bedeutungen haben, mit trans-2-Alkenylhalogenid, 3-Alkenylhalogenid oder Alkylhalogenid veräthert.

10. Verwendung der Verbindungen der in Anspruch 1 definierten Formel I für elektro-optische Zwecke.

**Patentansprüche** (für den Vertragsstaat AT)

1. Flüssigkristallines Gemisch mit mindestens 2 Komponenten, dadurch gekennzeichnet, dass mindestens eine Komponente eine Verbindung der allgemeinen Formel

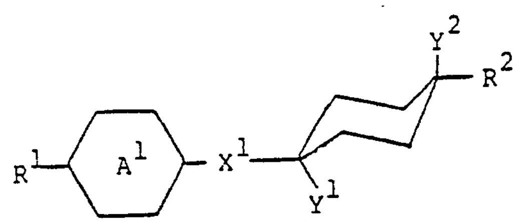

(I)

worin $X^1$ eine einfache Kovalenzbindung, $-CH_2CH_2-$, $-COO-$, $-OOC-$ oder 1,4-Phenylen darstellt ; eines der Symbole $Y^1$ und $Y^2$ Wasserstoff und das andere Cyano bezeichnet ; $R^2$ trans-1-Alkenyl, cis-2-Alkenyl, trans-3-Alkenyl, 4-Alkenyl oder Alkyl bedeutet ; $R^1$ für die Gruppe $R^{11}-A^4-A^5-$ steht und $A^4$ und $A^5$ unabhängig voneinander eine einfache Kovalenzbindung, 1,4-Phenylen oder trans-1,4-Cyclohexylen darstellen ; $R^{11}$ trans-1-Alkenyl, cis-2-Alkenyl, trans-3-Alkenyl, 4-Alkenyl, trans-2-Alkenyloxy, 3-Alkenyloxy oder, wenn $R^2$ für eine Alkenylgruppe steht, auch Alkyl oder Alkoxy bedeutet : Ring $A^1$ 1,4-Phenylen oder eine Gruppe der allgemeinen Formel

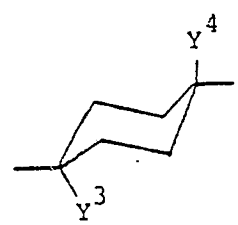

(II)

darstellt ; und $Y^3$ und $Y^4$ Wasserstoff bezeichnen oder, sofern $X^1$ $-CH_2CH_2-$, $-COO-$ oder $-OOC-$ darstellt, eines der Symbole $Y^3$ und $Y^4$ auch Cyano bezeichnet, ist.

2. Flüssigkristallines Gemisch nach Anspruch 1, dadurch gekennzeichnet, dass $R^1$ und $R^2$ unabhängig voneinander trans-1-Alkenyl, cis-2-Alkenyl, trans-3-Alkenyl oder 4-Alkenyl bedeuten oder einer der Reste $R^1$ und $R^2$ auch Alkyl bedeutet, und $Y^3$ und $Y^4$ in Formel II Wasserstoff bezeichnen oder, sofern $X^1$ $-COO-$ oder $-OOC-$ darstellt, eines der Symbole $Y^3$ und $Y^4$ auch Cyano bezeichnet.

3. Flüssigkristallines Gemisch nach Anspruch 1, dadurch gekennzeichnet, dass $X^1$ $-CH_2CH_2-$ darstellt, $R^1$ und $R^2$ unabhängig voneinander trans-1-Alkenyl, cis-2-Alkenyl, trans-3-Alkenyl oder 4-Alkenyl bedeuten oder einer der Reste $R^1$ und $R^2$ auch Alkyl bedeutet, Ring $A^1$ eine Gruppe der Formel II darstellt, und eines der Symbole $Y^3$ und $Y^4$ Wasserstoff und das andere Cyano bezeichnet.

4. Flüssigkristallines Gemisch nach Anspruch 2 oder 3, dadurch gekennzeichnet, dass $R^1$ und $R^2$ Reste mit höchstens 12 Kohlenstoffatomen, vorzugsweise höchstens 7 Kohlenstoffatomen bedeuten.

5. Flüssigkristallines Gemisch nach einem der Ansprüche 2 bis 4, dadurch gekennzeichnet, dass $R^1$ und $R^2$ unabhängig voneinander trans-1-Alkenyl, trans-3-Alkenyl oder 4-Alkenyl bedeuten oder einer der Reste $R^1$ und $R^2$, vorzugsweise $R^1$, auch Alkyl bedeutet.

6. Flüssigkristallines Gemisch nach Anspruch 5, dadurch gekennzeichnet, dass einer der Reste $R^1$

und $R^2$ trans-3-Alkenyl und der andere trans-1-Alkenyl, trans-3-Alkenyl, 4-Alkenyl oder Alkyl bedeutet.

7. Flüssigkristallines Gemisch nach einem der Ansprüche 1 bis 6, dadurch gekennzeichnet, dass $Y^1$ und ein gegebenenfalls vorhandener Substituent $Y^3$ Wasserstoff bedeuten und $Y^2$ Cyano bezeichnet.

8. Verfahren zur Herstellung der Verbindungen der in Anspruch 1 definierten Formel I, dadurch gekennzeichnet, dass man

a) zur Herstellung der Verbindungen der Formel I, worin $X^1$ —COO— oder —OOC— darstellt, eine Verbindung der allgemeinen Formel

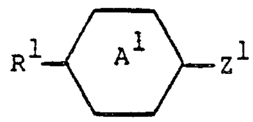

(III)

mit einer Verbindung der allgemeinen Formel

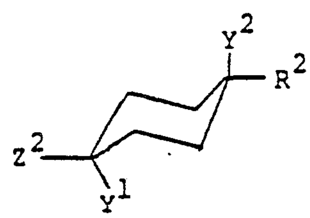

(IV)

worin eine der Gruppen $Z^1$ und $Z^2$ —COOH oder —COCl und die andere —OH bedeutet ; Ring $A^1$ 1,4-Phenylen oder eine Gruppe der in Anspruch 1 definierten Formel II darstellt ; $Y^3$ und $Y^4$ in Formel II Wasserstoff bezeichnen oder eines dieser Symbole auch Cyano bezeichnet ; und $R^1$, $R^2$, $Y^1$ und $Y^2$ die in Anspruch 1 gegebenen Bedeutungen haben, verestert, oder

b) zur Herstellung der Verbindungen der Formel I, worin $X^1$ eine einfache Kovalenzbindung, $—CH_2CH_2—$ oder 1,4-Phenylen darstellt, $Y^2$ Cyano bezeichnet und $R^2$ cis-2-Alkenyl, trans-3-Alkenyl, 4-Alkenyl oder Alkyl bedeutet, eine Verbindung der allgemeinen Formel

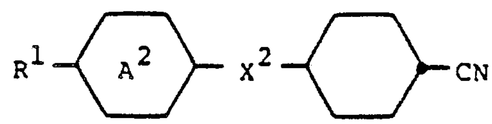

(V)

zuerst mit Base und dann mit einer Verbindung der allgemeinen Formel

$$Z^3—R^5 \qquad (VI)$$

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; $Z^3$ Halogen bezeichnet ; $R^5$ cis-2-Alkenyl, trans-3-Alkenyl, 4-Alkenyl oder Alkyl bedeutet ; und $R^1$ die obige Bedeutung hat, umsetzt, oder

c) zur Herstellung der Verbindungen der Formel I, worin $X^1$ eine einfache Kovalenzbindung oder $—CH_2CH_2—$ darstellt, Ring $A^1$ trans-1,4-Cyclohexylen bedeutet und $Y^1$ Cyano bezeichnet, eine Verbindung der allgemeinen Formel

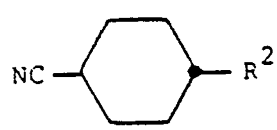

(VII)

zuerst mit Base und dann mit einer Verbindung der allgemeinen Formel

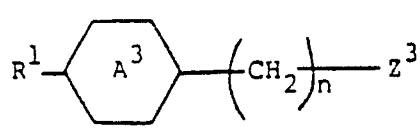

(VIII)

worin n für die Zahl 0 und Ring $A^3$ für cis-1,4-Cyclohexylen steht oder n für die Zahl 2 und Ring $A^3$ für trans-1,4-Cyclohexylen steht ; $Z^3$ Halogen bezeichnet ; und $R^1$ und $R^2$ die in Anspruch 1 gegebenen Bedeutungen haben, umsetzt, oder

d) zur Herstellung der Verbindungen der Formel I, worin $X^1$ eine einfache Kovalenzbindung, $-CH_2CH_2-$ oder 1,4-Phenylen darstellt und $Y^1$ Cyano bezeichnet, eine Verbindung der allgemeinen Formel

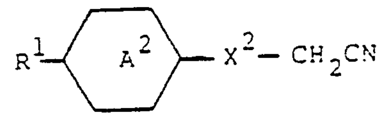

(IX)

zuerst mit Base und dann mit einer Verbindung der allgemeinen Formel

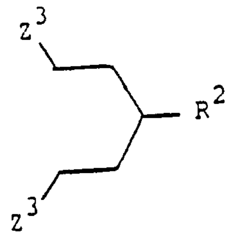

(X)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; $Z^3$ Halogen bezeichnet ; und $R^1$ und $R^2$ die in Anspruch 1 gegebenen Bedeutungen haben, umsetzt, oder

e) zur Herstellung der Verbindungen der Formel I, worin $X^1$ eine einfache Kovalenzbindung, $-CH_2CH_2-$ oder 1,4-Phenylen darstellt ; $Y^2$ Cyano bezeichnet und $R^2$ trans-1-Alkenyl bedeutet, eine Verbindung der allgemeinen Formel

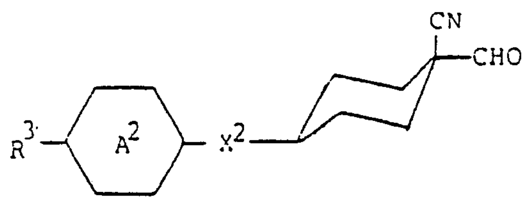

(XI)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; und $R^3$ die für $R^1$ in Formel I gegebenenen Bedeutungen hat, in Gegenwart einer Base mit Alkyl-triarylphosphoniumhalogenid umsetzt, oder

f) zur Herstellung der Verbindungen der Formel I, worin $X^1$ eine einfache Kovalenzbindung, $-CH_2CH_2-$ oder 1,4-Phenylen darstellt, an eine Verbindung der allgemeinen Formel

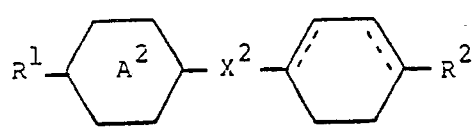

(XII)

worin $X^2$ eine einfache Kovalenzbindung, $-CH_2CH_2-$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $-CH_2CH_2-$ darstellt, eines der Symbole $Y^3$ und $Y^4$ auch Cyano bezeichnet ; eine der unterbrochenen Linien eine zusätzliche Bindung bezeichnet ; und $R^1$ und $R^2$ die in Anspruch 1 gegebenen Bedeutungen haben, HCN anlagert, oder

g) zur Herstellung der Verbindungen der Formel I, worin $X^1$ eine einfache Kovalenzbindung, $-CH_2CH_2-$ oder 1,4-Phenylen darstellt, und $R^1$ trans-2-Alkenyloxy, 3-Alkenyloxy oder Alkoxy bedeutet, eine Verbindung der allgemeinen Formel

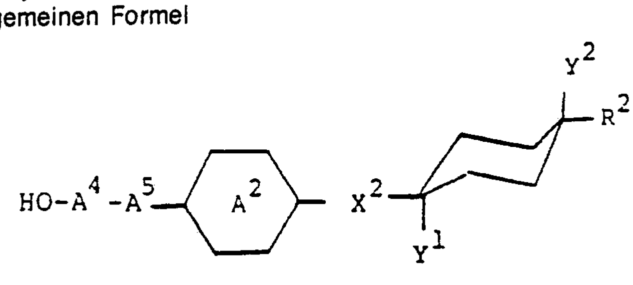

(LXXXI)

worin $X^2$ eine einfache Kovalenzbindung, $—CH_2CH_2—$ oder 1,4-Phenylen darstellt ; Ring $A^2$ 1,4-Phenylen oder eine Gruppe der Formel II bezeichnet ; $Y^3$ und $Y^4$ in Formel II Wasserstoff bedeuten oder, sofern $X^2$ $—CH_2CH_2—$ darstellt, eines der Symbole $Y^3$ und $Y^4$ auch Cyano bezeichnet ; und $A^4$, $A^5$, $R^2$, $Y^1$ und $Y^2$ die in Formel I gegebenen Bedeutungen haben, mit trans-2-Alkenylhalogenid, 3-Alkenylhalogenid oder Alkylhalogenid veräthert.

9. Verwendung der Verbindungen der in Anspruch 1 definierten Formel I für elektro-optische Zwecke.

**Claims** (for the Contracting States : BE, CH, DE, FR, GB, IT, LI, NL, SE)

1. Compounds of the general formula

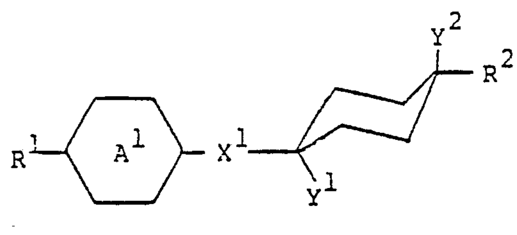

(I)

wherein $X^1$ represents a single covalent bond, $—CH_2CH_2—$, —COO—, —OOC— or 1,4-phenylene ; one of the symbols $Y^1$ and $Y^2$ denotes hydrogen and the other denotes cyano ; $R^2$ signifies trans-1-alkenyl, cis-2-alkenyl, trans-3-alkenyl, 4-alkenyl or alkyl ; $R^1$ stands for the group $R^{11}—A^4—A^5—$ and $A^4$ and $A^5$ independently of each other represent a single covalent bond, 1,4-phenylene or trans-1,4-cyclohexylene ; $R^{11}$ signifies trans-1-alkenyl, cis-2-alkenyl, trans-3-alkenyl, 4-alkenyl, trans-2-alkenyloxy, 3-alkenyloxy or, when $R^2$ stands for an alkenyl group, also alkyl or alkoxy ; ring $A^1$ represents 1,4-phenylene or a group of the general formula

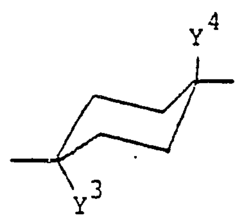

(II)

and $Y^3$ and $Y^4$ denote hydrogen or, insofar as $X^1$ represents $—CH_2CH_2—$, —COO— or —OOC—, one of the symbols $Y^3$ and $Y^4$ also denotes cyano.

2. Compounds according to claim 1, characterized in that $R^1$ and $R^2$ independently of each other signify trans-1-alkenyl, cis-2-alkenyl, trans-3-alkenyl or 4-alkenyl or one of the residues $R^1$ and $R^2$ also signifies alkyl, and $Y^3$ and $Y^4$ in formula II denote hydrogen or, insofar as $X^1$ represents —COO— or —OOC—, one of the symbols $Y^3$ and $Y^4$ also denotes cyano.

3. Compounds according to claim 1, characterized in that $X^1$ represents $—CH_2CH_2—$, $R^1$ and $R^2$ independently of each other signify trans-1-alkenyl, cis-2-alkenyl, trans-3-alkenyl or 4-alkenyl or one of the residues $R^1$ and $R^2$ also signifies alkyl, ring $A^1$ represents a group of formula II, and one of the symbols $Y^3$ and $Y^4$ denotes hydrogen and the other denotes cyano.

4. Compounds according to claim 2 or 3, characterized in that $R^1$ and $R^2$ signify residues with a maximum of 12 carbon atoms, preferably a maximum of 7 carbon atoms.

5. Compounds according to any one of claims 2 to 4, characterized in that $R^1$ and $R^2$ independently of each other signify trans-1-alkenyl, trans-3-alkenyl or 4-alkenyl or one of the residues $R^1$ and $R^2$, preferably $R^1$, also signifies alkyl.

6. Compounds according to claim 5, characterized in that one of the residues $R^1$ and $R^2$ signifies trans-3-alkenyl and the other signifies trans-1-alkenyl, trans-3-Alkenyl, 4-alkenyl or alkyl.

7. Compounds according to any one of claims 1 to 6, characterized in that $Y^1$ and a substituent $Y^3$ optionally present signify hydrogen and $Y^2$ signifies cyano.

8. A liquid crystalline mixture with at least 2 components, characterized in that at least one component is a compound of formula I defined in claim 1.

9. A process for the manufacture of the compounds of formula I defined in claim 1, characterized by

a) for the manufacture of the compounds of formula I in which $X^1$ represents —COO— or —OOC—, esterifying a compound of the general formula

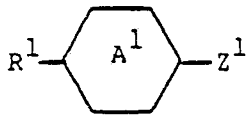

(III)

with a compound of the general formula

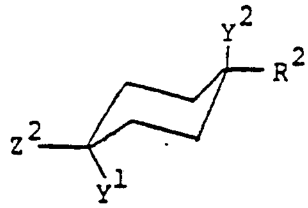

(IV)

wherein one of the groups $Z^1$ and $Z^2$ signifies —COOH or —COCl and the other signifies —OH ; ring $A^1$ represents 1,4-phenylene or a group of formula II defined in claim 1 ; $Y^3$ and $Y^4$ in formula II denote hydrogen or one of these symbols also denotes cyano ; and $R^1$, $R^2$, $Y^1$ and $Y^2$ have the significances given in claim 1, or

b) for the manufacture of the compounds of formula I in which $X^1$ represents a single covalent bond, $—CH_2CH_2—$ or 1,4-phenylene, $Y^2$ denotes cyano and $R^2$ signifies cis-2-alkenyl, trans-3-alkenyl, 4-alkenyl or alkyl, reacting a compound of the general formula

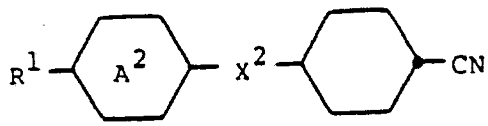

(V)

firstly with base and then with a compound of the general formula

$$Z^3—R^5 \quad (VI)$$

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano ; $Z^3$ denotes halogen ; $R^5$ signifies cis-2-alkenyl, trans-3-alkenyl, 4-alkenyl or alkyl ; and $R^1$ has the significance given in claim 1, or

c) for the manufacture of the compounds of formula I in which $X^1$ represents a single covalent bond or $—CH_2CH_2—$, ring $A^1$ signifies trans-1,4-cyclohexylene and $Y^1$ denotes cyano, reacting a compound of the general formula

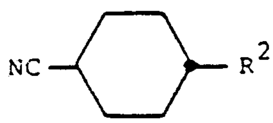

(VII)

firstly with base and then with a compound of the general formula

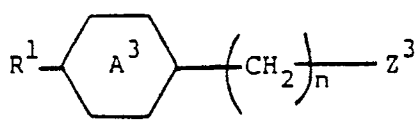

(VIII)

wherein n stands for the number 0 and ring $A^3$ stands for cis-1,4-cyclohexylene or n stands for the number 2 and ring $A^3$ stands for trans-1,4-cyclohexylene ; $Z^3$ denotes halogen ; and $R^1$ and $R^2$ have the significances given in claim 1, or

d) for the manufacture of the compounds of formula I in which $X^1$ represents a single covalent bond, $—CH_2CH_2—$ or 1,4-phenylene and $Y^1$ denotes cyano, reacting a compound of the general formula

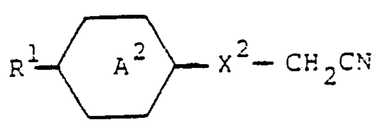

(IX)

firstly with base and then with a compound of the general formula

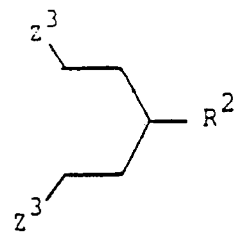

(X)

wherein $X^2$ represents a single covalent bond, $-CH_2CH_2-$ or 1,4-phenylene ; ring $A^2$ denotes 1,4-phenylene or a group of formula II ; $Y^3$ and $Y^4$ in formula II signify hydrogen or, insofar as $X^2$ represents $-CH_2CH_2-$, one of the symbols $Y^3$ and $Y^4$ also denotes cyano ; $Z^3$ denotes halogen ; and $R^1$ and $R^2$ have the significances given in claim 1, or

e) for the manufacture of the compounds of formula I in which $X^1$ represents a single covalent bond, $-CH_2CH_2-$ or 1,4-phenylene, $Y^2$ denotes cyano and $R^2$ signifies trans-1-alkenyl, reacting a compound of the general formula

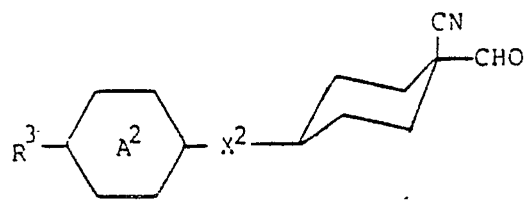

(XI)

wherein $X^2$ represents a single covalent bond, $-CH_2CH_2-$ or 1,4-phenylene ; ring $A^2$ denotes 1,4-phenylene or a group of formula II ; $Y^3$ and $Y^4$ in formula II signify hydrogen or, insofar as $X^2$ represents $-CH_2CH_2-$, one of the symbols $Y^3$ and $Y^4$ also denotes cyano ; and $R^3$ has the significances given for $R^1$ in formula I, in the presence of a base with an alkyl-triarylphosphonium halide, or

f) for the manufacture of the compounds of formula I in which $X^1$ represents a single covalent bond, $-CH_2CH_2-$ or 1,4-phenylene, adding HCN to a compound of the general formula

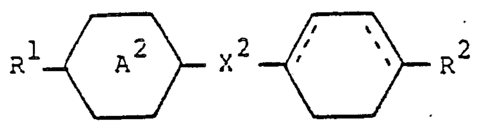

(XII)

wherein $X^2$ represents a single covalent bond, $-CH_2CH_2-$ or 1,4-phenylene ; ring $A^2$ denotes 1,4-phenylene or a group of formula II ; $Y^3$ and $Y^4$ in formula II signify hydrogen or, insofar as $X^2$ represents $-CH_2CH_2-$, one of the symbols $Y^3$ and $Y^4$ also denotes cyano, one of the dotted lines denotes an additional bond ; and $R^1$ and $R^2$ have the significances given in claim 1, or

g) for the manufacture of the compounds of formula I in which $X^1$ represents a single covalent bond, $-CH_2CH_2-$ or 1,4-phenylene and $R^1$ signifies trans-2-alkenyloxy, 3-alkenyloxy or alkoxy, etherifying a compound of the general formula

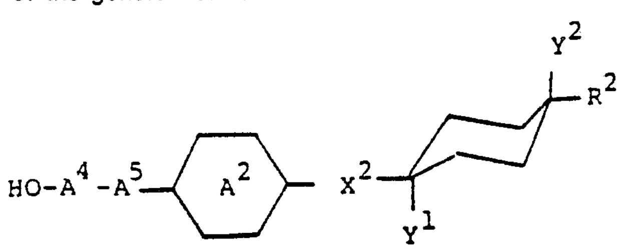

(LXXXI)

wherein $X^2$ represents a single covalent bond, $-CH_2CH_2-$ or 1,4-phenylene ; ring $A^2$ denotes 1,4-phenylene or a group of formula II ; $Y^3$ and $Y^4$ in formula II signify hydrogen or, insofar as $X^2$ represents $-CH_2CH_2-$, one of the symbols $Y^3$ and $Y^4$ also denotes cyano ; and $A^4$, $A^5$, $R^2$, $Y^1$ and $Y^2$ have the significances given in formula I, with a trans-2-alkenyl halide, a 3-alkenyl halide or an alkyl halide.

10. The use of the compounds of formula I defined in claim 1 for electro-optical purposes.

**Claims** (for the Contracting State AT)

1. A liquid crystalline mixture with at least 2 components, characterized in that at least one component is a compound of the general formula

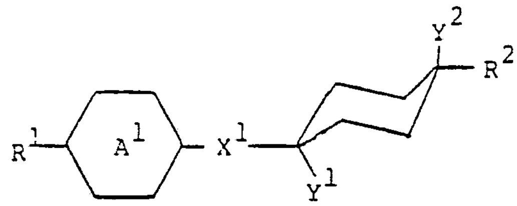

(I)

wherein $X^1$ represents a single covalent bond, —$CH_2CH_2$—, —COO—, —OOC— or 1,4-phenylene ; one of the symbols $Y^1$ and $Y^2$ denotes hydrogen and the other denotes cyano ; $R^2$ signifies trans-1-alkenyl, cis-2-alkenyl, trans-3-alkenyl, 4-alkenyl or alkyl ; $R^1$ stands for the group $R^{11}$—$A^4$—$A^5$— and $A^4$ and $A^5$ independently of each other represent a single covalent bond, 1,4-phenylene or trans-1,4-cyclohexylene ; $R^{11}$ signifies trans-1-alkenyl, cis-2-alkenyl, trans-3-alkenyl, 4-alkenyl, trans-2-alkenyloxy, 3-alkenyloxy or, when $R^2$ stands for an alkenyl group, also alkyl or alkoxy ; ring $A^1$ represents 1,4-phenylene or a group of the general formula

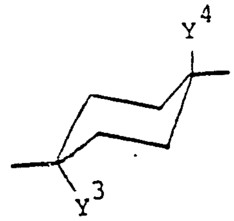

(II)

and $Y^3$ and $Y^4$ denote hydrogen or, insofar as $X^1$ represents —$CH_2CH_2$—, —COO— or —OOC—, one of the symbols $Y^3$ and $Y^4$ also denotes cyano.

2. A liquid crystalline mixture according to claim 1, characterized in that $R^1$ and $R^2$ independently of each other signify trans-1-alkenyl, cis-2-alkenyl, trans-3-alkenyl or 4-alkenyl or one of the residues $R^1$ and $R^2$ also signifies alkyl, and $Y^3$ and $Y^4$ in formula II denote hydrogen or, insofar as $X^1$ represents —COO— or —OOC—, one of the symbols $Y^3$ and $Y^4$ also denotes cyano.

3. A liquid crystalline mixture according to claim 1, characterized in that $X^1$ represents —$CH_2CH_2$—, $R^1$ and $R^2$ independently of each other signify trans-1-alkenyl, cis-2-alkenyl, trans-3-alkenyl or 4-alkenyl or one of the residues $R^1$ and $R^2$ also signifies alkyl, ring $A^1$ represents a group of formula II, and one of the symbols $Y^3$ and $Y^4$ denotes hydrogen and the other denotes cyano.

4. A liquid crystalline mixture according to claim 2 or 3, characterized in that $R^1$ and $R^2$ signify residues with a maximum of 12 carbon atoms, preferably a maximum of 7 carbon atoms.

5. A liquid crystalline mixture according to any one of claims 2 to 4, characterized in that $R_1$ and $R^2$ independently of each other signify trans-1-alkenyl, trans-3-alkenyl or 4-alkenyl or one of the residues $R^1$ and $R^2$, preferably $R^1$, also signifies alkyl.

6. A liquid crystalline mixture according to claim 5, characterized in that one of the residues $R^1$ and $R^2$ signifies trans-3-alkenyl and the other signifies trans-1-alkenyl, trans-3-alkenyl, 4-alkenyl or alkyl.

7. A liquid crystalline mixture according to any one of claims 1 to 6, characterized in that $Y^1$ and a substituent $Y^3$ optionally present signify hydrogen and $Y^2$ signifies cyano.

8. A process for the manufacture of the compounds of formula I defined in claim 1, characterized by

a) for the manufacture of the compounds of formula I in which $X^1$ represents —COO— or —OOC—, esterifying a compound of the general formula

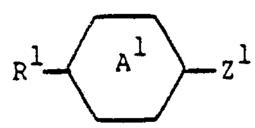

(III)

with a compound of the general formula

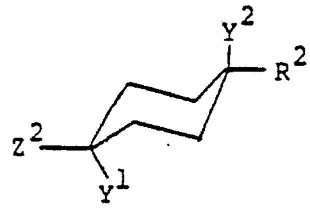

(IV)

wherein one of the groups $Z^1$ and $Z^2$ signifies —COOH or —COCl and the other signifies —OH ; ring $A^1$ represents 1,4-phenylene or a group of formula II defined in claim 1 ; $Y^3$ and $Y^4$ in formula II denote

hydrogen or one of these symbols also denote cyano ; and $R^1$, $R^2$, $Y^1$ and $Y^2$ have the significances given in claim 1, or

b) for the manufacture of the compounds of formula I in which $X^1$ represents a single covalent bond, $—CH_2CH_2—$ or 1,4-phenylene, $Y^2$ denotes cyano and $R^2$ signifies cis-2-alkenyl, trans-3-alkenyl, 4-alkenyl or alkyl, reacting a compound of the general formula

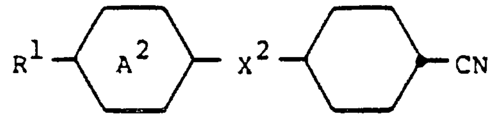

(V)

firstly with base and then with a compound of the general formula

$$Z^3—R^5 \quad (VI)$$

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano ; $Z^3$ denotes halogen ; $R^5$ signifies cis-2-alkenyl, trans-3-alkenyl, 4-alkenyl or alkyl ; and $R^1$ has the significance given in claim 1, or

c) for the manufacture of the compounds of formula I in which $X^1$ represents a single covalent bond or $—CH_2CH_2—$, ring $A^1$ signifies trans-1,4-cyclohexylene and $Y^1$ denotes cyano, reacting a compound of the general formula

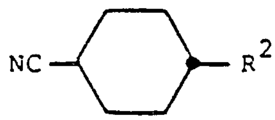

(VII)

firstly with base and then with a compound of the general formula

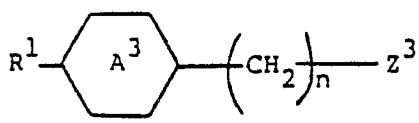

(VIII)

wherein n stands for the number 0 and ring $A^3$ stands for cis-1,4-cyclohexylene or n stands for the number 2 and ring $A^3$ stands for trans-1,4-cyclohexylene ; $Z^3$ denotes halogen ; and $R^1$ and $R^2$ have the significances given in claim 1, or

d) for the manufacture of the compounds of formula I in which $X^1$ represents a single covalent bond, $—CH_2CH_2—$, or 1,4-phenylene and $Y^1$ denotes cyano, reacting a compound of the general formula

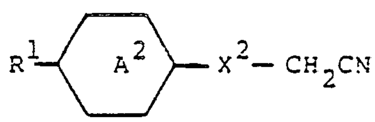

(IX)

firstly with base and then with a compound of the general formula

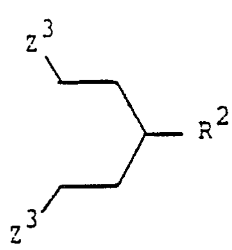

(X)

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano ; $Z^3$ denotes halogen ; and $R^1$ and $R^2$ have the significances given in claim 1, or

e) for the manufacture of the compounds of formula I in which $X^1$ represents a single covalent bond, $—CH_2CH_2—$ or 1,4-phenylene, $Y^2$ denotes cyano and $R^2$ signifies trans-1-alkenyl, reacting a compound of the general formula

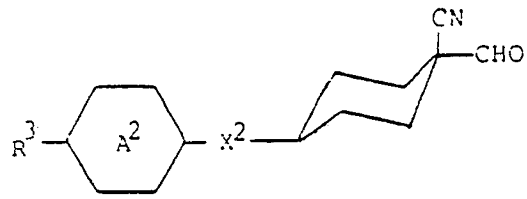

(XI)

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano ; and $R^3$ has the significances given for $R^1$ in formula I, in the presence of a base with an alkyl-triarylphosphonium halide, or

f) for the manufacture of the compounds of formula I in which $X^1$ represents a single covalent bond, $—CH_2CH_2—$ or 1,4-phenylene, adding HCN to a compound of the general formula

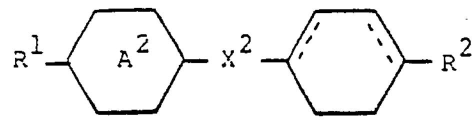

(XII)

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano, one of the dotted lines denotes an additional bond ; and $R^1$ and $R^2$ have the significances given in claim 1, or

g) for the manufacture of the compounds of formula I in which $X^1$ represents a single covalent bond, $—CH_2CH_2—$ or 1,4-phenylene and $R^1$ signifies trans-2-alkenyloxy, 3-alkenyloxy or alkoxy, etherifying a compound of the general formula

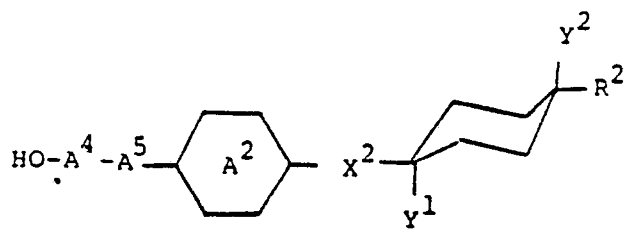

(LXXXI)

wherein $X^2$ represents a single covalent bond, $—CH_2CH_2—$ or 1,4-phenylene ; ring $A^2$ denotes 1,4-phenylene or a group of formula II ; $Y^3$ and $Y^4$ in formula II signify hydrogen or, insofar as $X^2$ represents $—CH_2CH_2—$, one of the symbols $Y^3$ and $Y^4$ also denotes cyano ; and $A^4$, $A^5$, $R^2$, $Y^1$ and $Y^2$ have the significances given in formula I, with a trans-2-alkenyl halide, a 3-alkenyl halide or an alkyl halide.

9. The use of the compounds of formula I defined in claim 1 for electro-optical purposes.

**Revendications** (pour les Etats contractants : BE, CH, DE, FR, GB, IT, LI, NL, SE)

1. Composés de formule générale

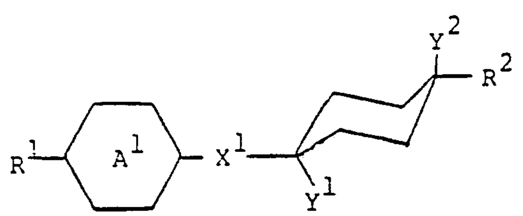

(I)

où X1 représente une liaison de covalence simple, $—CH_2CH_2—$, —COO—, —OOC— ou un 1,4-phénylène ; l'un des symboles Y1 et Y2 représente un hydrogène et l'autre un cyano ; R2 représente un trans-1-alcényle, cis-2-alcényle, trans-3-alcényle, 4-alcényle ou alcoyle ; R1 représente le groupe $R^{11}—A^4—A^5—$ et A4 et A5 représentent indépendamment l'un de l'autre une liaison de covalence simple, un 1,4-phénylène ou trans-1,4-cyclohexylène ; R11 représente un trans-1-alcényle, cis-2-alcényle, trans-3-alcényle, 4-alcényle, trans-2-alcényloxy, 3-alcényloxy ou encore, lorsque R2 représente un groupe alcényle, un alcoyle ou un alcoxy ; le noyau A1 représente un 1,4-phénylène ou un groupe de formule générale

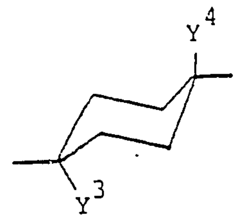

(II)

et Y3 et Y4 représentent un hydrogène ou, si X1 représente —$CH_2CH_2$—, —COO— ou —OOC—, l'un des symboles Y3 et Y4 représente également un cyano.

2. Composés selon la revendication 1, caractérisés en ce que R1 et R2 représentent indépendamment l'un de l'autre un trans-1-alcényle, cis-2-alcényle, trans-3-alcényle ou 4-alcényle, ou l'un des radicaux R1 et R2 représentent également un alcoyle, et Y3 et Y4 dans la formule II représentent un hydrogène, ou, si X1 représente —COO— ou —OOC—, l'un des symboles Y3 et Y4 représente également un cyano.

3. Composés selon la revendication 1, caractérisés en ce que X1 représente —$CH_2CH_2$—, R1 et R2 représentent indépendamment l'un de l'autre un trans-1-alcényle, cis-2-alcényle, trans-3-alcényle ou 4-alcényle, ou l'un des radicaux R1 et R2 représente également un alcoyle, le noyau A1 représente un groupe de formule II, et l'un des symboles Y3 et Y4 représente un hydrogène et l'autre un cyano.

4. Composés selon la revendication 2 ou 3, caractérisés en ce que R1 et R2 représentent des radicaux ayant au plus 12 atomes de carbone, de préférence au plus 7 atomes de carbone.

5. Composés selon l'une des revendications 2 à 4, caractérisés en ce que R1 et R2 représentent indépendamment l'un de l'autre un trans-1-alcényle, trans-3-alcényle ou 4-alcényle, ou l'un des radicaux R1 et R2, de préférence R1, représente également un alcoyle.

6. Composés selon la revendication 5, caractérisés en ce que l'un des radicaux R1 et R2 représente un trans-3-alcényle et l'autre un trans-1-alcényle, trans-3-alcényle, 4-alcényle ou alcoyle.

7. Composés selon l'une des revendications 1 à 6, caractérisés en ce que Y1 et un substituant Y3 éventuellement présent représentent un hydrogène et Y2 un cyano.

8. Mélange de cristaux liquides avec au moins 2 composants, caractérisé en ce qu'au moins un composant est un composé de formule I définie dans la revendication 1.

9. Procédé de préparation des composés de formule I définie dans la revendication 1, caractérisé en ce que

a) pour préparer les composés de formule I où X1 représente —COO— ou —OOC—, on estérifie un composé de formule générale

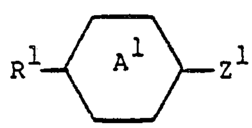

(III)

avec un composé de formule générale

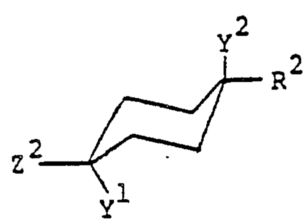

(IV)

où l'un des groupes Z1 et Z2 représente —COOH ou —COCl et l'autre représente —OH ; le noyau A1 représente un 1,4-phénylène ou un groupe de formule II définie dans la revendication 1 ; Y3 et Y4 dans la formule II représentent un hydrogène, ou l'un de ces symboles représente également un cyano ; et R1, R2, Y1 et Y2 ont les significations données dans la revendication 1, ou

b) pour préparer les composés de formule I où X1 représente une liaison de covalence simple, —$CH_2CH_2$— ou un 1,4-phénylène, Y2 représente un cyano et R2 représente un cis-2-alcényle, trans-3-alcényle, 4-alcényle ou alcoyle, on fait réagir un composé de formule générale

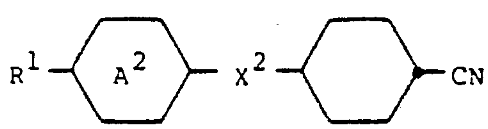

(V)

tout d'abord avec une base puis avec un composé de formule générale

$Z^3$—$R^5$ (VI)

où X2 représente une liaison simple, —$CH_2CH_2$— ou un 1,4-phénylène ; le noyau A2 représente un 1,4-phénylène ou un groupe de formule II ; Y3 et Y4 dans la formule II représentent un hydrogène ou, si X2 représentent —$CH_2CH_2$—, l'un des symboles Y3 et Y4 représente également un cyano ; Z3 représente un halogène ; R5 représente un cis-2-alcényle, trans-3-alcényle, 4-alcényle ou alcoyle ; et R1 a la signification donnée ci-dessus, ou

c) pour préparer les composés de formule I où X1 représente une liaison de covalence simple ou —$CH_2CH_2$—, le noyau A1 représente un trans-1,4-cyclohexylène et Y1 représente un cyano, on fait réagir un composé de formule générale

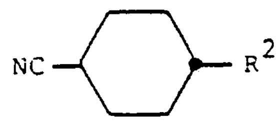

(VII)

tout d'abord avec une base puis avec un composé de formule générale

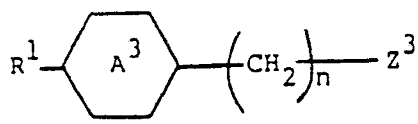

(VIII)

où n représente le nombre 0 et le noyau A3 représente un cis-1,4-cyclohexylène ou n représente le nombre 2 et le noyau A3 représente un trans-1,4-cyclohexylène ; Z3 représente un halogène ; et R1 et R2 ont les significations données dans la revendication 1, ou

d) pour préparer les composés de formule I où X1 représente une liaison de covalence simple, —$CH_2CH_2$— ou un 1,4-phénylène et Y1 représente un cyano, on fait réagir un composé de formule générale

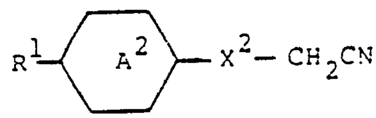

(IX)

tout d'abord avec une base puis avec un composé de formule générale

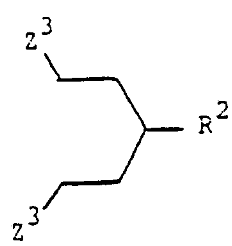

(X)

où X2 représente une liaison de covalence simple, —$CH_2CH_2$— ou un 1,4-phénylène ; le noyau A2 représente un 1,4-phénylène ou un groupe de formule II ; Y3 et Y4 dans la formule II représentent un hydrogène ou, si X2 représente —$CH_2CH_2$—, l'un des symboles Y3 et Y4 représente également un cyano ; Z3 représente un halogène ; et R1 et R2 ont les significations données dans la revendication 1, ou

e) pour préparer des composés de formule I où X1 représente une liaison de covalence simple, —$CH_2CH_2$— ou un 1,4-phénylène ; Y2 représente un cyano et R2 un trans-1-alcényle, on fait réagir un composé de formule générale

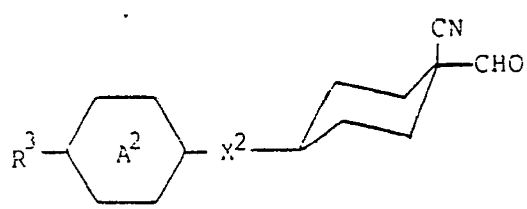

(XI)

où X2 représente une liaison de covalence simple, —$CH_2CH_2$— ou un 1,4-phénylène ; le noyau A2 représente un 1,4-phénylène ou un groupe de formule II ; Y3 et Y4 dans la formule II représentent un hydrogène ou, si X2 représente —$CH_2CH_2$—, l'un des symboles Y3 et Y4 représente également un cyano ; et R3 a les significations données pour R1 dans la formule I, en présence d'une base avec un halogénure d'alcoyltriarylphosphonium, ou

f) pour préparer des composés de formule I où X1 représente une liaison de covalence simple, $—CH_2CH_2—$ ou un 1,4-phénylène, on fixe HCN sur un composé de formule générale

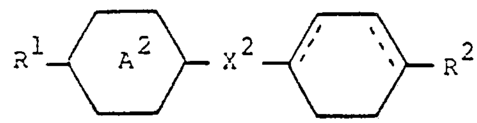

(XII)

où X2 représente une liaison de covalence simple, $—CH_2CH_2—$ ou un 1,4-phénylène ; le noyau A2 représente un 1,4-phénylène ou un groupe de formule II ; Y3 et Y4 dans la formule II représentent un hydrogène ou, si X2 représente $—CH_2CH_2—$, l'un des symboles Y3 et Y4 représente également un cyano ; l'une des lignes interrompues représente une liaison supplémentaire ; et R1 et R2 ont les significations données dans la revendication 1, ou

g) pour préparer des composés de formule I où X1 représente une liaison de covalence simple, $—CH_2CH_2—$ ou un 1,4-phénylène, et R1 représente un trans-2-alcényloxy, 3-alcényloxy ou alcoxy, on éthérifie un composé de formule générale

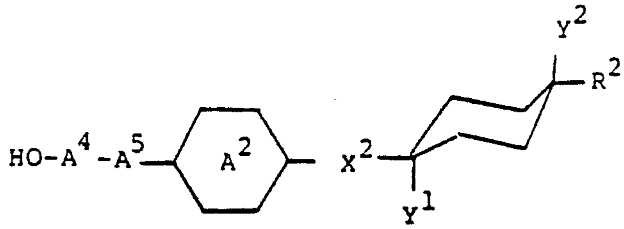

(LXXXI)

où X2 représente une liaison de covalence simple, $—CH_2CH_2—$ ou un 1,4-phénylène ; le noyau A2 représente un 1,4-phénylène ou un groupe de formule II ; Y3 et Y4 dans la formule II représentent un hydrogène ou, si X2 représente $—CH_2CH_2—$, l'un des symboles Y3 et Y4 représente également un cyano ; et A4, A5, R2, Y1 et Y2 ont les significations données dans la formule I, avec un halogénure de trans-2-alcényle, un halogénure de 3-alcényle ou un halogénure d'alcoyle.

10. Application des composés de formule I définie dans la revendication à des buts électro-optiques.

**Revendications** (pour l'Etat contractant AT)

1. Mélange de cristaux liquides avec au moins 2 composants, caractérisé en ce qu'au moins un composant est un composé de formule générale

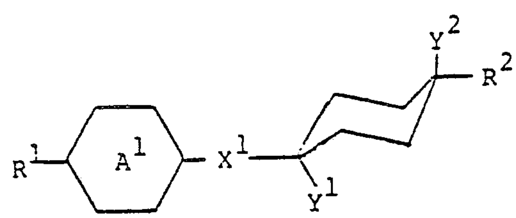

(I)

où X1 représente une liaison de covalence simple, $—CH_2CH_2—$, —COO—, —OOC— ou un 1,4-phénylène ; l'un des symboles Y1 et Y2 représente un hydrogène et l'autre un cyano ; R2 représente un trans-1-alcényle, cis-2-alcényle, trans-3-alcényle, 4-alcényle ou alcoyle ; R1 représente le groupe $R^{11}—A^4—A^5—$ et A4 et A5 représentent indépendamment l'un de l'autre une liaison de covalence simple, un 1,4-phénylène ou trans-1,4-cyclohexylène ; R11 représente un trans-1-alcényle, cis-2-alcényle, trans-3-alcényle, 4-alcényle, trans-2-alcényloxy, 3-alcényloxy ou encore, lorsque R2 représente un groupe alcényle, un alcoyle ou un alcoxy ; le noyau A1 représente un 1,4-phénylène ou un groupe de formule générale

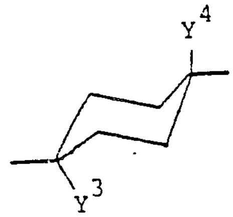

(II)

et Y3 et Y4 représentent un hydrogène ou, si X1 représente $—CH_2CH_2—$, —COO— ou —OOC—, l'un des symboles Y3 et Y4 représente également un cyano.

2. Mélange de cristaux liquides selon la revendication 1, caractérisé en ce que R1 et R2 représentent indépendamment l'un de l'autre un trans-1-alcényle, cis-2-alcényle, trans-3-alcényle ou 4-alcényle, ou encore l'un des radicaux R1 et R2 représente un alcoyle, et Y3 et Y4 dans la formule II représentent un hydrogène ou, si X1 représente —COO— ou —OOC—, l'un des symboles Y3 et Y4 représente également un cyano.

3. Mélange de cristaux liquides selon la revendication 1, caractérisé en ce que X1 représente —$CH_2CH_2$—, R1 et R2 représentent indépendamment l'un de l'autre un trans-1-alcényle, cis-2-alcényle, trans-3-alcényle ou 4-alcényle ou l'un des radicaux R1 et R2 représente également un alcoyle, le noyau A1 représente un groupe de formule II, et l'un des symboles Y3 et Y4 représente un hydrogène et l'autre un cyano.

4. Mélange de cristaux liquides selon la revendication 2 ou 3, caractérisé en ce que R1 et R2 représentent des radicaux avec au plus 12 atomes de carbone, de préférence au plus 7 atomes de carbone.

5. Mélange de cristaux liquides selon l'une des revendications 2 à 4, caractérisé en ce que R1 et R2 représentent indépendamment l'un de l'autre un trans-1-alcényle, trans-3-alcényle ou 4-alcényle ou l'un des radicaux R1 et R2, de préférence R1, représente également un alcoyle.

6. Mélange de cristaux liquides selon la revendication 5, caractérisé en ce que l'un des radicaux R1 et R2 représente un trans-3-alcényle et l'autre un trans-1-alcényle, trans-3-alcényle, 4-alcényle ou alcoyle.

7. Mélange de cristaux liquides selon l'une des revendications 1 à 6, caractérisé en ce que Y1 et un substituant Y3 éventuellement présent représentent un hydrogène et Y2 un cyano.

8. Procédé de préparation des composés de formule I définie dans la revendication 1, caractérisé en ce que

a) pour préparer les composés de formule I où X1 représente —COO— ou —OOC—, on estérifie un composé de formule générale

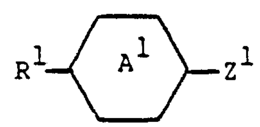

(III)

avec un composé de formule générale

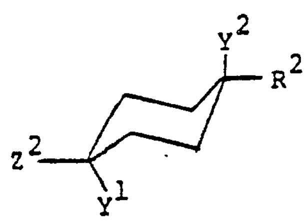

(IV)

où l'un des groupes Z1 et Z2 représente —COOH ou —COCl et l'autre représente —OH ; le noyau A1 représente un 1,4-phénylène ou un groupe de formule II définie dans la revendication 1 ; Y3 et Y4 dans la formule II représentent un hydrogène, ou l'un de ces symboles représente également un cyano ; et R1, R2, Y1 et Y2 ont les significations données dans la revendication 1, ou

b) pour préparer les composés de formule I où X1 représente une liaison de covalence simple, —$CH_2CH_2$— ou un 1,4-phénylène, Y2 représente un cyano et R2 représente un cis-2-alcényle, trans-3-alcényle, 4-alcényle ou alcoyle, on fait réagir un composé de formule générale

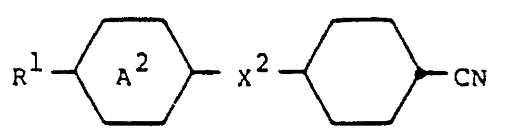

(V)

tout d'abord avec une base puis avec un composé de formule générale

$$Z^3—R^5 \qquad (VI)$$

où X2 représente une liaison simple, —$CH_2CH_2$— ou un 1,4-phénylène ; le noyau A2 représente un 1,4-phénylène ou un groupe de formule II ; Y3 et Y4 dans la formule II représentent un hydrogène ou, si X2 représente —$CH_2CH_2$—, l'un des symboles Y3 et Y4 représente également un cyano ; Z3 représente un halogène ; R5 représente un cis-2-alcényle, trans-3-alcényle, 4-alcényle ou alcoyle ; et R1 a la signification donnée ci-dessus, ou

c) pour préparer les composés de formule I où X1 représente une liaison de covalence simple ou —$CH_2CH_2$—, le noyau A1 représente un trans-1,4-cyclohexylène et Y1 représente un cyano, on fait réagir un composé de formule générale

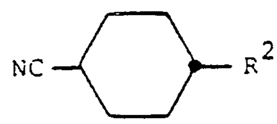

(VII)

tout d'abord avec une base puis avec un composé de formule générale

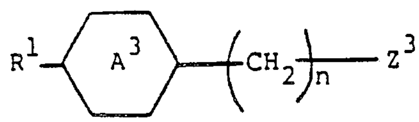

(VIII)

où n représente le nombre 0 et le noyau A3 représente un cis-1,4-cyclohexylène ou n représente le nombre 2 et le noyau A3 représente un trans-1,4-cyclohexylène ; Z3 représente un halogène ; et R1 et R2 ont les significations données dans la revendication 1, ou

d) pour préparer les composés de formule I où X1 représente une liaison de covalence simple, $—CH_2CH_2—$ ou un 1,4-phénylène et Y1 représente un cyano, on fait réagir un composé de formule générale

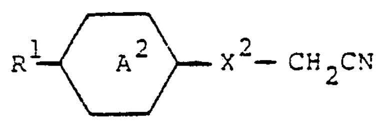

(IX)

tout d'abord avec une base puis avec un composé de formule générale

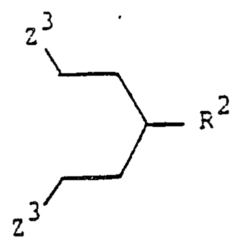

(X)

où X2 représente une liaison de covalence simple, $—CH_2CH_2—$ ou un 1,4-phénylène ; le noyau A2 représente un 1,4-phénylène ou un groupe de formule II ; Y3 et Y4 dans la formule II représentent un hydrogène ou, si X2 représente $—CH_2CH_2—$, l'un des symboles Y3 et Y4 représente également un cyano ; Z3 représente un halogène ; et R1 et R2 ont les significations données dans la revendication 1, ou

e) pour préparer des composés de formule I où X1 représente une liaison de covalence simple, $—CH_2CH_2—$ ou un 1,4-phénylène ; Y2 représente un cyano et R2 un trans-1-alcényle, on fait réagir un composé de formule générale

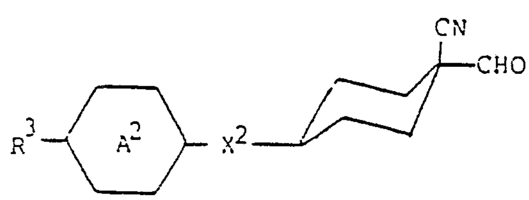

(XI)

où X2 représente une liaison de covalence simple, $—CH_2CH_2—$ ou un 1,4-phénylène ; le noyau A2 représente un 1,4-phénylène ou un groupe de formule II ; Y3 et Y4 dans la formule II représentent un hydrogène ou, si X2 représente $—CH_2CH_2—$, l'un des symboles Y3 et Y4 représente également un cyano ; et R3 a les significations données pour R1 dans la formule I, en présence d'une base avec un halogénure d'alcoyltriarylphosphonium, ou

f) pour préparer des composés de formule I où X1 représente une liaison de covalence simple, $—CH_2CH_2—$ ou un 1,4-phénylène, on fixe HCN sur un composé de formule générale

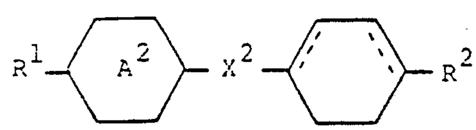

(XII)

où X2 représente une liaison de covalence simple, $—CH_2CH_2—$ ou un 1,4-phénylène ; le noyau A2

représente un 1,4-phénylène ou un groupe de formule II ; Y3 et Y4 dans la formule II représentent un hydrogène ou, si X2 représente $-CH_2CH_2-$, l'un des symboles Y3 et Y4 représente également un cyano ; l'une des lignes interrompues représente une liaison supplémentaire ; et R1 et R2 ont les significations données dans la revendication 1, ou

g) pour préparer des composés de formule I où X1 représente une liaison de covalence simple, $-CH_2CH_2-$ ou un 1,4-phénylène, et R1 représente un trans-2-alcényloxy, 3-alcényloxy ou alcoxy, on éthérifie un composé de formule générale

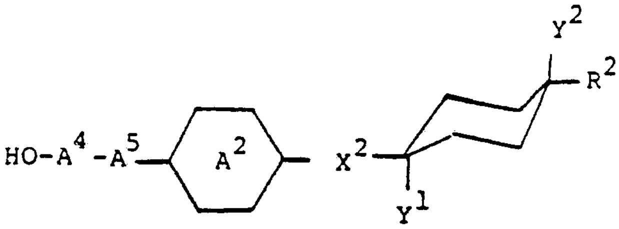

(LXXXI)

où X2 représente une liaison de covalence simple, $-CH_2CH_2-$ ou un 1,4-phénylène ; le noyau A2 représente un 1,4-phénylène ou un groupe de formule II ; Y3 et Y4 dans la formule II représentent un hydrogène ou, si X2 représente $-CH_2CH_2-$, l'un des symboles Y3 et Y4 représente également un cyano ; et A4, A5, R2, Y1 et Y2 ont les significations données dans la formule I, avec un halogénure de trans-2-alcényle, un halogénure de 3-alcényle ou un halogénure d'alcoyle.

9. Application des composés de formule I définie dans la revendication 1 à des buts électro-optiques.